# EUROPEAN PATENT APPLICATION

(11) **EP 3 842 049 A1**
(43) Date of publication of application: **30.06.2021**
(21) Application number: 21157385.2
(22) Date of filing: 13.07.2017
(51) Int. Cl.: A61K 35/28, C12N 5/0775, G01N 33/68, A61P 25/28, A61P 25/16, A61P 25/00, A61P 9/10, A61P 3/00, G01N 33/573

(54) **METHODS FOR DIAGNOSING AMYOTROPHIC LATERAL SCLEROSIS (ALS)**

(30) Priority: 18.07.2016 US 201662363672 P
(62) Divisional of application: 17754804.7
(71) Applicant: Brainstorm Cell Therapeutics Ltd., 4912502 Petach Tikva (IL)
(72) Inventor: ARICHA, Revital, 5442502 Givat Shmuel (IL); GOTHELF, Yael, 5556015 Kiryat Ono (IL); ABRAMOV, Natalie, 7666609 Rehovot (IL); KASPI, Haggai, 5591899 Ganei Tikva (IL)
(74) Representative: Pearl Cohen Zedek Latzer Baratz UK LLP

(57) **Abstract**

A method of diagnosing amyotrophic lateral sclerosis by detecting the level of chitinase 1 in a cerebrospinal fluid sample from the subject.

## Description

### FIELD OF DISCLOSURE

The disclosure relates to cells and populations thereof which can be used for treating neurodegenerative diseases, for example amyotrophic lateral sclerosis (ALS) disease. Specifically, the disclosure relates to treating ALS by administering a mesenchymal stem cell induced to secrete neurotrophic factors (NTFs).

### BACKGROUND

Amyotrophic lateral sclerosis (ALS) is a fatal neurological disease in which the degeneration and death of motor neurons (MNs) leads to weakness, paralysis and eventually respiratory failure requiring ventilator support. There is currently no available treatment to stop or reverse its progressive course; thus, there remains an unmet medical need for safe and effective treatments for people with ALS.

While the vast majority of ALS cases are sporadic, a number of forms of familial ALS have been identified which account for about 10% of ALS cases. The identification of some ALS-specific mutations, such as in the superoxide dismutase gene, have led to a number of mechanistic hypotheses regarding the etiology of neurodegeneration in ALS. Nevertheless, there may be a variety of mechanisms that lead to a final common pathway of motor neuron degeneration. Based on putative pathophysiologic mechanisms, a variety of therapeutics have been or are being investigated in ALS, including anti-glutamatergic agents, drugs targeting protein misfolding and accumulation, antioxidant therapy, immunomodulatory agents, and stem cell transplantation. Neurotrophic factors (NTFs), which promote the growth and survival of neurons, have been an area of significant prior pre-clinical and clinical research.

Chitotriosidase (CHIT1) belongs primarily to the chitinase family1, which represents a class of enzymes that catalyze the hydrolysis of chitin to simple sugars. Chitin, the natural substrate of CHIT-1, is an insoluble N-acetylglucosamine polymer found in invertebrates and human parasites.

In a healthy population, CHIT1 activity is very low and originates in circulating polymorphonuclear cells. Conversely, during the development of acute/chronic inflammatory disorders, the enzymatic activity of CHIT1 increases significantly throughout effective maturation of monocytes into macrophages.

CHIT1 has been included as one of the secreted biomarkers for Gaucher's disease. The elevation of CHIT1 in these patients may reflect a particular state of activation of macrophages. Moreover, CHIT1 has been widely implicated in a variety of diseases involving immune dysfunction. Recently, CHIT1 has come under increasing scrutiny due to its excess secretion into the serum or over expression in tissues which are chronically inflamed.

Chitin is absent in the human brain, however, even in the absence of the substrate the enzyme is synthesized by microglia or infiltrating macrophages. CHIT1 seems to play a role in neuroinflammation and it has been found to be increased in multiple sclerosis and Alzheimer's disease.

Accordingly, there exists a need for improved compositions and methods for treating ALS. Further, as described here, CHIT1 may provide a biomarker with which to follow the progression of ALS, which in combination with treatment methods could provide a benefit for subjects suffering from ALS.

### SUMMARY OF THE DISCLOSURE

In one aspect, described herein is a method of treating a neurodegenerative disease in a subject in need thereof, the method comprising administering to said subject a therapeutically effective amount of a cell population of mesenchymal stem cells (MSC) cells that have been induced to secrete at least one neurotrophic factor (NTF), wherein said cell population comprises MSC-NTF cells, thereby treating said neurodegenerative disease in said subject.

In a related aspect, said neurodegenerative disease comprises Amyotrophic Lateral Sclerosis (ALS); Frontotemporal Dementia (FTD); Parkinson's disease; Multiple System Atrophy (MSA); Huntington's disease; Alzheimer's disease; Rett Syndrome; lysosomal storage diseases; "white matter disease" or glial/demyelination disease, including Sanfilippo, Gaucher disease; Tay Sachs disease (beta hexosaminidase deficiency); multiple sclerosis (MS); Neuromyelitis Optica (NMO); NMO spectrum disease; brain injury or trauma caused by ischemia, accidents, or environmental insult; stroke; cerebral palsy (CP); autism and autism spectrum disorder; spinal cord damage; or ataxia; or any combination thereof. In another related aspect, said mesenchymal stem cells comprise: (a) bone marrow mesenchymal stem cells, adipocyte mesenchymal stem cells, dental pulp mesenchymal stem cells placenta mesenchymal stem cells, synovial membrane mesenchymal stem cells, peripheral blood mesenchymal stem cells, periodontal ligament mesenchymal stem cells, endometrium mesenchymal stem cells, umbilical cord mesenchymal stem cells, or umbilical cord blood mesenchymal stem cells; (b) cells autologous with said subject; or (c) cells allogeneic with said subject; or any combination thereof. In another related aspect, said MSC-NTF comprise: (a) non-genetically modified human cells; or (b) mesenchymal stem cells induced *ex vivo* to express and secrete at least one neurotrophic factor (NTF); or any combination thereof.

In a related aspect, the basal secretion of said at least one NTF from said MSC-NTF is greater than a basal secretion of said NTF in a non-differentiated mesenchymal stem cell.

In a related aspect, the method further comprises a step of assaying a biological sample from said subject prior to and following said administration. In another related aspect, said biological sample comprises blood, serum, urine, or cerebrospinal (CSF). In another related aspect, following said administration, said biological sample comprises increased levels of at least one neurotrophic factor (NTF) compared with a control biological sample. In another related aspect, said neurotropic factor (NTF) is selected from the group comprising a vascular endothelial growth factor (VEGF), a hepatocyte growth factor (HGF), a leukemia inhibitory factor (LIF), Granulocyte Stimulating factor (G-CSF), a Brain-derived neurotrophic factor (BDNF), a Tumor necrosis factor-inducible gene 6 protein (TSG-6; also known as TNF-stimulated gene 6 protein), Bone morphogenetic protein 2 (BMP2), Fibroblast Growth Factor 2 (FGF2), or a Neublastin, or any combination thereof.

In a related aspect, following said administration, said biological sample comprises decreased levels of at least one inflammatory factor or pro-apoptotic factor or factor that influences inflammatory factors compared with a control biological sample. In another related aspect, said inflammatory factor or pro-apoptotic factor or factor that influence inflammatory factors is selected from the group comprising a chitinase 1 (CHIT1), a C-reactive protein (CRP), a monocyte chemotactic protein 1 (MCP1), a stromal derived factor 1 (SDF-1), Macrophage Inflammatory protein (MIP)-1b, Glutamate, or a caspase 3 (CASP3), or any combination thereof. In another related aspect, following said administration said biological sample comprises increased levels of at least one neurotrophic factor and decreased levels of at least one inflammatory factor or pro-apoptotic factor or factor that influences inflammatory factors compared with a control biological sample.

In a related aspect, the gene expression of at least one biomarker is modulated in said MSC-NTF cells compared with control MSC cells. In another related aspect, said biomarker comprises any one of TOP2A, FGF2, MEST, SLC1A1, or TUBB3, or a combination thereof, wherein said modulated gene expression comprises decreased gene expression. In another related aspect, said biomarker comprises any one of BMP2, LIF, WNT5A, AREG, HGF, BDNF, PCSK1, RAB27B, SNAP25, SLC1A3, or SLC16A6, or a combination thereof, wherein said modulated gene expression comprises increased gene expression.

In a related aspect, said administration comprises administering to (a) the cerebrospinal fluid or the central nervous system of the subject; or (b) intramuscular (IM) injection or intrathecal (IT) injection, or a combination thereof; or any combination thereof. In another related aspect, said IM injection is at a dose of about 2 x 10⁶ MSC-NTF cell per injection, and wherein said IT injection is at a dose of about 100-125 x 10⁶ MSC-NTF cells. In another related aspect, said administration comprises multiple IM injections, wherein said multiple injections comprise a dose of about 48 x 10⁶ MSC-NTF. In another related aspect, the administration comprises a therapeutically effective number of time points.

In a related aspect, the method further comprises a step of detecting a biomarker associated with said ALS, or a biomarker that identifies progression of ALS, or a combination thereof. In another related aspect, said biomarker comprises chitinase 1 (CHIT1), MCP-1, VEGF, miR-34a, miR-376-a, or miR-132, or any combination thereof.

In one aspect, described herein is a composition comprising a cell population present in an amount therapeutically effective to treat neurodegenerative disease in a subject, said cell population comprising mesenchymal stem cells (MSC) cells that have been induced to secrete at least one neurotrophic factor (NTF), wherein said cell population comprises MSC-NTF cells. In a related aspect, said neurodegenerative disease comprises Amyotrophic Lateral Sclerosis (ALS); frontotemporal dementia (FTD); Parkinson's disease; Multiple System Atrophy (MSA); Huntington's disease; Alzheimer's disease; Rett Syndrome; lysosomal storage diseases; "white matter disease" or glial/demyelination disease, including Sanfilippo, Gaucher disease; Tay Sachs disease (beta hexosaminidase deficiency); multiple sclerosis (MS); Neuromyelitis Optica (NMO); NMO spectrum disease; brain injury or trauma caused by ischemia, accidents, or environmental insult; stroke; cerebral palsy (CP); autism and autism spectrum disorder; spinal cord damage; or ataxia; or any combination thereof.

In another related aspect, said mesenchymal stem cells comprise (a) bone marrow mesenchymal stem cells, adipocyte mesenchymal stem cells, dental pulp mesenchymal stem cells placenta mesenchymal stem cells, synovial membrane mesenchymal stem cells, peripheral blood mesenchymal stem cells, periodontal ligament mesenchymal stem cells, endometrium mesenchymal stem cells, umbilical cord mesenchymal stem cells, or umbilical cord blood mesenchymal stem cells; (b) cells autologous to said subject; or (c) cells allogeneic to said subject; any combination thereof. In another related aspect, said MSC-NTF comprise non-genetically modified human cells; or mesenchymal stem cells induced *ex vivo* to express and secrete at least one neurotrophic factor (NTF); or a combination thereof. In another related aspect, a basal secretion of said at least one NTF is greater in said MSC-NTF cells compared with a basal secretion of said at least one NTF in a non-differentiated, mesenchymal stem cell. In another related aspect, said at least one NTF is selected from the group comprising a vascular endothelial growth factor (VEGF), a hepatocyte growth factor (HGF), a leukemia inhibitory factor (LIF), Granulocyte Stimulating factor (G-CSF), a Brain-derived neurotrophic factor (BDNF), a Tumor necrosis factor-inducible gene 6 protein (TSG-6; also known as TNF-stimulated gene 6 protein), Bone morphogenetic protein 2 (BMP2), Fibroblast Growth Factor 2 (FGF2), or a Neublastin, or any combination thereof.

In one aspect, described herein is a method for detecting CHIT1 in a subject, the method comprising: obtaining a biological sample from said subject; detecting the level of a chitinase 1 (CHIT1) in the sample, wherein the level of said CHIT1 is in the range of 500-300,000 pg/ml.

In a related aspect, said biological sample comprises a cerebrospinal fluid (CSF) sample, a urine sample, a blood sample, or a serum sample, from said subject. In another related aspect, said detecting further comprising detecting MCP-1 in said subject. In another related aspect, said subject has ALS disease and said detecting determines progression of ALS.

In one aspect, described herein is a method for diagnosis amyotrophic lateral sclerosis (ALS) in a subject, the method comprising: obtaining a biological sample from said subject; detecting the level of a chitinase 1 (CHIT1) in the sample, wherein the level of said CHIT1 in the range of 500-300,000 pg/ml indicates that said subject has said ALS disease.

In a related aspect, said biological sample comprises a cerebrospinal fluid (CSF) sample, a urine sample, a blood sample, or a serum sample, from said subject. In another related aspect, said detecting further comprises detecting MCP-1 in said subject, and wherein the level of MCP-1 indicates that said subject has said ALS disease. In another related aspect, said diagnosis determines progression of ALS.

In one aspect, described herein is a method for treating amyotrophic lateral sclerosis (ALS) in a subject, the method comprising: obtaining a biological sample from said subject; detecting the level of a chitinase 1 (CHIT1), wherein the level of said CHIT1 in the range of 1,600-107,600 pg/ml indicates that said subject has said ALS disease; and based on the detection of said CHIT1, treating said ALS in said subject.

In a related aspect, said biological sample comprises a cerebrospinal fluid (CSF) sample, urine, or a blood sample from said subject. In another related aspect, following said treating said method increases the level of VEGF, HGF, LIF, G-CSF, BDNF, TSG-6, miR-34a, miR-132, miR-19, miR376-a, or miR-146a-5p, or any combination thereof in the biological sample of said subject compared with a level in a control biological sample.

In another related aspect, said method decreases the level of CHIT1, CRP, MCP1, SDF-1, Macrophage Inflammatory protein (MIP)-1b, Glutamate, or CASP3, or any combination thereof in the sample said subject compared with the level in control sample. In another related aspect, a lower basal level of miR-34a, miR-376a, or miR-132, or any combination thereof in a biological sample from a subject to be treated compared with a biological sample from a responder patient, indicates a non-responder to MSC-NTF cell treatment.

In one aspect, described herein is a method for modulating a neurotrophic or an inflammatory factor or a pro-apoptotic factor or a factor that influence inflammatory factors in a subject, the method comprising administering to said subject a therapeutically effective amount of a cell population of mesenchymal stem cells (MSC) cells that have been induced to secrete at least one neurotrophic factor (NTF), wherein said cell population comprises MSC-NTF cells, thereby modulating said neurotrophic or an inflammatory factor or a pro-apoptotic factor or a factor that influence inflammatory factors in said subject.

### BRIEF DESCRIPTION OF THE DRAWINGS

The following drawings form part of the present specification and are included to further demonstrate certain embodiments of the present disclosure. The cells and the methods of use thereof, described herein, may be better understood by reference to one or more of these drawings in combination with the detailed description of specific embodiments presented herein.

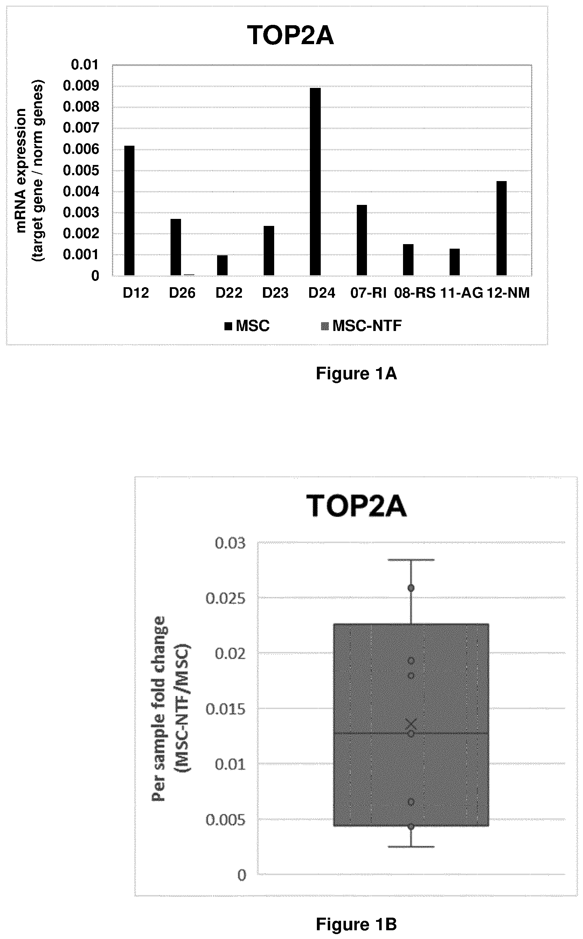

**Figures 1A and 1B. Figure 1A** presents a bar graph showing topoisomerase (DNA) II Alpha (*Top2A*) gene expression in MSC (black) and MSC-NTF (grey) from normal (healthy) donors and from ALS patients. **Figure 1B** presents the sample fold change (MSC-NTF/MSC) for the *Top2A* gene expression. (D12, D26, D22, D23, and D24 are from healthy donors; 07-RI, 08-RS, 11-AG, and 12-NM are from ALS patients).

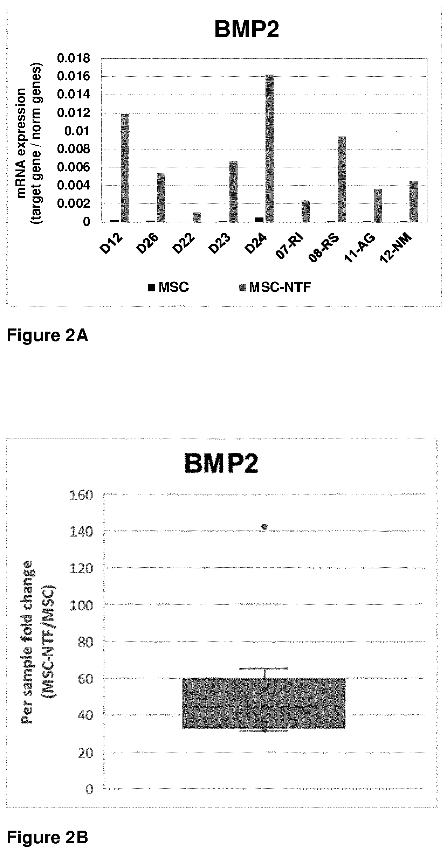

**Figures 2A and 2B. Figure 2A** presents a bar graph showing Bone morphogenetic protein *2* (*BMP2*) gene expression in MSC (black) and MSC-NTF (grey) from normal (healthy) donors and from ALS patients. **Figure 2B** presents the sample fold change (MSC-NTF/MSC) for the *BMP2* gene expression. (D12, D26, D22, D23, and D24 are from healthy donors; 07-RI, 08-RS, 11-AG, and 12-NM are from ALS patients).

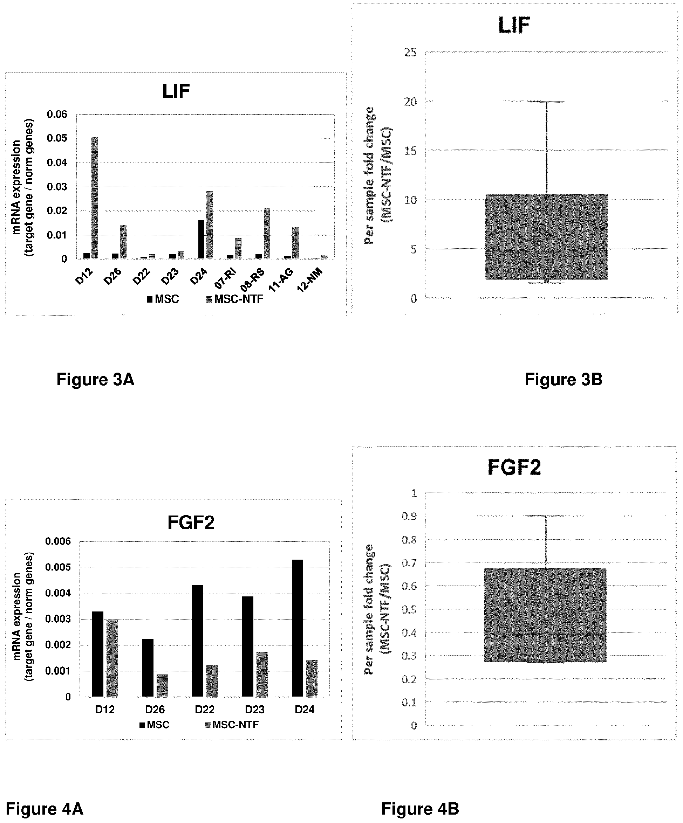

**Figures 3A and 3B. Figure 3A** presents a bar graph showing Leukemia inhibitory factor (*LIF*) gene expression in MSC (black) and MSC-NTF (grey) from normal (healthy) donors and from ALS patients. **Figure 3B** presents the sample fold change (MSC-NTF/MSC) for the *LIF* gene expression. (D12, D26, D22, D23, and D24 are from healthy donors; 07-RI, 08-RS, 11-AG, and 12-NM are from ALS patients).
**Figures 4A and 4B. Figure 4A** presents a bar graph showing Fibroblast Growth Factor 2 (*FGF2*) gene expression in MSC (black) and MSC-NTF (grey) from normal (healthy) donors. **Figure 4B** presents the sample fold change (MSC-NTF/MSC) for the *FGF2* gene expression. (D12, D26, D22, D23, and D24 are from healthy donors).

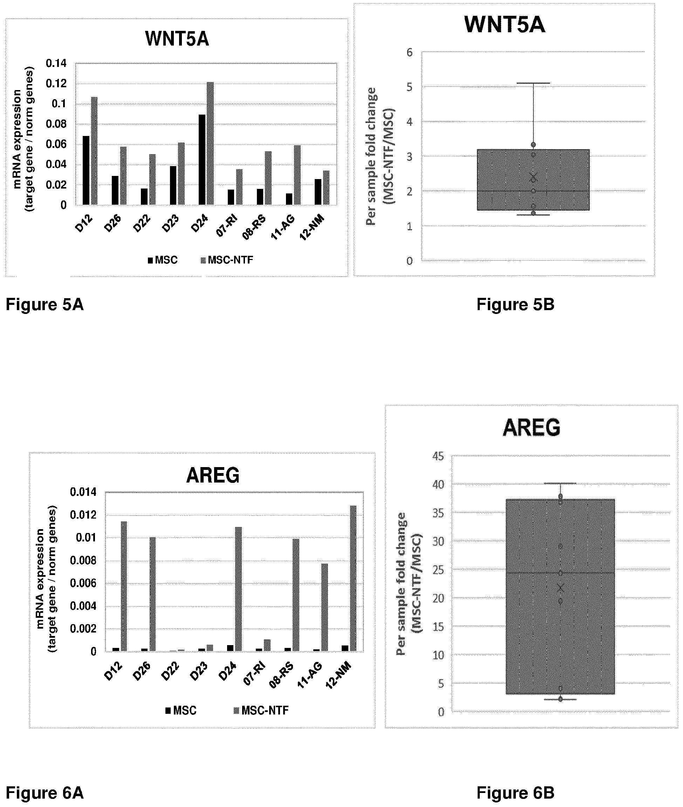

**Figures 5A and 5B. Figure 5A** presents a bar graph showing Wnt Family Member 5A (*WNT5A*) gene expression in MSC (black) and MSC-NTF (gray) from normal (healthy) donors and from ALS patients. **Figure 5B** presents the sample fold change (MSC-NTF/MSC) for the *WNT5A* gene expression. (D12, D26, D22, D23, and D24 are from healthy donors; 07-RI, 08-RS, 11-AG, and 12-NM are from ALS patients).
**Figures 6A and 6B. Figure 6A** presents a bar graph showing Amphiregulin *(AREG)* gene expression in MSC (black) and MSC-NTF (gray) from normal (healthy) donors and from ALS patients. **Figure 6B** presents the sample fold change (MSC-NTF/MSC) for the *AREG* gene expression. (D12, D26, D22, D23, and D24 are from healthy donors; 07-RI, 08-RS, 11-AG, and 12-NM are from ALS patients).

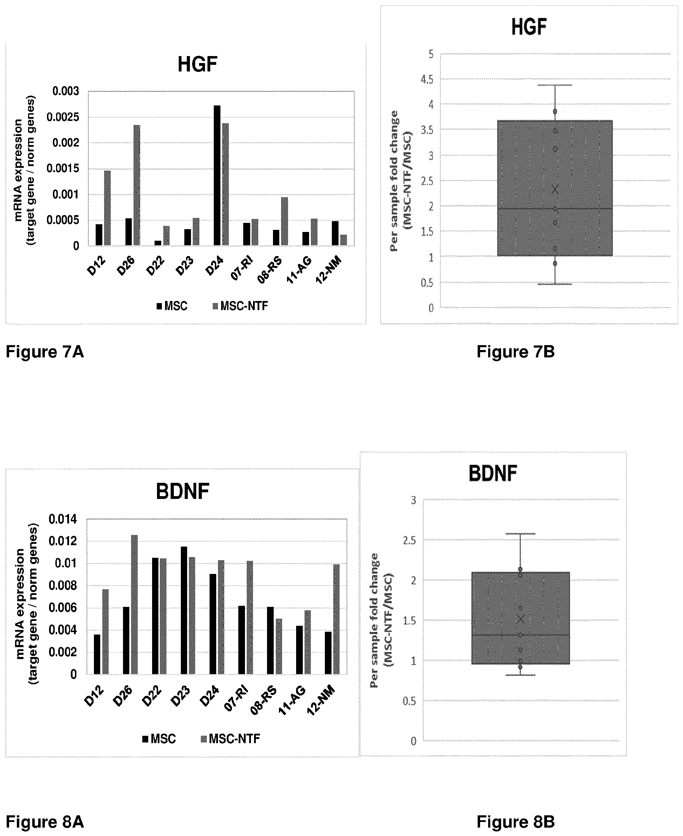

**Figures 7A and 7B. Figure 7A** presents a bar graph showing Hepatocyte Growth factor *(HGF)* gene expression in MSC (black) and MSC-NTF (gray) from normal (healthy) donors and from ALS patients. **Figure 7B** presents the sample fold change (MSC-NTF/MSC) for the *HGF* gene expression. (D12, D26, D22, D23, and D24 are from healthy donors; 07-RI, 08-RS, 11-AG, and 12-NM are from ALS patients).
**Figures 8A and 8B. Figure 8A** presents a bar graph showing Brain Derived Neurotrophic Factor *(BDNF)* gene expression in MSC (black) and MSC-NTF (gray) from normal (healthy) donors and from ALS patients. **Figure 8B** presents the sample fold change (MSC-NTF/MSC) for the *BDNF* gene expression. (D12, D26, D22, D23, and D24 are from healthy donors; 07-RI, 08-RS, 11-AG, and 12-NM are from ALS patients).

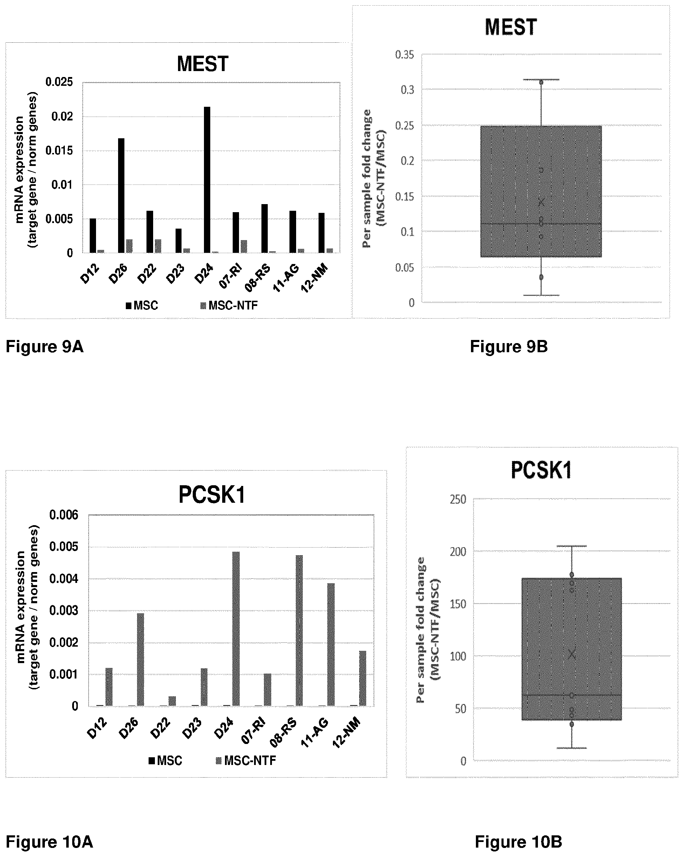

**Figures 9A and 9B. Figure 9A** presents a bar graph showing Mesoderm Specific Transcript (*MEST*) gene expression in MSC (black) and MSC-NTF (gray) from normal (healthy) donors and from ALS patients. **Figure 9B** presents the sample fold change (MSC-NTF/MSC) for the *MEST* gene expression. (D12, D26, D22, D23, and D24 are from healthy donors; 07-RI, 08-RS, 11-AG, and 12-NM are from ALS patients).
**Figures 10A and 10B. Figure 10A** presents a bar graph showing Proprotein Convertase Subtilisin/Kexin Type 1 (*PCSK1*) gene expression in MSC (black) and MSC-NTF (gray) from normal (healthy) donors and from ALS patients. **Figure 10B** presents the sample fold change (MSC-NTF/MSC) for the *PCSK1* gene expression. (D12, D26, D22, D23, and D24 are from healthy donors; 07-RI, 08-RS, 11-AG, and 12-NM are from ALS patients).

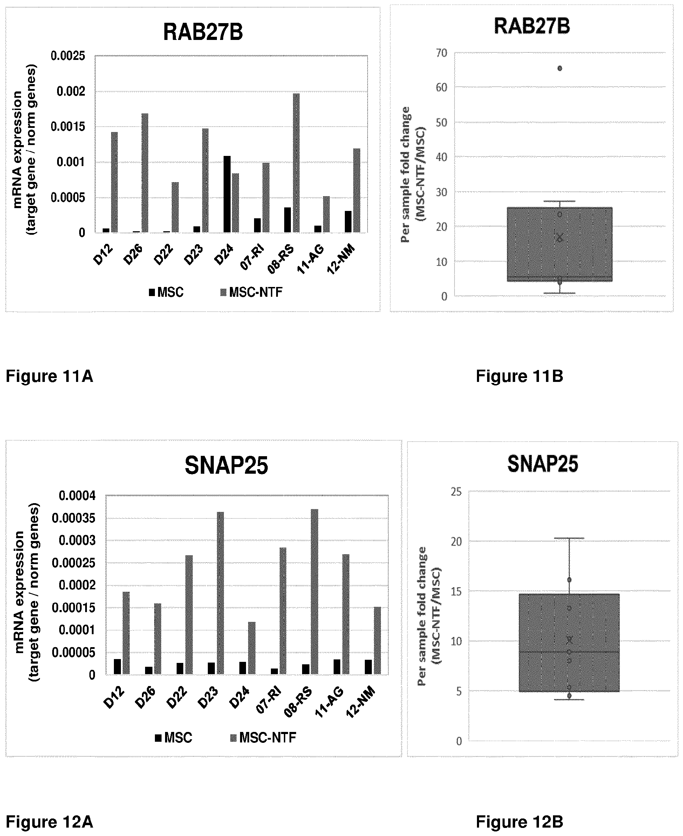

**Figures 11A and 11B. Figure 11A** presents a bar graph showing RAB27B, Member RAS Oncogene Family *(RAB27b)* gene expression in MSC (black) and MSC-NTF (gray) from normal (healthy) donors and from ALS patients. **Figure 11B** presents the sample fold change (MSC-NTF/MSC) for the *RAB27b* gene expression. (D12, D26, D22, D23, and D24 are from healthy donors; 07-RI, 08-RS, 11-AG, and 12-NM are from ALS patients).
**Figures 12A and 12B. Figure 12A** presents a bar graph showing Synaptosome Associated Protein 25 (*SNAP25*) gene expression in MSC (black) and MSC-NTF (gray) from normal (healthy) donors and from ALS patients. **Figure 12B** presents the sample fold change (MSC-NTF/MSC) for the *SNAP25* gene expression. (D12, D26, D22, D23, and D24 are from healthy donors; 07-RI, 08-RS, 11-AG, and 12-NM are from ALS patients).

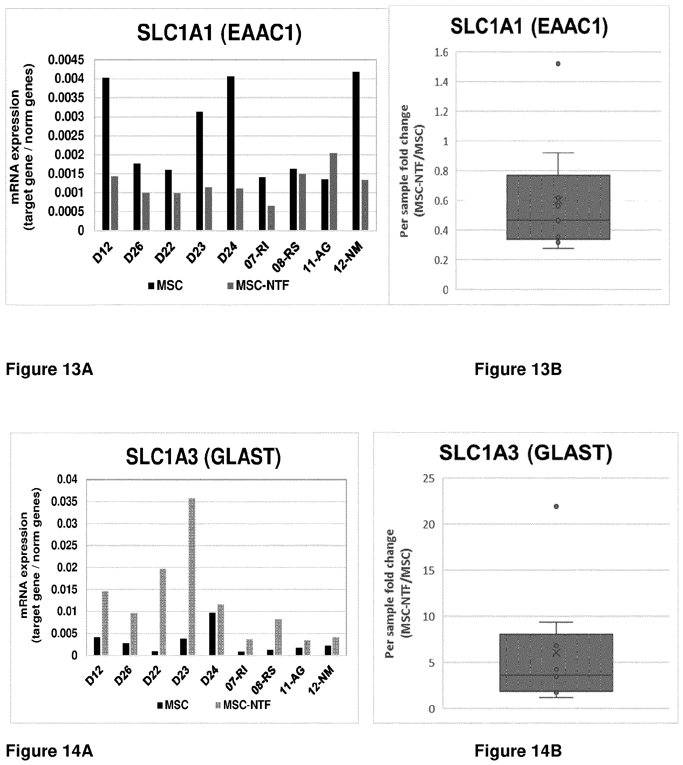

**Figures 13A and 13B. Figure 13A** presents a bar graph showing Solute Carrier Family 1 Member 1 (*SLC1A1* (*EAAC1*) gene expression in MSC (black) and MSC-NTF (gray) from normal (healthy) donors and from ALS patients. **Figure 13B** presents the sample fold change (MSC-NTF/MSC) for the *SLC1A1 (EAAC1)* gene expression. (D12, D26, D22, D23, and D24 are from healthy donors; 07-RI, 08-RS, 11-AG, and 12-NM are from ALS patients).
**Figures 14A and 14B. Figure 14A** presents a bar graph showing Solute Carrier Family 1 Member 3 (*SLC1A3 (GLAST)*) gene expression in MSC (black) and MSC-NTF (gray) from normal (healthy) donors and from ALS patients. **Figure 14B** presents the sample fold change (MSC-NTF/MSC) for the *SLC1A3 (GLAST)* gene expression. (D12, D26, D22, D23, and D24 are from healthy donors; 07-RI, 08-RS, 11-AG, and 12-NM are from ALS patients).

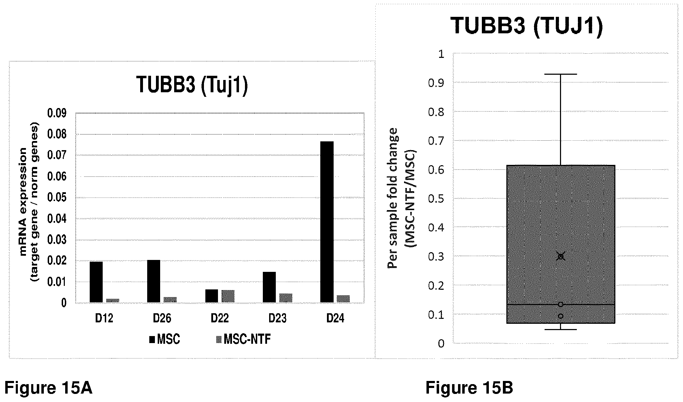

**Figures 15A and 15B. Figure 15A** presents a bar graph showing Tubulin Beta 3 Class III (*TUBB3 (TUJ1)*) gene expression in MSC (black) and MSC-NTF (gray) from normal (healthy) donors. **Figure 15B** presents the sample fold change (MSC-NTF/MSC) for the *TUBB3 (TUJ1)* gene expression. (D12, D26, D22, D23, and D24 are from healthy donors).

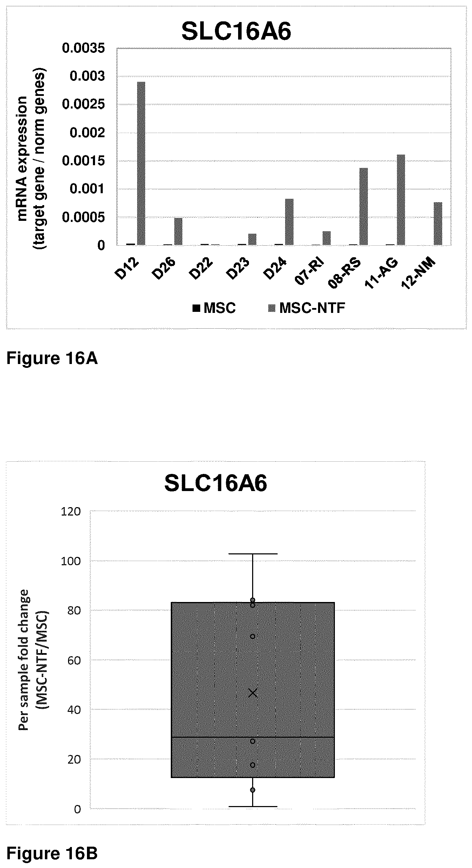

**Figures 16A and 16B. Figure 16A** presents a bar graph showing Solute Carrier Family 16 Member 6 *(SLC16A6)* gene expression in MSC (black) and MSC-NTF (gray) from normal (healthy) donors. **Figure 16B** presents the sample fold change (MSC-NTF/MSC) for the *SLC16A6* gene expression. (D12, D26, D22, D23, and D24 are from healthy donors, 07-RI, 08-RS, 11-AG, and 12-NM are from ALS patients).

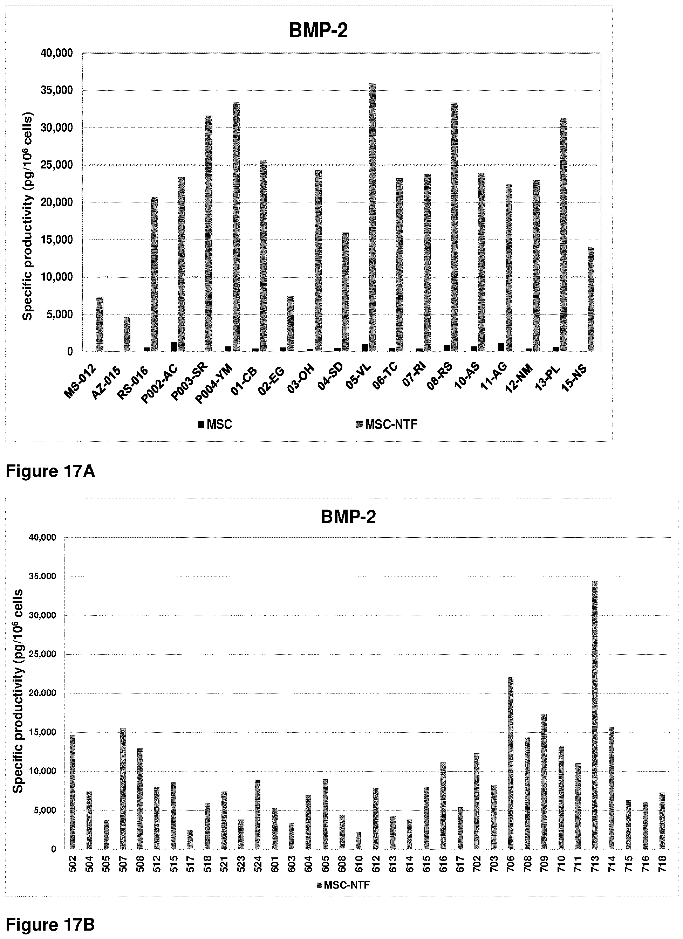

**Figures 17A and 17B. Figure 17A** shows comparative results of Bone morphogenetic protein 2 polypeptide (BMP-2) secretion from MSC (black) and MSC-NTF (gray), as measured in the culture supernatant, from cells obtained from ALS patients who participated in the Phase 2a clinical trial (Clinicaltrials.gov identifier: NCT01777646). **Figure 17B** shows Bone morphogenetic protein 2 polypeptide (BMP-2) secretion results from MSC-NTF, as measured in the culture supernatant, from cells obtained from ALS patients who participated in a Phase 2 clinical trial (ClinicalTrials.gov Identifier: NCT02017912) described in Example 3 herein.

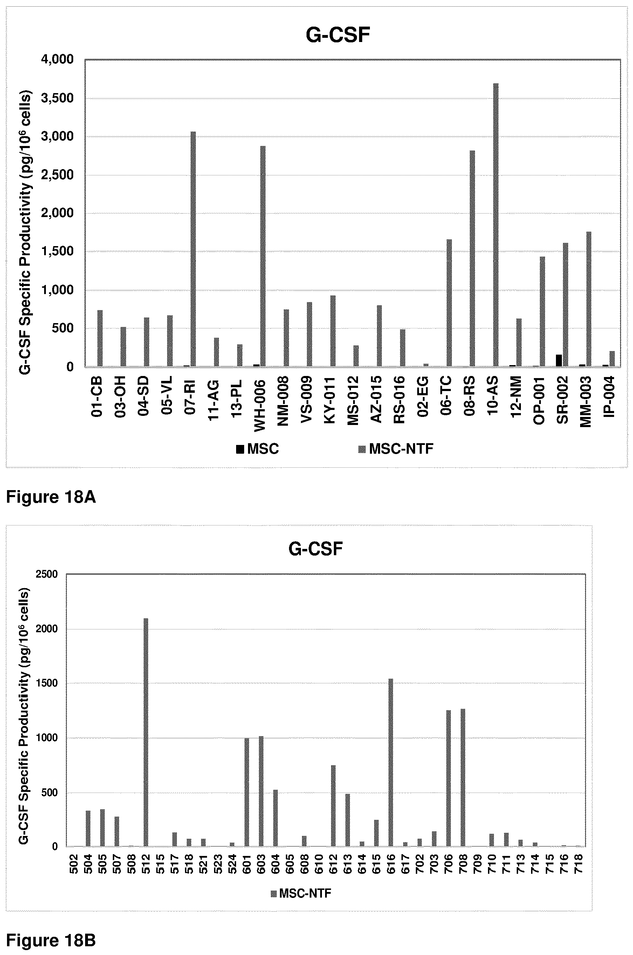

**Figures 18A and 18B. Figure 18A** shows comparative results of Colony Stimulating Factor 3 polypeptide (G-CSF) secretion from MSC (black) and MSC-NTF (gray), as measured in the culture supernatant, from cells obtained from ALS patients who participated in the Phase 2a clinical trial (Clinicaltrials.gov identifier: NCT01777646). **Figure 18B** shows Colony Stimulating Factor 3 polypeptide (G-CSF) secretion results from MSC-NTF, as measured in the culture supernatant, from cells obtained from ALS patients who participated in the United States clinical trial described in Example 3 herein.

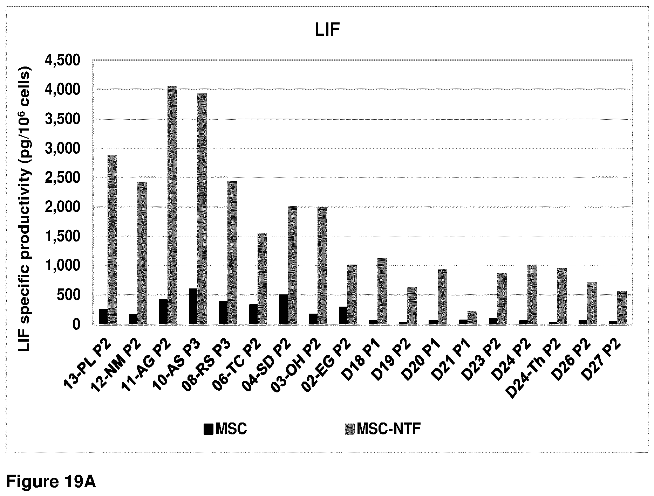

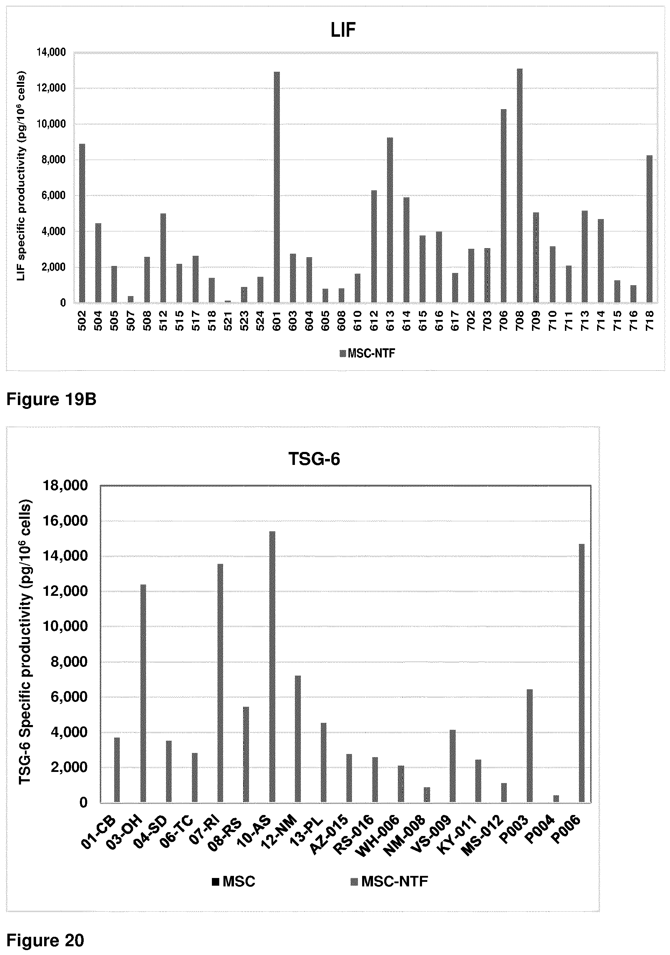

**Figures 19A** **and** **19B****.** **Figure 19A** shows comparative results of Leukemia Inhibitory Factor polypeptide (LIF) secretion from MSC (black) and MSC-NTF (gray), as measured in the culture supernatant, from cells obtained from ALS patients who participated in the Phase 2a clinical trial (Clinicaltrials.gov identifier: NCT01777646). **Figure 19B** shows Leukemia Inhibitory Factor polypeptide (LIF) secretion results from MSC-NTF, as measured in the culture supernatant, from cells obtained from ALS patients who participated in the Phase 2 clinical trial (ClinicalTrials.gov Identifier: NCT02017912) described in Example 3 herein.
**Figure 20** shows the results of Tumor necrosis factor (TNF)-stimulated gene-6 (TSG-6) secretion from MSC (black) and MSC-NTF (gray), as measured in the culture supernatant, from cells obtained from ALS patients who participated in the Phase 2a clinical trial (Clinicaltrials.gov identifier: NCT01777646).

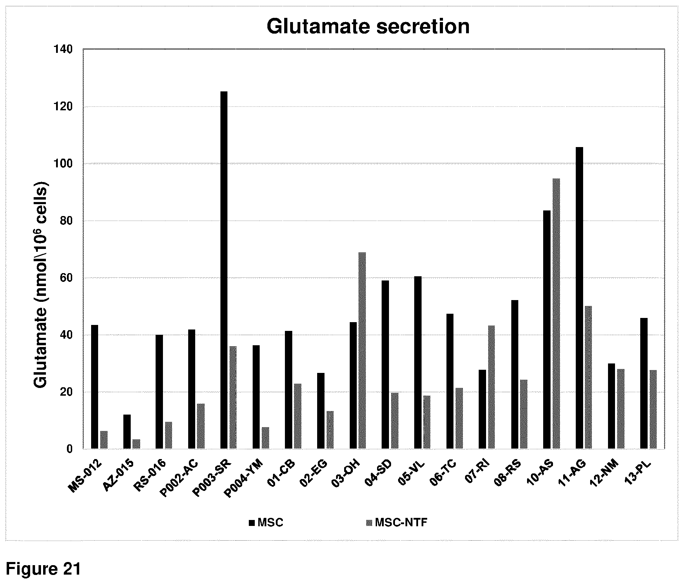

**Figure 21** shows the results of Glutamate secretion from MSC (black) and MSC-NTF (gray), as measured in the culture supernatant, from cells obtained from ALS patients in the Phase 2a clinical trial (Clinicaltrials.gov identifier: NCT01777646).

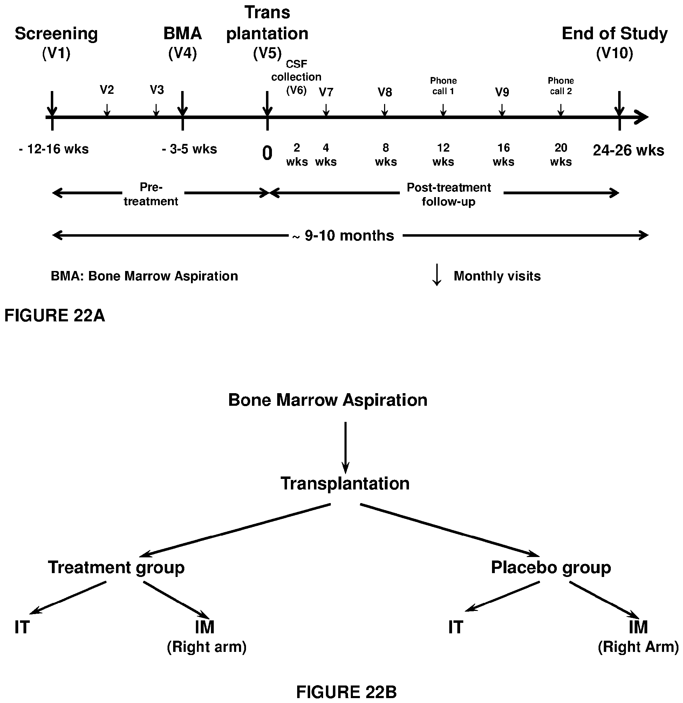

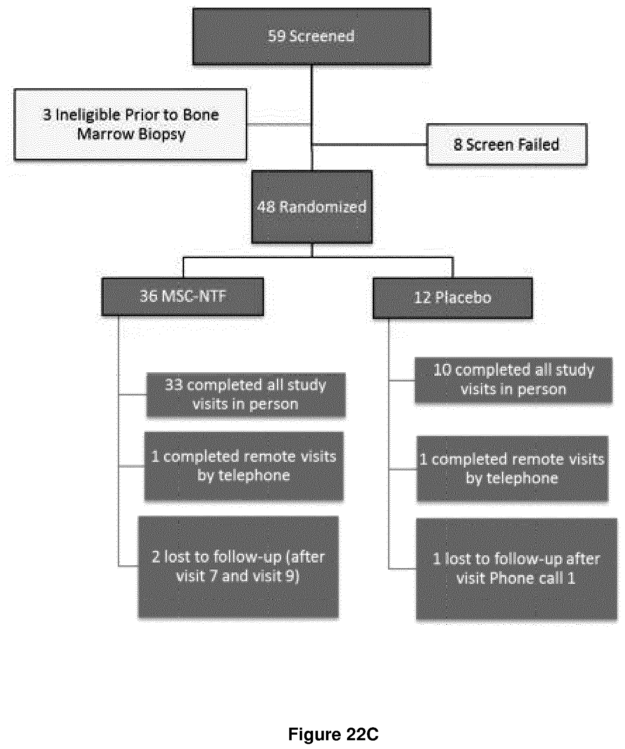

**Figures 22A, 22B****, and** **22C****.** **Figure 22A** illustrates the study schematic (Clinical Trial design), according to an embodiment disclosed herein. Visit 1 (V1) as a screening visit. Visit 2 (V2) occurred at 4-6 weeks following V1, and was a pre-transplantation visit. Visit 3 (V3) occurred at 8-10 weeks following V1, and was a 2^{nd} pre-transplantation visit. Visit 4 (V4) occurred at 9-11 weeks following V1, and was for bone marrow aspiration. Visit 5 (V5) occurred on day 0 (24-38 days from bone marrow aspiration and was for cell transplantation (treatment). Visits 6 (V6) through visits 10 (V10) occurred on a regular basis up-through weeks 24-26 from transplantation. **Figure 22B****.** Presents a study groups flowchart delineating placebo and cell transplantation in the treatment and placebo groups. **Figure 22C** presents a flow chart illustrating the participant enrollment, intervention allocation, and follow-up for the trial.

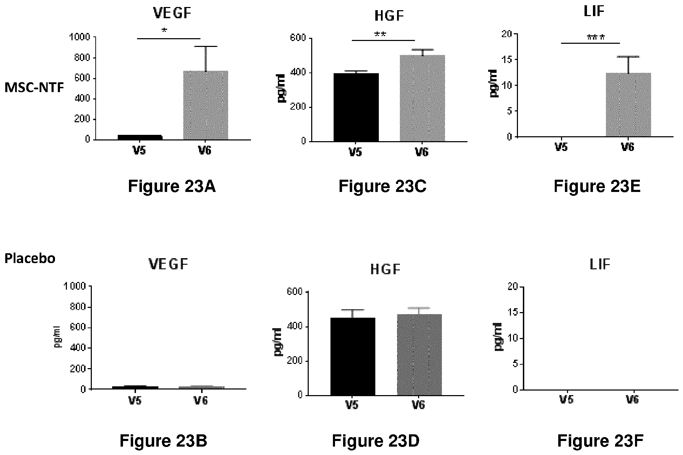

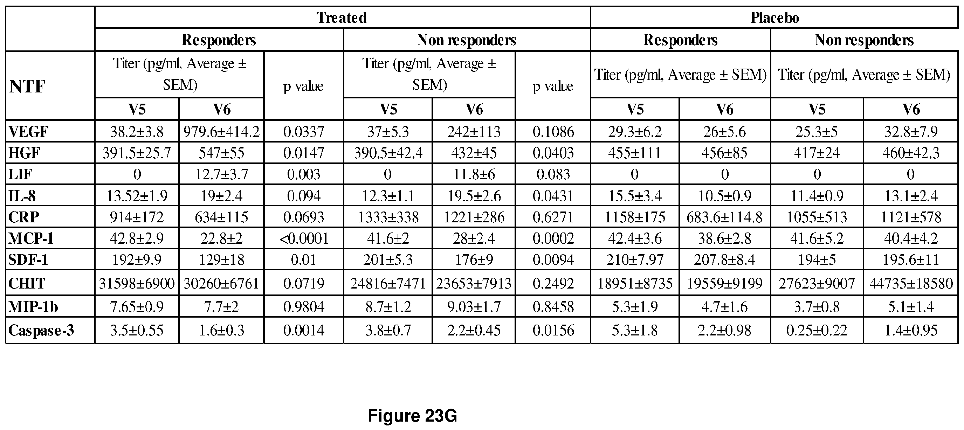

**Figures 23A-23G****.** Presents CSF analysis measurements of neurotrophic factors pre-transplantation (V5) and two weeks post-transplantation (V6). Significant increase in CSF Vascular endothelial growth factor (VEGF) (**Figures 23A and 23B**), Hepatocyte Growth Factor (HGF) (**Figures 23C and 23D**), and Leukemia Inhibitory Factor (LIF) (**Figures 23E and 23F**) titers in patients treated with MSC-NTF cells with, no change in patients treated with placebo. Responders to treatment was defined as those patients having a 50% improvement post-transplant as compared to pre-transplant ALSFRS-R slope at 8 weeks post-transplant. * p< 0.05 ** p<0.01 *** p< 0.001. **Figure 23G** presents a Table showing the change in titers of neurotrophic and inflammatory factors in the CSF of patients responding to MSC-NTF cells treatment.

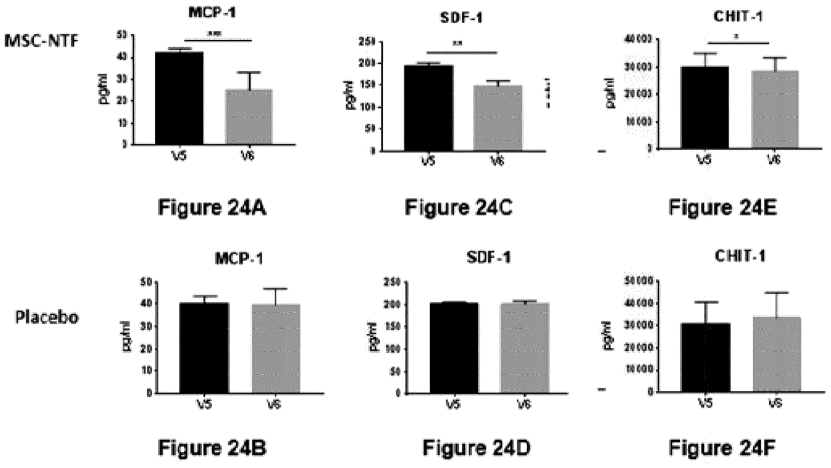

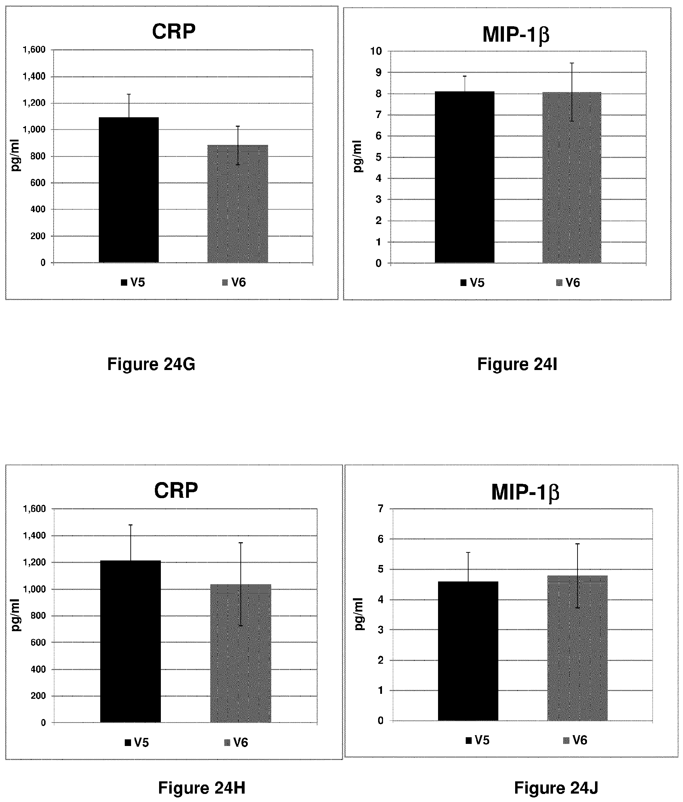

**Figures 24A-J**. Presents CSF analysis measurements of inflammatory biomarkers pre-transplantation (V5) and two weeks post-transplantation (V6). A significant decrease in MCP-1 (**Figures 24A and 24B**), SDF-1 (**Figures 24C and 24D**), and CHIT-1 levels (**Figures 24E and 24F**), is shown in the CSF of the MSC-NTF treated patients (upper panels) with no significant change in the placebo group (lower panels). * p< 0.05 ** p<0.01 *** p< 0.001. In addition, modulation of inflammatory markers CRP (**Figures 24G and 24H**) and MIP-1β (**Figures 24I and 24J**) was analyzed. (**Figures 24A. 24C, 24E****,** **24G, and 24I** were MSC-NTF treated; **Figures 24B, 24D, 24F****,** **24H, and 24J** were placebo treated)

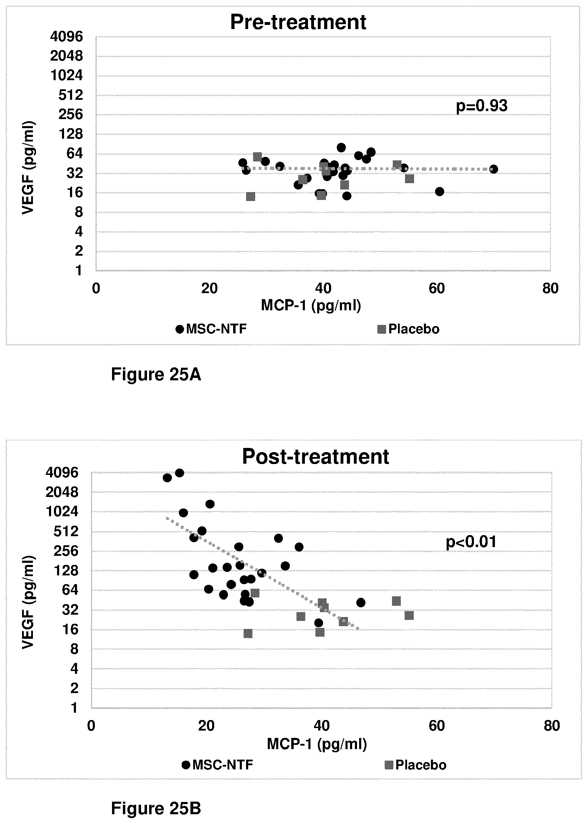

**Figures 25A and 25B****.** Correlation between the increase in VEGF and the decrease in MCP-1 in the CSF post treatment with MSC-NTF cells. A significant correlation between VEGF increase and MCP-1 decrease is shown in the CSF of the MSC-NTF treated patients at visit 6 two weeks post-transplantation (**Figure 25A**) with no significant change in the placebo group (**Figure 25B**). No correlation was seen between VEGF and MCP-1 levels prior to treatment (V5).

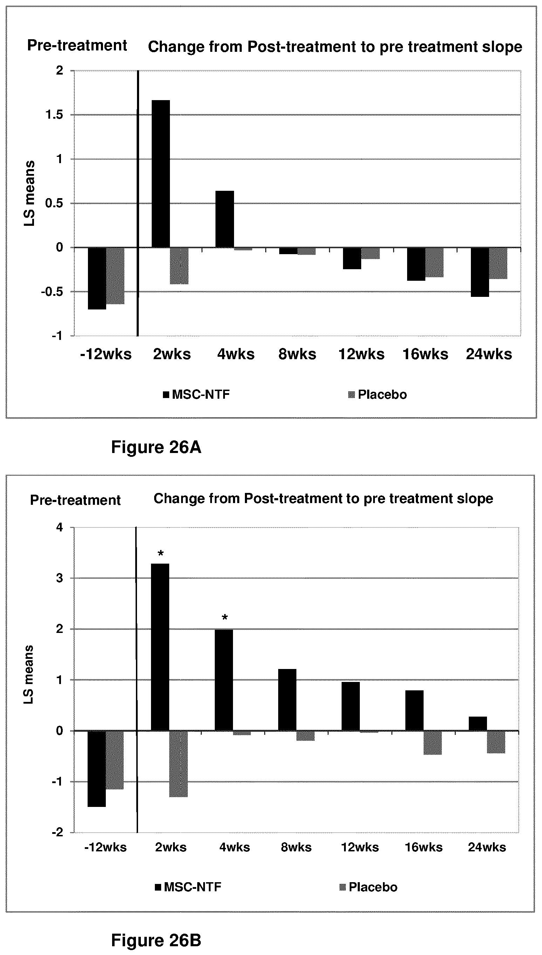

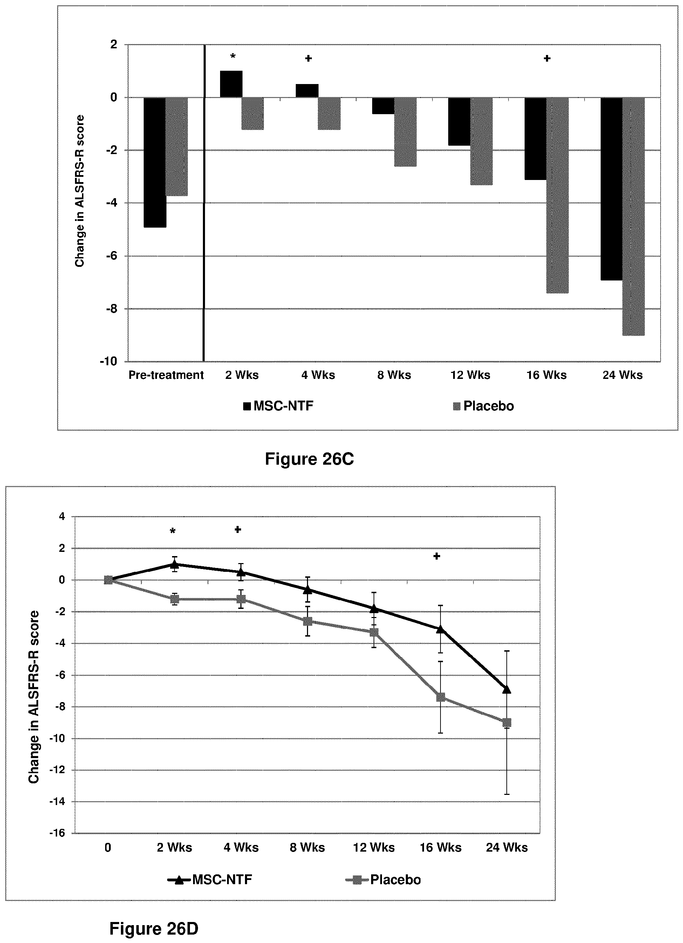

**Figures 26A, 26B****,** **26C,** and **26D****.** Presents mean change in ALSFRS-R slope over time results. Least Square means of the 12 weeks pre-treatment slope (-12wks) for the MSC-NTF cells group (black) and the placebo group (gray) and the mean change in slope (post-treatment minus pre-treatment) for each of the post-treatment time points for the total population (**Figure 26A**) and excluding slow progressors (defined as those participants with a pre-treatment ALSFRS-R change ≥ -2 between screening and baseline) (**Figure 26B**). The difference between the treated and placebo groups was statistically significant at the 2 and 4 week time points (p= 0.021, and 0.033, respectively, indicated by a * for p<0.05). **Figure 26C** presents LS Mean Change from Baseline in ALSFRS-R Score by Week - Excluding Slow Progressors (Change from V1 [Screening] to V5 [Baseline] of ≥ -2 points) Abbreviations: ALSFRS-R= ALS Functional Rating Scale -Revised, wks=Weeks. The difference between the treated and placebo groups was statistically significant at the 2, 4, and 16-week time points (p= 0.0248, 0.1031, and 0.1493, respectively, indicated by a * or +, using * for p < 0.05 or a + for p < 0.2 two-sided). **Figure 26D** presents changes in the in ALSFRS-R Score by Week - Excluding Slow Progressors (Change from V1 [Screening] to V5 [Baseline] of ≥ -2 points). Abbreviations: ALSFRS-R: ALS Functional Rating Scale -Revised. Line plots above display mean changes from baseline to each time point. Bars at each time point represent standard errors of the mean (SE). The difference between the treated and placebo groups was statistically significant at the 2, 4, and 16-week time points (p= 0.0248, 0.1031, and 0.1493, respectively, indicated by a * or +, using * for p < 0.05 or a + for p < 0.2 two-sided)

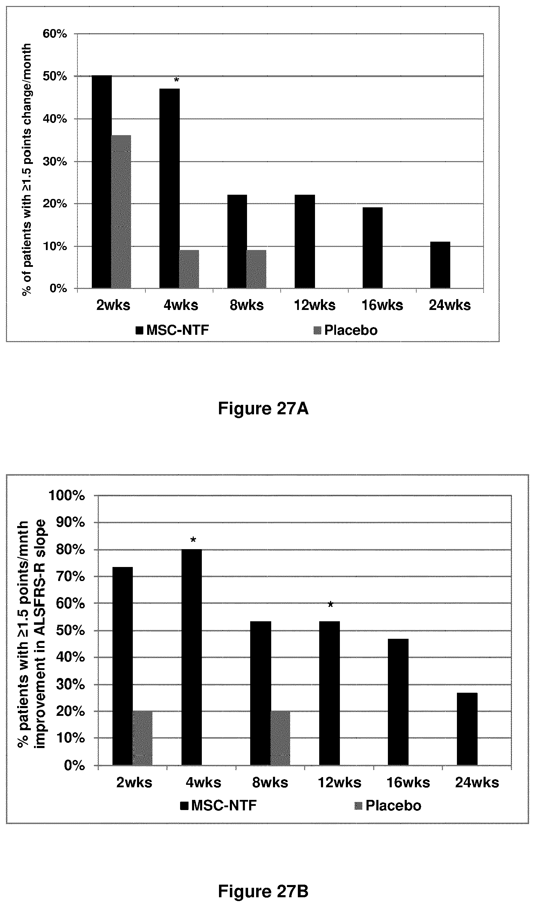

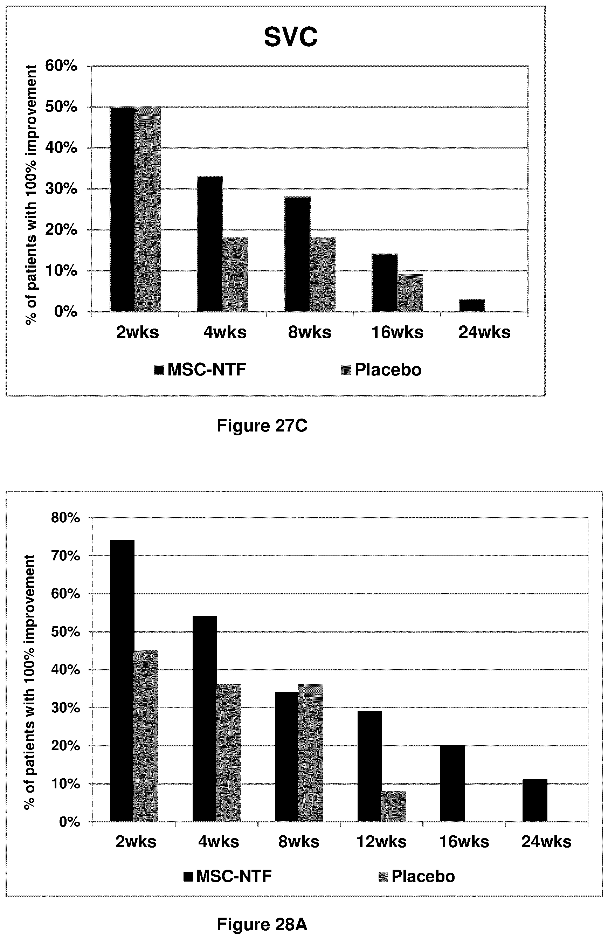

**Figures 27A, 27B****, and** **27C****.** Responder analyses: ≥1.5 point ALSFRS-R slope improvement over the post treatment follow up period. The percentage of participants with a ≥ 1.5-point improvement in the ALSFRS-R slope at the indicated time points as compared to their pre-treatment slope over the ∼12 weeks pre-treatment period in the treated (MSC-NTF) and the placebo group total population (**Figure 27A**) and excluding slow progressors (defined as participants with a pre-treatment ALSFRS-R change ≥ -2 between screening and baseline) (**Figure 27B**). **Figure 27C** shows the changes in (slow vital capacity) SVC score for the ≥100% Improvement responder definition.

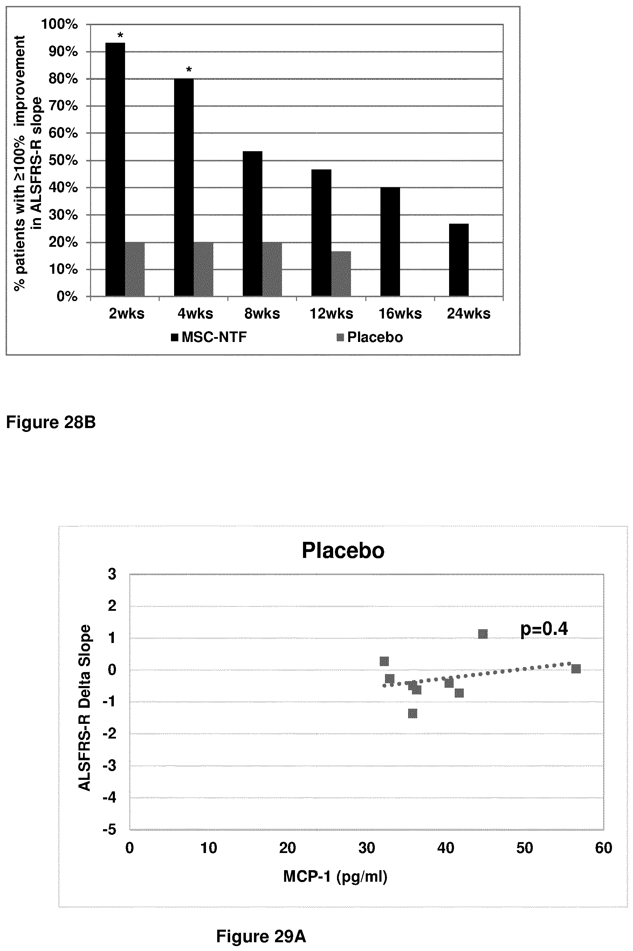

**Figures 28A** **and** **28B****.** Responder analyses: 100% ALSFRS-R slope improvement over the post treatment follow-up period. The percentage of participants with a 100% improvement in their ALSFRS-R at the indicated time points as compared to their pre-treatment slope over the -12 weeks pre-treatment period in the treated (MSC-NTF) and the Placebo group total population (**Figure 28A**) and excluding slow progressors (defined as those participants with a pre-treatment ALSFRS-R change ≥ -2 between screening and baseline, **Figure 28B**). Excluding slow progressors the differences between the treated and placebo groups was statistically significant at the 2 and 4weekstimepoint (p= 0.005 and 0.031 respectively, indicated by a * for p<0.05)

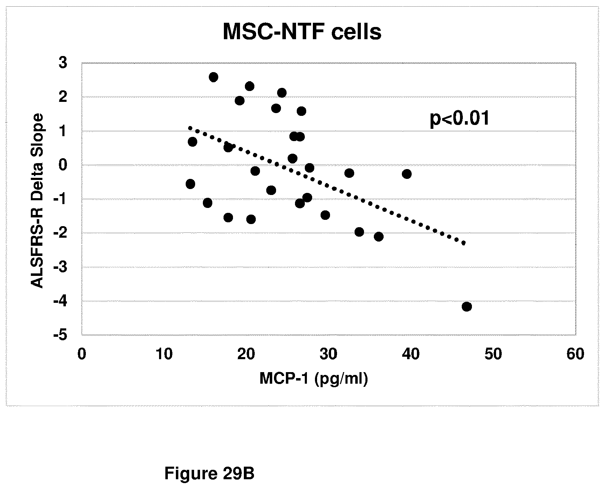

**Figures 29A** **and** **29B****.** A significant correlation between MCP-1 in the CSF at two weeks post treatment (visit 6, **Figure 29B**) and a slower disease progression at 12 weeks post-treatment, with no significant change in the placebo group (**Figure 29A**).

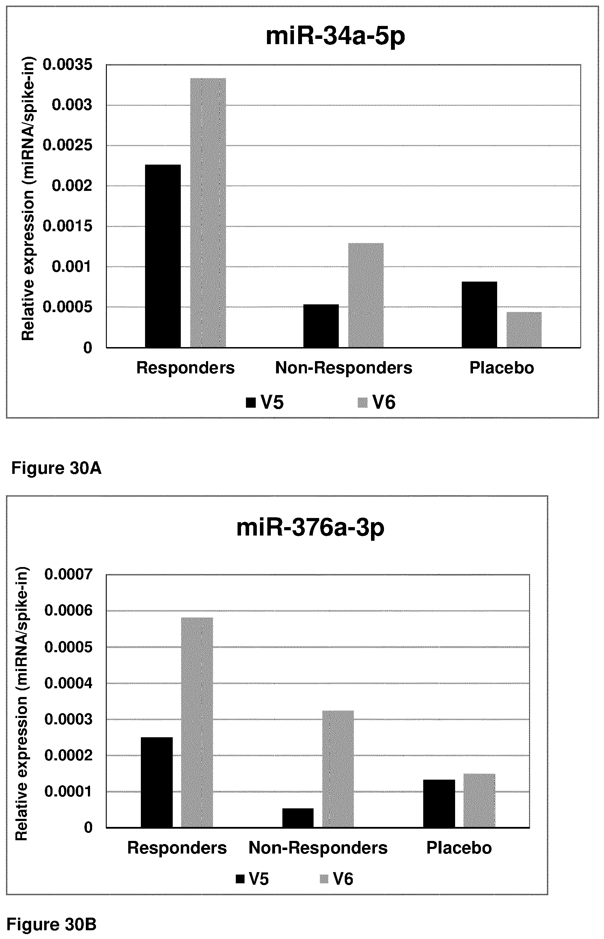

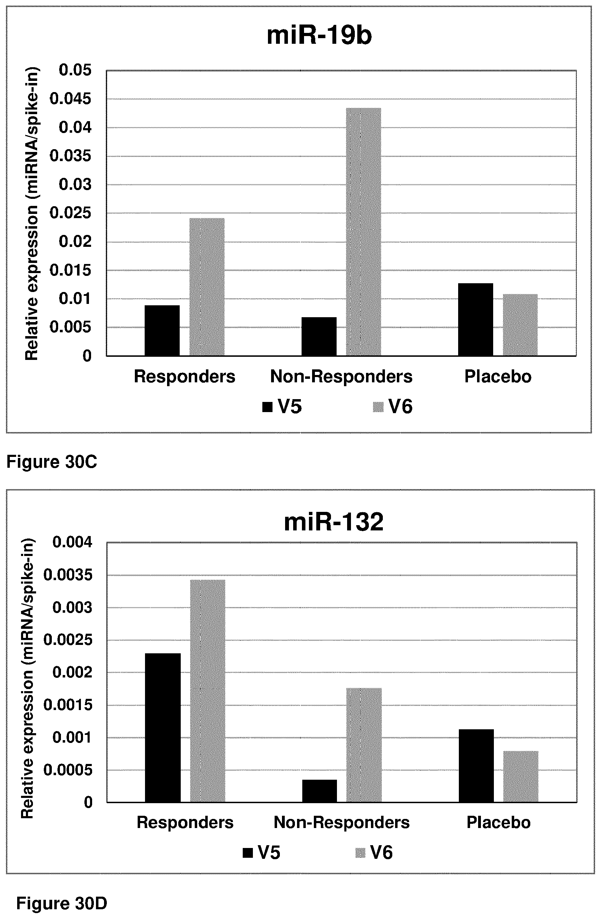

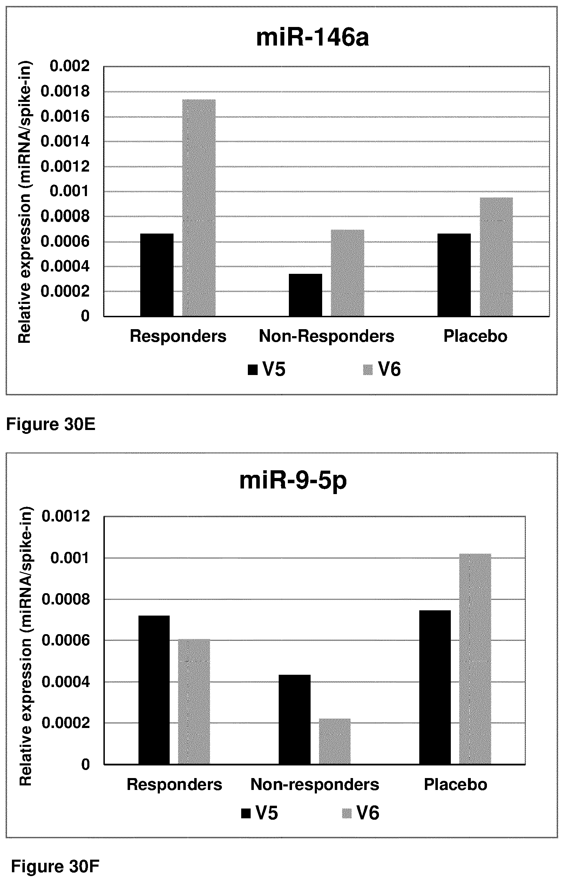

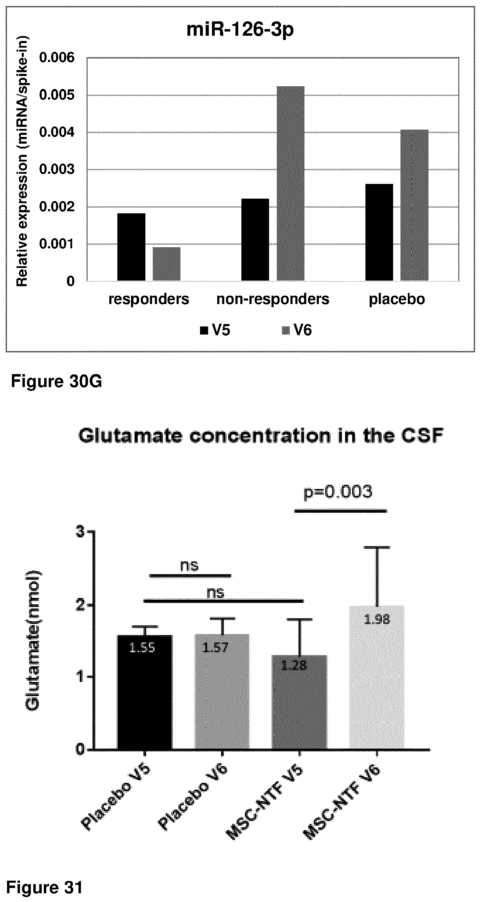

**Figures 30A-30G****.** Pooled CSF from responder patients (n=6), non-responder patients (n=9) and placebo patients (n=6), was sampled pre-transplantation (V5) and two weeks post-transplantation (V6), and specific miRNAs measured. **Figure 30A** (miR-34a-5p); **Figure 30B** (miR-376a-3p); **Figure 30C** (miR-19b); Figure **30D** (miR-132); Figure **30E** (miR-146a); Figure **30F** (miR-9-5p); and **Figure 30G** (miR-126-3p).
**Figure 31****.** Bar Graph showing significant elevation of glutamate in the CSF of patients treated with MSC-NTF compared with controls.

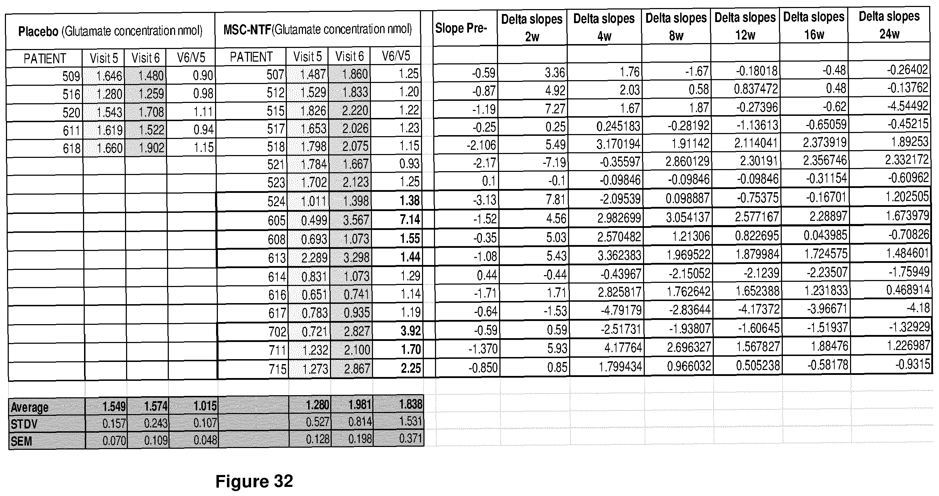

**Figure 32****.** Table showing changes in glutamate levels in the CSF post treatment with MSC-NTF versus change in ALSFRS-R slope.

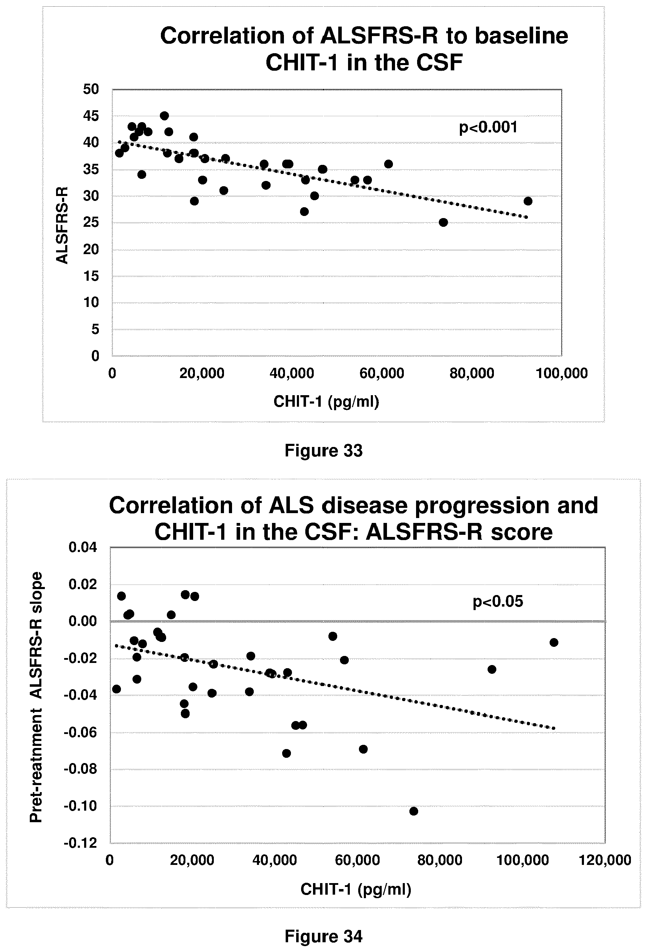

**Figure 33****.** Inverse correlation between ALSFRS-R score at visit 5 (pre-treatment) and CHIT-1 levels in the CSF. The correlation between the ALSFRS-R score and CHIT levels in the CSF is statistically significant (p<0.001).
**Figure 34****.** Inverse correlation between ALS disease progression and CHIT-1 levels in the CSF. ALS disease progression as determined by the slope in ALSFRS-R score in the three months prior to treatment. Slow disease progression (positive or slightly negative slope) is correlated to low levels of CHIT-1 (p<0.05).

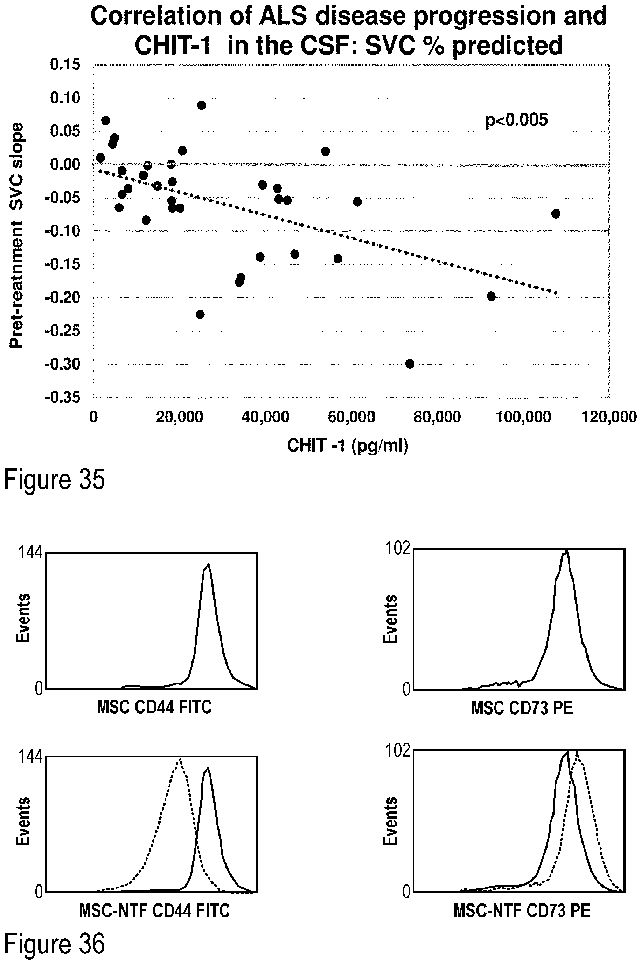

**Figure 35****.** Inverse correlation between ALS disease progression as determined by SVC and CHIT-1 levels in the CSF. ALS disease progression as determined by the slope in SVC (%predicted) in the three months prior to treatment. Slow disease progression (positive or slightly negative slope) is correlated to low levels of CHIT-1 (p<0.005).
**Figure 36****.** Presents CD44 and CD73 expression in AD-MSC and AD-MSC-NTF cells. Left panels: CD44 - Black histogram represents AD-MSC, green histogram represents AD-MSC-NTF. Right panels: CD73 - Black histogram represents AD-MSC, red histogram represents AD-MSC-NTF.

### DETAILED DESCRIPTION

Described herein are mesenchymal stem cells (MSC) and populations thereof which may be used for treating neurodegenerative diseases, including amyotrophic lateral sclerosis (ALS) disease. Specifically, described are methods of treating a neurodegenerative disease, for example ALS, by administering a differentiated mesenchymal stem cell capable of secreting a neurotrophic factor (NTF).

### Mesenchymal Stem Cells and Compositions thereof

The term "mesenchymal stem cell" "mesenchymal stromal cell", "Multipotent Stromal Cells", or "MSC" is used interchangeably for adult cells which are not terminally differentiated, which can divide to yield cells that are either stem cells, or which, irreversibly differentiate to give rise to cells of a mesenchymal cell lineage or transdifferentiate into cells of other non-mesodermal lineages such as the neural lineage. In one embodiment, MSC comprise autologous cells. In an alternative embodiment, MSC comprise allogeneic cells. In some embodiment, MSC cells are transdifferentiated into non-mesenchymal lineage cells. In some embodiments, MSC cells are differentiated into mesenchymal lineage cells.

Mesenchymal stem cells (MSC) can be found in nearly all tissues and may be isolated from various tissues. Although bone marrow (BM) is the most widely recognized source of MSCs, recent research has identified) alternative sources of MSC-like cells, including adipose tissue (AT), placenta, dental pulp, synovial membrane, peripheral blood, periodontal ligament, endometrium, umbilical cord (UC), and umbilical cord blood (UCB). In fact, evidence has suggested that MSCs may be present virtually in any vascularized tissues throughout the whole body. In some embodiments, MSC described herein were isolated from any tissue in which they are present. In some embodiments, the tissue from which MSC may be isolated includes but is not limited to bone marrow, adipose tissue, placenta, dental pulp, synovial membrane, peripheral blood, periodontal ligament, endometrium, umbilical cord, and umbilical cord blood.

A method of isolating mesenchymal stem cells from peripheral blood is described by Kassis et al (Bone Marrow Transplant. 2006 May; 37(10):967-76). A method of isolating mesenchymal stem cells from placental tissue is described by Zhang et al (Chinese Medical Journal, 2004, 117 (6):882-887). Methods of isolating and culturing adipose tissue, placental and cord blood mesenchymal stem cells are described by Kern et al (Stem Cells, 2006; 24:1294-1301). In some embodiments, any method known in the art may be used for isolating mesenchymal stem cells from a tissue.

It will be appreciated that the MSC described herein may be derived from any stem cell. In one embodiment, MSC cells comprise bone marrow MSC. In another embodiment, MSC cells comprise adipocyte MSC. In another embodiment, MSC cells comprise dental pulp mesenchymal stem cells. In another embodiment, MSC cells comprise mesenchymal stem cells obtained from tendon. In another embodiment, MSC cells comprise mesenchymal stem cells obtained from placenta. In another embodiment, MSC cells comprise mesenchymal stem cells obtained from umbilical cord. In another embodiment, MSC cells comprise mesenchymal stem cells obtained from adipose tissue. In another embodiment, MSC cells comprise mesenchymal stem cells obtained from synovial membrane. In another embodiment, MSC cells comprise mesenchymal stem cells obtained from peripheral blood. In another embodiment, MSC cells comprise mesenchymal stem cells obtained from periodontal ligament. In another embodiment, MSC cells comprise mesenchymal stem cells obtained from endometrium. In another embodiment, MSC cells comprise mesenchymal stem cells obtained from umbilical cord blood. In another embodiment, MSC are not derived from embryonic stem (ES) cells. In another embodiment, MSC comprise adult stem cells.

According to one embodiment, the mesenchymal stem cells are human. In one embodiment, the mesenchymal stem cells are autologous to a subject. In another embodiment, the mesenchymal stem cells are non-autologous to a subject. In another embodiment, the mesenchymal stem cells are allogeneic to a subject.

In some embodiments, the mesenchymal stem cells are non-genetically modified. In some embodiments, mesenchymal stem cells are human cells. In one embodiment, bone marrow can be isolated from the iliac crest of an individual by aspiration. Low-density BM mononuclear cells (BMMNC) may be separated by, for example, a FICOL-PAQUE density gradient centrifugation. In order to obtain mesenchymal stem cells, a cell population comprising the mesenchymal stem cells (e.g. BMMNC) may be cultured in a proliferating medium capable of maintaining and/or expanding the cells in the presence of, for example, platelet lysate. According to one embodiment, the populations are plated on polystyrene plastic surfaces (e.g. in a tissue culture flask) and mesenchymal stem cells are isolated by removing non-adherent cells. Alternatively mesenchymal stem cell may be isolated by FACS using mesenchymal stem cell markers.

Following isolation the cells are typically expanded by culturing in a proliferation medium capable of maintaining and/or expanding the isolated cells *ex vivo* in the presence of, for example, platelet lysate. The proliferation medium may include DMEM, alpha-MEM or DMEM/F12.

In one embodiment, when the mesenchymal stem cells are human, the platelet lysate is also obtained from human cells. According to one embodiment, the medium is devoid of xeno contaminants i.e. free of animal derived components. An embodiment of a mesenchymal stem cell isolation and propagation protocol is presented in Example 1.

Verification that the isolated (and optionally propagated) cell population comprises mesenchymal stem cells may be effected by identification of phenotypic and functional criteria. The phenotypic criteria may include the expression of specific surface antigens: CD73, CD90 and CD105 (>=95% positive) and the absence (<2%) of CD-3 (T-cells surface marker), CD14 (Monocyte surface marker), CD19 (B cells), CD34 (Hematopoietic stem cells), CD45 (Hematopietic cells), and HLA-DR (Human Class II Histocompatibility antigen). The surface expression of these cells may be analyzed using methods known in the art-for example by Flow Cytometry.

Examples of antibodies that may be used to verify the presence of mesenchymal stem cells include, for example, but not limited to, CD73 PE conjugated (BD Pharmingen), CD90 PE-Cy5 conjugated (eBioscience) CD105 PE conjugated (Beckman Coulter) CD14 FITC conjugated (eBioscience) CD19 PE-Cy5 conjugated (eBioscience) CD34 FITC conjugated (Beckman Coulter), CD45 PE conjugated (eBioscience) and HLA-DR PE-Cy5 conjugated (BD Pharmingen).

Another method for verifying the presence of mesenchymal stem cells is by showing that the cells are capable of differentiating into multi-lineages such as for example adipocytes, osteocytes and chondrocytes. This may be performed, for example, by using Human Mesenchymal Stem Cell Functional Identification Kit (R&D Systems).

As described herein, following propagation of mesenchymal stem cells in a platelet lysate containing medium, the cells may be differentiated in a differentiating medium to generating cells useful for treating a neurodegenerative disorder.

Differentiating media and their components are well known in the art. It will be appreciated that the components of the differentiating medium are selected according to the cell phenotype required.

In some embodiments, mesenchymal stem cells (MSC) cells are induced to secrete at least one neurotrophic factor (NTF), wherein said cell population comprises MSC-NTF cells.

As used herein, the phrase "neurotrophic factor" ("NTF") refers to a cell factor that acts on the cerebral nervous system comprising growth, differentiation, functional maintenance and/or survival effects on neurons. Examples of neurotrophic factors include, for example, but are not limited to, a vascular endothelial growth factor (VEGF), a hepatocyte growth factor (HGF), a leukemia inhibitory factor (LIF), a glial derived neurotrophic factor (GDNF), a neurotrophin-3 (NT-3), a neurotrophin-4/5, a Neurturin (NTN), a Neurotrophin-4, a Persephin, artemin (ART), a ciliary neurotrophic factor (CNTF), an insulin growth factor-I (IGF-1), Growth and differentiation Factor (GDF-15), Granulocyte Stimulating factor (G-CSF), a Brain-derived neurotrophic factor (BDNF), a Tumor necrosis factor-inducible gene 6 protein (TSG-6; also known as TNF-stimulated gene 6 protein), Bone morphogenetic protein 2 (BMP2), Fibroblast Growth Factor 2 (FGF2), and a Neublastin.

In one embodiment, an NTF is selected from the group comprising a vascular endothelial growth factor (VEGF), a hepatocyte growth factor (HGF), a leukemia inhibitory factor (LIF), an , a glial derived neurotrophic factor (GDNF), a neurotrophin-3 (NT-3), a neurotrophin-4/5, a Neurturin (NTN), a Neurotrophin-4, a Persephin, artemin (ART), a ciliary neurotrophic factor (CNTF), an insulin growth factor-I (IGF-1), a Growth and differentiation Factor (GDF-15), a Granulocyte Stimulating factor (G-CSF) a Brain-derived neurotrophic factor (BDNF), a Tumor necrosis factor-inducible gene 6 protein (TSG-6; also known as TNF-stimulated gene 6 protein), Bone morphogenetic protein 2 (BMP2), Fibroblast Growth Factor 2 (FGF2), and an Neublastin, or any combination thereof.

In another embodiment, an NTF is a vascular endothelial growth factor (VEGF). In another embodiment, an NTF is a hepatocyte growth factor (HGF). In another embodiment, an NTF is a leukemia inhibitory factor (LIF). In another embodiment, an NTF is a glial derived neurotrophic factor (GDNF). In another embodiment, an NTF is a neurotrophin-3 (NT-3). In another embodiment, an NTF is a neurotrophin-4/5. In another embodiment, an NTF is a Neurturin (NTN). In another embodiment, an NTF is a Neurotrophin-4. In another embodiment, an NTF is a Persephin, artemin (ART). In another embodiment, an NTF is a ciliary neurotrophic factor (CNTF). In another embodiment, an NTF is an insulin growth factor-I (IGF-1). In another embodiment, an NTF is Growth and differentiation Factor (GDF-15). In another embodiment, an NTF is a Granulocyte Stimulating factor (G-CSF). In another embodiment, an NTF is a Brain-derived neurotrophic factor (BDNF). In another embodiment, an NTF is a Tumor necrosis factor-inducible gene 6 protein (TSG-6; also known as TNF-stimulated gene 6 protein). In another embodiment, an NTF is a Bone morphogenetic protein 2 (BMP2). In another embodiment, an NTF is a Fibroblast Growth Factor 2 (FGF2). In another embodiment, an NTF is a Neublastin.

In another embodiment, a MSC-NTF cell secretes at least one NTF, wherein said NTF is selected from the group comprising a vascular endothelial growth factor (VEGF), a hepatocyte growth factor (HGF), a leukemia inhibitory factor (LIF), a glial derived neurotrophic factor (GDNF), a neurotrophin-3 (NT-3), a neurotrophin-4/5, a Neurturin (NTN), a Neurotrophin-4, a Persephin, artemin (ART), a ciliary neurotrophic factor (CNTF), an insulin growth factor-I (IGF-1), a Growth and differentiation Factor (GDF-15), a Granulocyte Stimulating factor (G-CSF), a Brain-derived neurotrophic factor (BDNF), a Tumor necrosis factor-inducible gene 6 protein (TSG-6; also known as TNF-stimulated gene 6 protein), Bone morphogenetic protein 2 (BMP2), Fibroblast Growth Factor 2 (FGF2), and a Neublastin, or any combination thereof.

In another embodiment, a MSC-NTF cell secretes a vascular endothelial growth factor (VEGF). In another embodiment, a MSC-NTF cell secretes a hepatocyte growth factor (HGF). In another embodiment, a MSC-NTF secretes a leukemia inhibitory factor (LIF). In another embodiment, a MSC-NTF cell secretes a glial derived neurotrophic factor (GDNF). In another embodiment, a MSC-NTF cell secretes a neurotrophin-3 (NT-3). In another embodiment, a MSC-NTF cell secretes a neurotrophin-4/5. In another embodiment, a MSC-NTF cell secretes a Neurturin (NTN). In another embodiment, a MSC-NTF cell secretes a Neurotrophin-4. In another embodiment, a MSC-NTF cell secretes a Persephin. In another embodiment, a MSC-NTF cell secretes an artemin (ART). In another embodiment, a MSC-NTF cell secretes a ciliary neurotrophic factor (CNTF). In another embodiment, a MSC-NTF cell secretes an insulin growth factor-I (IGF-1). In another embodiment, a MSC-NTF cell secretes a Growth and differentiation Factor (GDF-15). In another embodiment, a MSC-NTF cell secretes a Granulocyte Stimulating factor (G-CSF). In another embodiment, a MSC-NTF cell secretes a Brain-derived neurotrophic factor (BDNF). In another embodiment, a MSC-NTF cell secretes a Tumor necrosis factor-inducible gene 6 protein (TSG-6; also known as TNF-stimulated gene 6 protein). In another embodiment, a MSC-NTF secretes a Bone morphogenetic protein 2 (BMP2). In another embodiment, a MSC-NTF secretes a Fibroblast Growth Factor 2 (FGF2). In another embodiment, a MSC-NTF cell secretes a Neublastin.

In another embodiment, a MSC-NTF cell secretes at least 2 NTFs. In another embodiment, a MSC-NTF cell secretes at least 3 NTFs. In another embodiment, a MSC-NTF cell secretes at least 4 NTFs. In another embodiment, a MSC-NTF cell secretes at least 5 NTFs.

Neurotrophic factors-secreting human mesenchymal stromal stem cells are well known in the art and fully described in PCT International Patent Application Publication Nos. WO2014/024183 and WO 2015/121859; Gothelf et al., 2014, Clin Transl Med., vol. 3, page 21; Petrou et al. 2014; Muscle & Nerve. 49(3):455-457 and Petrou et al., 2016, JAMA Neurol. Jan. 11:1-8; all of which are incorporated by reference herein in their entirety.

In one embodiment, MSC-NTF cells described herein secrete at least one NTF selected from the group comprising a vascular endothelial growth factor (VEGF), a hepatocyte growth factor (HGF), a leukemia inhibitory factor (LIF), a Brain-derived neurotrophic factor (BDNF), a Tumor necrosis factor-inducible gene 6 protein (TSG-6; also known as TNF-stimulated gene 6 protein), Bone morphogenetic protein 2 (BMP2), Fibroblast Growth Factor 2 (FGF2), or Granulocyte Stimulating factor (G-CSF), or any combination thereof.

In another embodiment, MSC-NTF cells described herein secrete NTF selected from the group comprising a vascular endothelial growth factor (VEGF), a hepatocyte growth factor, and a leukemia inhibitory factor (LIF). In another embodiment, MSC-NTF cells described herein secrete NTF selected from the group consisting of a vascular endothelial growth factor (VEGF), a hepatocyte growth factor, a leukemia inhibitory factor (LIF), , a Brain-derived neurotrophic factor (BDNF), a Tumor necrosis factor-inducible gene 6 protein (TSG-6; also known as TNF-stimulated gene 6 protein), Bone morphogenetic protein 2 (BMP2), Fibroblast Growth Factor 2 (FGF2), and Granulocyte Stimulating factor (G-CSF).

In some embodiments, at least 50%, at least 60%, at least 70%, at least 80%, at least 90% or more of a population of the MSC -NTF cells described herein express at least one NTF. In some embodiments, at least 50%, at least 60%, at least 70%, at least 80%, at least 90% or more of a population of the MSC -NTF cells described herein secrete at least one NTF.

In some embodiments, MSC-NTF cells described herein, express at least one miRNA molecule. In some embodiments, MSC-NTF cells described herein, express and secrete at least one miRNA molecule. In some embodiments, miRNA describe herein are present in a biological sample.

In some embodiments, expression of an at least one miRNA molecule in MSC-NTF cells is greater than that observed in control MSC. In some embodiments, the level of an at least one miRNA molecule in a biological sample from a subject administered MSC-NTF, for example a CSF biological sample, is greater than that observed in an equivalent control biological sample. In some embodiments, miRNAs may be excreted from MSC-NTF cells, for example by way of an exosome. In some embodiments, the expression and/or secretion of an at least one miRNA molecule from MSC-NTF cells is greater than that observed in control MSC.

In some embodiments, MSC-NTF cells described herein have reduced or no expression of at least one miRNA, compared with control MSC. In some embodiments, MSC-NTF cells described herein have reduced or no secretion of at least one miRNA, compared with control MSC. In some embodiments, MSC-NTF cells described herein have reduced or no expression or secretion of at least one miRNA, compared with control MSC..

In some embodiments, MSC-NTF cells described herein, are able to induce expression of at least one miRNA molecule in another cell. In some embodiments, MSC-NTF cells described herein, are able to induce at least one miRNA molecule in another cell. In some embodiments, MSC-NTF cells described herein, are able to induce expression of at least one miRNA molecule in another cell. Cell-to-cell communication in which a cell produces a signal to induce changes in nearby cells, altering the behavior of those cells may in some embodiments, be referred to as a paracrine response.

A skilled artisan would appreciate that the terms "miRNA", "miR", and "microRNA" may be used interchangeably having all the same meanings and qualities and encompass short noncoding RNAs. In some embodiments, miRNAs comprise about 21-25 nucleotides. In some embodiments, miRNAs comprise about 21-24 nucleotides. In some embodiments, miRNAs comprise 21, 22, 23, 24, or 25 nucleotides. In some embodiments, miRNAs may play an important role in the regulation of cellular gene expression by either suppressing translation of protein coding genes or by cleaving target mRNA to induce their degradation. In some embodiments, miRNAs may serve as paracrine signaling mediators.

Examples of miRNAs include, for example, but are not limited to miR-34a-5p, miR-132, miR-376-3p, miR-19b, miR-146a, miR-126-3p, miR-9-5p, miR-155 and miR-577.

In some embodiments, MSC-NTF cells described herein express at least one miRNAs selected from the group comprising miR-34a, miR-132, miR-376a, miR-19b, and miR-146a. In some embodiments, expression in MSC-NTF cells is elevated compared to MSC. In some embodiments, the level of an miRNA is elevated in a biological sample from a subject treated with MSC-NTF, for example but not limited to the CSF sample, compared with a control biological sample from an untreated (placebo) subject or a sample collected prior to treatment. Examples of elevated miRNAs include, but are not limited to miR-34a-5p, miR-132. In some embodiments, MSC-NTF cells express miR-34a. In some embodiments, MSC-NTF cells express miR-132. In some embodiments, MSC-NTF cells express miR-376a-3p. In some embodiments, MSC-NTF cells express miR-19b. In some embodiments, MSC-NTF cells express miR-146a.

In some embodiments, MSC-NTF cells described herein secrete at least one miRNAs selected from the group comprising miR-34a, miR-132, miR-376a, miR-19b, and miR-146a. In some embodiments, secretion is elevated compared to MSC. In some embodiments, the level of miRNAs selected from the group comprising miR-34a, miR-132, miR-376a, miR-19b, and miR-146a is elevated in a biological sample from a subject treated with MSC-NTF cells compared with a control biological sample. Examples of elevated miRNAs include, but are not limited to miR-34a-5p, miR-132, miR-376-3p, miR-19b, and miR-146a. In some embodiments, MSC-NTF cells secrete miR-34a. In some embodiments, MSC-NTF cells secrete miR-132. In some embodiments, MSC-NTF cells secrete miR-376a. In some embodiments, MSC-NTF cells miR-19b. In some embodiments, MSC-NTF cells secrete and miR-146a. In some embodiments, miR-34a levels are elevated in a biological sample from a subject treating with MSC-NTF compared with a control sample. In some embodiments, miR-132 levels are elevated in a biological sample from a subject treated with MSC-NTF cells compared with a control sample. In some embodiments, miR-376a levels are elevated in a biological sample from a subject treated with MSC-NTF cells compared with a control sample. In some embodiments, miR-19b levels are elevated in a biological sample from a subject treated with MSC-NTF cells compared with a control sample. In some embodiments, miR-146a levels are elevated in a biological sample from a subject treated with MSC-NTF cells compared with a control sample.

In some embodiments, MSC-NTF cells do not express or have low expression of at least one miRNA. In some embodiments, MSC-NTF cells do not express or have low expression of miR-126. In some embodiments, MSC-NTF cells do not express or have low expression of miR-9. In some embodiments, MSC-NTF cells do not express or have low expression of miR-126. In some embodiments, MSC-NTF cells do not express or have low expression of miR-155. In some embodiments, MSC-NTF cells do not express or have low expression of miR-577.

In some embodiments, miRNAs are globally down-regulated in motor neurons of ALS patients.

In one embodiment, MSC-NTF cells are *ex vivo* differentiated from mesenchymal stem cells, expressing at least one mesenchymal stem cell marker.

In one embodiment, the mesenchymal stem cells described herein are not genetically manipulated (i.e. transformed with an expression construct) to generate the differentiated cells and cell populations described herein.

In another embodiment, an isolated human cell comprising a MSC-NTF cell comprising at least one mesenchymal stem cell phenotype and secreting at least one NTF, for example VEGF, GDNF, LIF, G-CSF, BDNF, TGS-6, BMP2, FGF2, neuroblastin, or HGF, or any combination thereof comprises a basal secretion of the NTF that is greater than a basal secretion of the NTF in a non-differentiated mesenchymal stem cell.

The term "isolated" as used herein refers to a cell that has been removed from its *in-vivo* location (for example but not limited to bone marrow, neural tissue, adipose tissue, dental pulp, placenta, synovial membrane, peripheral blood, periodontal ligament, endometrium, umbilical cord, and umbilical cord blood). In one embodiment, the isolated cell is substantially free from other substances (e.g., other cell types) that are present in its *in-vivo* location.

The mesenchymal stem cell phenotypes which are comprised in the cells described herein are typically structural. For example, the cells described herein may show a morphology similar to that of mesenchymal stem cells (a spindle-like morphology). Alternatively or additionally the cells described herein may express a marker (e.g. surface marker) typical to mesenchymal stem cells but atypical to native astrocytic cells. Examples of mesenchymal stem cell surface markers include but are not limited to CD105, CD29, CD44, CD90, CD73, CD34, CD45, CD49, CD19, CD5, CD20, CD11B, and FMC7. Other mesenchymal stem cell markers include but are not limited to tyrosine hydroxylase, nestin and H-NF.

As used herein the term "basal secretion" refers to a secretion which does not involve addition of stimulants. MSC-NTF cells may be produced from non-differentiated MSC using methods described herein. Thus typically, the non-differentiated mesenchymal stem cell is in an identical medium to the MSC-NTF cells but without the addition of differentiating agents.

As used herein, in some embodiments, the term "express" may encompass the synthesis and/or secretion of a neurotrophic factor as described herein. In some embodiments, the term "express" may encompass the synthesis and/or secretion of an miRNA as described herein.

### VEGF

Vascular endothelial growth factor (VEGF) induces endothelial cell growth and angiogenesis. Studies on neuronal regeneration observed the neurotrophic proprieties of VEGF. In animal models of ALS, VEGF treatment leads to a delay of disease onset, an improvement of motor functions, protection of MNs and neuromuscular junctions, and increase in survival.

### HGF

Hepatocyte growth factor (HGF) comprises a growth factor acting on the liver that exerts anti-apoptotic activity in the liver after endotoxin-induced hepatic failure. In addition to its protective effects on MNs in culture and *in vivo* after axotomy, HGF was found to reduce MN degeneration and increase survival in rodent models of ALS.

HGF appears to be a good candidate for the treatment of ALS, especially since HGF levels in ALS patients appear to be dysregulated.

### LIF

Leukemia inhibitory factor (LIF), is a member of the interleukin-6 family of cytokines, is a multifunctional cytokine that exert different effects on different cell types. LIF was shown to support MN survival *in-vitro* and to reduce MN loss following nerve damage. In addition, it was demonstrated that LIF takes part in control of neuromuscular connectivity. LIF also support oligodendrocytes function, by promoting proliferation of oligodendrocytes precursors and enhancing axon remyelination.

### miR-34a-5p

miR-34a-5p is a tumor suppressor miRNA which is activated by p53. In some embodiments, it regulates neurite outgrowth and spinal morphology. In some embodiments, it is up-regulated in plasma of pre-symptomatic Huntington Disease (HD) gene carriers.

### miR-132

In some embodiments, miR-132 positively controls angiogenesis in response to VEGF. In some embodiments, it is a negative regulator of inflammation. In some embodiments, miR-132 is highly enriched in neurons and promotes neuronal outgrowth *in vitro* and *in vivo.* In some embodiments, cytoplasmic TDP-43 hinder the biogenesis of miR-132-3p.

### miR-376a

In some embodiments, miR-376a is enriched in neurons and promotes neuronal differentiation.

### miR-19b

In some embodiments, miR-19b has anti-apoptotic and pro-proliferative activity. In some embodiments, miR-19b is down-regulated in motor cortex and brainstem motor nuclei of SOD1 mice.

### miR-146a

In some embodiments, miR-146a mediates suppression of the inflammatory response. In some embodiments, miR-146a reduces the levels of MCP-1.

### miR-126

In some embodiments, miR-126 is primarily an endothelial miRNA. In some embodiments, miR-126 is expressed in motoneurons. In some embodiments, miR-126 enhances the inflammatory response.

### miR-9

In some embodiments, miR-9 is one of the most highly expressed microRNAs in the developing and adult brain. In some embodiments, miR-9 is a key regulator of neurogenesis. In some embodiments, miR-9 was shown to repress Neurofilament light (NFL) expression and to be involved in microglia activation.

### Inflammatory factors or pro-apoptotic factors or factors that influence inflammatory factors

Accumulating evidence indicates that abnormal immune response and inflammation participate to the course of ALS onset and disease progression. These observations include microglia activation, increased number of reactive astrocytes, infiltration of macrophages and T lymphocytes. Although the true nature of the immune response in ALS is not fully understood, the current view is that it confers a neuroprotective effect in the early stages of the disease which turns into a neurotoxic effect during ALS progression.

### CRP

C-Reactive Protein (CRP) is a liver produced protein which is widely used as an inflammation marker due to its high magnitude of increase in the serum and its correlation with the inflammatory state. In addition, it was reported that CRP levels in the CSF can be used to identify inflammations in the CNS, such as bacterial meningitis. Furthermore, CRP levels were shown be elevated in the CSF of many ALS patients.

### MCP-1

Monocyte Chemotactic Protein 1 (MCP1/CCL2) is one of the chief chemokines which take part in regulation of monocytes/macrophages infiltration and migration in response to inflammation. It was previously reported that MCP1 expression is upregulated in spinal cord post-mortem samples of ALS patients. Increased levels of MCP1 in the CSF of ALS patients were also observed in several studies.

### SDF-1

Stromal cell-derived factors 1 (SDF1/CXCL12) is a chemokine involved in recruitment of different cell types through binding to its receptor CXCR4. Both SDF1 and CXCR4 are expressed in the CNS and are known to regulate neurotransmission and neuron-glia interactions. Normally, SDF1 regulates glutamate release from astrocytes. However, upregulation of SDF1 expression results in an increase of glutamate release, which in turn may result in neurotoxicity and apoptosis. Reduction in SDF1 levels would lead to a reduction of glutamate and decreased neurotoxicity and apoptosis. Recently, it was shown that administration of CXCR4 antagonist to ALS mice model extended their lifespan, improved motor function and decreased proinflammatory cytokines in spinal cords.

Interestingly, GLAST is a transporter that removes glutamate from the extracellular space. The results presented in Example 2 show increased GLAST secretion, which may explain the decrease of glutamate in the MSC-NTF cells culture supernatants, as compared to the MSC cells culture supernatants. In one embodiment, secretion of SDF-1 is reduced in MSC-NTF cells, and secretion of GLAST is increased in MSC-NTF cells.

### CHIT

Chitinase 1 (CHIT1) is a secreted enzyme which is expressed in chronically activated tissue macrophage and microglia. In previous studies, CHIT was suggested as a potential biomarker for ALS progression since CHIT was upregulated both in the CSF and in blood samples ALS patients. Moreover, blood CHIT levels were significantly higher in rapidly progressing ALS patients than in slowly progressing patients.

### Caspase 3

Caspase 3 (CASP3) is one of the key mediators in execution of apoptosis. In ALS mice models it was shown to be involved in motor neuron death. Moreover, administration of a caspase inhibitor to ALS mouse models which inhibited Caspase3 upregulation, also delayed disease onset and mortality.

In one embodiment, MSC-NTF cells described herein do not secrete nerve growth factor (NGF).

A skilled artisan would appreciate that the term "biomarker", a portmanteau of "biological marker", encompasses a broad subcategory of medical signs - that is, objective indications of medical state observed from outside the patient - which can be measured accurately and reproducibly. In 1998, the National Institutes of Health Biomarkers Definitions Working Group defined a biomarker as "a characteristic that is objectively measured and evaluated as an indicator of normal biological processes, pathogenic processes, or pharmacologic responses to a therapeutic intervention."

In some embodiments, a biomarker comprises an indicator of a neurodegenerative disease in a subject. In some embodiments, a biomarker may be used to follow the progression of a neurodegenerative disease. In some embodiments, a biomarker may be used to diagnose a neurodegenerative disease. In some embodiments, multiple biomarkers are used as an indicator of a neurodegenerative disease in a subject. In some embodiments, multiple biomarkers are used to follow the progression of a neurodegenerative disease. In some embodiments, multiple biomarkers are used to diagnose a neurodegenerative disease.

In some embodiments, a biomarker comprises an indicator of ALS in a subject. In some embodiments, a biomarker may be used to follow the progression of ALS. In some embodiments, a biomarker may be used to diagnose ALS. In some embodiments, multiple biomarkers are used as an indicator of ALS in a subject. In some embodiments, multiple biomarkers are used to follow the progression of ALS. In some embodiments, multiple biomarkers are used to diagnose ALS.

In some embodiments, a biomarker that is an indicator of ALS comprises a neurotropic factor, an inflammatory factor or pro-apoptotic factor or factor that influence inflammatory factors, or an miRNA, or any combination thereof. In some embodiments a biomarker used to follow the progression of ALS comprises a neurotropic factor, an inflammatory factor or pro-apoptotic factor or factor that influence inflammatory factors, or an miRNA, or any combination thereof. In some embodiments a biomarker used to diagnose of ALS comprises a neurotropic factor, an inflammatory factor or pro-apoptotic factor or factor that influence inflammatory factors, or an miRNA, or any combination thereof.

In some embodiments, a biomarker is selected from the group comprising a vascular endothelial growth factor (VEGF), a hepatocyte growth factor (HGF), a leukemia inhibitory factor (LIF), a Brain-derived neurotrophic factor (BDNF), a Tumor necrosis factor-inducible gene 6 protein (TSG-6; also known as TNF-stimulated gene 6 protein), or Granulocyte Stimulating factor (G-CSF), a chitinase 1 (CHIT1), a C-reactive protein (CRP), a monocyte chemotactic protein 1 (MCP1), a stromal derived factor 1 (SDF-1), Macrophage Inflammatory protein (MIP)-1b, Glutamate, or a caspase 3 (CASP3), a miR-a 34a-5p, a miR-132, a miR-376-3p, a miR-19b, a miR-146a, a miR-126-3p, a miR-9-5p, a miR-155, or a miR-577, or any combination thereof.

In some embodiments, a biomarker used as an indicator of ALS treatment efficacy comprises a gene whose expression is altered in a biological sample obtained from a treated subject compared with a control biological sample. In some embodiments, a biomarker used as an indicator of ALS treatment efficacy comprises TOP2A, RAB27b, WNT5A, SNAP25, AREG, SLC1A1, SLC16A6, MEST, SLC1A3, PCSK1, or TUBB3, or any combination thereof.

In some embodiments, the secretion of at least one biomarker increases and the secretion of at least one other biomarker decreases in a biological sample obtained from a treated subject compared with a control biological sample.

It will be appreciated that cell populations obtained according to the methods describe herein are typically non-homogeneous.

Thus according to another embodiment, described herein there is provided an isolated cell population comprising human cells wherein:(i) at least N % of the human cells secreting (a neurotrophic factor as described herein, for example GDNF, VEGF, HGF, LIF, BDNF, TSG-6, BMP2, FGF2, or G-CSF, or any combination thereof, wherein a basal secretion of the neurotrophic factor is greater than a basal secretion of the same neurotrophic factor in a non-differentiated mesenchymal stem cell; (ii) at least M % of the human cells comprise at least one mesenchymal stem cell phenotype; and (iii) at least one of the human cells secretes the neurotrophic factor and the at least one mesenchymal stem cell phenotype; where M and N are each independently selected between 1 and 99.

M % may be any percent from 1% to 99% e.g. 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99%.

N % may be any percent from 1% to 99% e.g. 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99%.

The percentage of cells which secrete the neurotrophic factor may be raised or lowered according to the intended needs.

Once differentiated and optionally isolated, the cells may be tested (in culture) for their ability to secrete a neurotrophic factor, as described herein. One skilled in the art would appreciate that methods of analyzing secretion of neurotrophic factors, including those described herein, are well known in the art.

In one embodiment, an ELISA assay may be used for the detection of secreted NTFs. For analysis of secreted NTFs, supernatant is collected from cultures of MSCs or of NTF-secreting cells at the end of the differentiation procedure described herein, and cells are harvested and counted. The amount of NTFs, for example, Glial Derived Neurotrophic Factor, (GDNF), vascular endothelial growth factor (VEGF), hepatocyte growth factor (HGF), leukemia inhibitory factor (LIF), Granulocyte Stimulating factor (G-CSF), GDF-15, a Brain-derived neurotrophic factor (BDNF), a Tumor necrosis factor-inducible gene 6 protein (TSG-6; also known as TNF-stimulated gene 6 protein), Bone morphogenetic protein 2 (BMP2), or Fibroblast Growth Factor 2 (FGF2), in the cell's culture supernatants can be quantified by using a ELISA assays according to the manufacturer's protocol(s).

In one embodiment, methods of use of the MSC-NTF cells described herein increase the concentration of neurotrophic factors (e.g., VEGF, HGF, LIF, BDNF, TSG-6, G-CSF, BMP2, FGF2) in a biological sample of a subject compared with a control biological sample. In another embodiment, the biological sample is cerebrospinal fluid. In another embodiment, the biological sample is blood or blood serum. In another embodiment, the biological sample is urine.

In one embodiment, methods of use of the MSC-NTF cells described herein, decrease the concentration of inflammatory factors or pro-apoptotic factors or factors that influence inflammatory factors(for example but not limited to CHIT1, CRP, MCP1, SDF-1, MIP-1b, Glutamate, CASP3) in a biological sample of a subject compared with a control biological sample.. In another embodiment, the biological sample is cerebrospinal fluid. In another embodiment, the biological sample is blood or blood serum. In another embodiment, the biological sample is urine.

In another embodiment, the methods of use of the MSC-NTF cells described herein, increases the concentration of neurotrophic factors in the CSF of a subject and concurrently decrease the concentration of inflammatory factors or pro-apoptotic factors or factors that influence inflammatory factors in the CSF said subject. In another embodiment, the methods of use of the MSC-NTF cells described herein, increases the concentration of neurotrophic factors in the blood or blood serum of a subject and concurrently decrease the concentration of inflammatory factors or pro-apoptotic factors or factors that influence inflammatory factors in the blood or blood serum said subject compared with a control biological sample. In another embodiment, the methods of use of the MSC-NTF described herein, increases the concentration of neurotrophic factors in the urine of a subject and concurrently decrease the concentration of inflammatory factors or pro-apoptotic factors or factors that influence inflammatory factors in the urine said subject compared with a control biological sample.

In some embodiments, methods of used described herein follow expression of biomarkers, for example but not limited to NTFs, miRNAs, or inflammatory factors or pro-apoptotic factors or factors that influence inflammatory factors, or any combination thereof, wherein the expression of the biomarkers in the circulation is compared with the presence of the biomarker in the CSF. In some embodiments, methods of use described herein follow expression of biomarkers, for example but not limited to NTFs, miRNAs, or inflammatory factors or pro-apoptotic factors or factors that influence inflammatory factors, or any combination thereof, wherein the presence of the biomarkers in the circulation is compared with the presence of the biomarker in the CSF.

In one embodiment, MSC-NTF cells produced by the methods disclosed here, may be incorporated into compositions suitable for administration. Such compositions may comprise the MSC-NTF cell population, and a pharmaceutically acceptable carrier. As used herein, "pharmaceutically acceptable carrier" is intended to include any and all solvents, dispersion media, coatings, antibacterial and antifungal agents, isotonic and absorption delaying agents, and the like, compatible with pharmaceutical administration. Suitable carriers are described in the most recent edition of Remington's Pharmaceutical Sciences, a standard reference text in the field, which is incorporated herein by reference. Examples of such carriers or diluents include, but are not limited to, water, saline, finger's solutions, dextrose solution, basal medium (DMEM) a biopreservation medium and 5% human serum albumin. Except insofar as any conventional media or agent is incompatible with the active compound, use thereof in the compositions is contemplated. Supplementary active compounds can also be incorporated into the compositions.

A composition disclosed here is formulated to be compatible with its intended route of administration. Examples of routes of administration include intramuscular and intrathecal.

In one embodiment, compositions suitable for injectable use include sterile aqueous solutions. In all cases, the composition must be sterile and should be fluid to the extent that easy syringeability exists. It must be stable under the conditions of manufacture and storage and must be preserved against the contaminating action of microorganisms such as bacteria and fungi.

In one embodiment, a composition may be included in a container, vial, pack, or dispenser, for example a syringe, together with instructions for administration.

In some embodiments, a composition described herein comprises a cell population present in an amount therapeutically effective to treat neurodegenerative disease in a subject, said cell population comprising MSC-NTF cells induced to secrete at least one neurotrophic factor (NTF).

In some embodiments, a composition described herein comprises a cell population present in an amount therapeutically effective to treat neurodegenerative disease in a subject, said cell population comprising MSC-NTF cells induced to express or secrete, or a combination thereof, at least one miRNA. In some embodiment, a composition described herein comprises a cell population present in an amount therapeutically effective to treat neurodegenerative disease in a subject, said cell population comprising MSC-NTF cells induced to express and secrete at least one miRNA. In some embodiment, a composition described herein comprises a cell population present in an amount therapeutically effective to treat neurodegenerative disease in a subject, said cell population comprising MSC-NTF cells induce paracrine signaling.

In some embodiment, a composition described herein comprises a cell population present in an amount therapeutically effective to treat neurodegenerative disease in a subject, said cell population comprising MSC-NTF cells induced to express and secrete at least one neurotropic factor (NTF) and to express, or express and secrete at least one miRNA.

A skilled artisan would appreciate that a "therapeutically effective amount" may encompass an amount effective, at dosages and for periods of time necessary, to achieve the desired therapeutic result. A therapeutically effective amount may vary according to factors such as the disease state, age, sex, and weight of the individual, and the ability of the cells to elicit a desired response in the individual. A therapeutically effective amount is also one in which any toxic or detrimental effects of the cells are outweighed by the therapeutically beneficial effects.

In one embodiment, a composition described herein effectively treat a neurodegenerative disease, wherein said neurodegenerative disease comprises Amyotrophic Lateral Sclerosis (ALS); frontotemporal dementia (FTD); Parkinson's disease; Multiple System Atrophy (MSA); Huntington's disease; Alzheimer's disease; Rett Syndrome; lysosomal storage diseases; "white matter disease" or glial/demyelination disease, including Sanfilippo, Gaucher disease; Tay Sachs disease (beta hexosaminidase deficiency); multiple sclerosis (MS); Neuromyelitis Optica (NMO); NMO spectrum disease; brain injury or trauma caused by ischemia, accidents, or environmental insult; stroke; cerebral palsy (CP); autism and autism spectrum disorder; spinal cord damage; or ataxia; or any combination thereof.

In another embodiment, a composition comprises MSC-NTF cells as described and exemplified herein. In another embodiment, the MSC cells comprise populations of bone marrow mesenchymal stem cells. In another embodiment, the MSC cells comprise populations of adipocyte stem cells. In another embodiment, the MSC cells comprise populations of dental pulp mesenchymal stem cells.

In another embodiment, a composition comprises MSC-NTF cells that are *ex vivo* differentiated from mesenchymal stem cells, and are expressing at least one mesenchymal stem cell marker. In another embodiment, a composition comprises MSC-NTF that secrete at least on neurotrophic factor, as described in detail herein. In still another embodiment, said MSC-NTF cells secrete at least one NTF, said NTF comprising a vascular endothelial growth factor (VEGF), a hepatocyte growth factor (HGF), a leukemia inhibitory factor (LIF), , a Granulocyte Stimulating factor (G-CSF), or a Brain-derived neurotrophic factor (BDNF), or a Tumor necrosis factor-inducible gene 6 protein (TSG-6; also known as TNF-stimulated gene 6 protein), Bone morphogenetic protein 2 (BMP2), or Fibroblast Growth Factor 2 (FGF2), or any combination thereof.

### Method of Use

In one embodiment, according to the phenotype, the MSC-NTF cells and populations thereof, described herein, may be used to treat a neurodegenerative disease or disorder. In one embodiment, a method described herein comprises treating a neurodegenerative disease in a subject in need thereof, the method comprising administering to said subject a therapeutically effective amount of a cell population of MSC-NTF induced to secrete at least one neurotrophic factor (NTF), thereby treating said neurodegenerative disease in said subject.

In some embodiments, said MSC-NTF cells may be used in combination with an additional treatment or therapy used for treating neurodegenerative diseases in a subject. In some embodiments, the additional treatment or therapy comprises administration of riluzole. In some embodiments, the additional treatment or therapy comprises administration of edaravone (Radicava).

A skilled artisan would appreciate that in one embodiment, the term "treating" and its grammatical equivalents, e.g., treatment, may be used interchangeable herein with the term "transplanting" having all the same meanings and qualities. In some embodiments, transplanting is performed by injecting a composition described herein into a subject in need. In some embodiments, transplanting is performed by injecting a MSC-NTF cell population described herein into a subject in need.

In another embodiment, the neurodegenerative disease comprises Amyotrophic Lateral Sclerosis (ALS). In another embodiment, the neurodegenerative disease comprises frontotemporal dementia (FTD). In another embodiment, the neurodegenerative disease comprises Parkinson's disease. In another embodiment, the neurodegenerative disease comprises Multiple System Atrophy (MSA). In another embodiment, the neurodegenerative disease comprises Huntington's disease. In another embodiment, the neurodegenerative disease comprises Alzheimer's disease. In another embodiment, the neurodegenerative disease comprises Rett Syndrome. In another embodiment, the neurodegenerative disease comprises lysosomal storage diseases. In another embodiment, the neurodegenerative disease comprises "white matter disease" or glial/demyelination disease, including Sanfilippo. In another embodiment, the neurodegenerative disease comprises Gaucher disease. In another embodiment, the neurodegenerative disease comprises Tay Sachs disease (beta hexosaminidase deficiency). In another embodiment, the neurodegenerative disease comprises multiple sclerosis (MS). In another embodiment, the neurodegenerative disease comprises neuromyelitis optica (NMO). In another embodiment, the neurodegenerative disease comprises NMO spectrum disease. In another embodiment, the neurodegenerative disease comprises brain injury or trauma caused by ischemia, accidents, or environmental insult. In another embodiment, the neurodegenerative disorder comprises stroke. In another embodiment, the neurodegenerative disorder comprises cerebral palsy (CP). In another embodiment, the neurodegenerative disease comprises autism or an autism spectrum disorder, or any combination thereof. In another embodiment, the neurodegenerative disease comprises spinal cord damage. In another embodiment, the neurodegenerative disease comprises ataxia.

In one embodiment, methods described herein use a MSC-NTF cell population or a composition thereof, directly following differentiation. In another embodiment, methods described herein use a MSC-NTF cell population or a composition thereof that has been enriched for a particular phenotype. Certain neurotrophic factors or set of neurotrophic factors have been shown to be particularly beneficial for treating ALS.

In another embodiment, a MSC-NTF cell population or a composition thereof used in methods of treating a neurodegenerative disease or disorder, comprises MSC-NTF cells as described herein. In another embodiment, said MSC-NTF cells or population thereof, or composition thereof, secretes NTF beneficial for treating the neurodegenerative disease or disorder being treated. For example, in one embodiment, methods described herein for treating ALS comprise the use of a MSC-NTF cell population or a composition thereof, which secretes a vascular endothelial growth factor (VEGF), a hepatocyte growth factor, a leukemia inhibitory factor (LIF), a Brain-derived neurotrophic factor (BDNF), a Tumor necrosis factor-inducible gene 6 protein (TSG-6; also known as TNF-stimulated gene 6 protein), Bone morphogenetic protein 2 (BMP2), Fibroblast Growth Factor 2 (FGF2), or Granulocyte Stimulating factor (G-CSF), or any combination thereof.

In another embodiment, a method described herein comprises a step of assaying a biological sample from said subject prior to and following administration of said therapeutically effective amount of MSC-NTF cells or population or compositin thereof. In another embodiment, a biological sample described herein comprises blood, serum, urine, or cerebrospinal fluid (CSF).

In some embodiments, following administration of MSC-NTF cells or a population or composition thereof as described herein, a biological sample comprises increased levels of at least one neurotrophic factor compared with a biological sample from a control subject receiving a placebo. In one embodiment, a control subject is a non-treated subject. In some embodiments, following administration of MSC-NTF cells or a population or composition thereof as described herein, the biological sample comprises increased levels of at least one miRNA compared with a biological sample from a control subject receiving a placebo. In another embodiment, a control subject is a placebo treated subject. In some embodiments, a control biological sample comprises a sample obtained from a control subject receiving a placebo. In some embodiments, a biological sample comprises a sample obtained from the subject being treated, wherein the control biological sample was obtained prior to administration with MSC-NTF cells.

In some embodiments, a control sample comprises MSC cells from the same subject that have not been induced to secrete increased levels of at least one NTF. A skilled artisan would appreciate that while MSC and MSC-NTF may both secrete the same at least one NTF, MSC-NTF cells have been induced to have increased secretion of an at least one NTF compared with the MSC from which the MSC-NTF cells were derived. In some embodiments, a control sample comprises undifferentiated MSC from a subject to be treated with MSC-NTF.

In some embodiments, a control biological sample comprises MSC cells from the same donor/patient from which the MSC-NTF cells were derived (induced to differentiate, namely before and after differentiation). In some embodiments, a control biological sample comprises a sample obtained from a patient treated with MSC-NTF cells - prior to treatment. In some embodiments, a control sample comprises a sample from a non-treated patient (Placebo).

In one embodiment, following said administration said biological sample comprises decreased levels of at least one inflammatory factor or pro-apoptotic factor or factor that influence inflammatory factors, compared with a biological sample from a control subject. In another embodiment, the inflammatory factor or pro-apoptotic factor or factor that influence inflammatory factors is selected from the group comprising a chitinase 1 (CHIT1), a C-reactive protein (CRP), a monocyte chemotactic protein 1 (MCP1), a stromal derived factor 1 (SDF-1), Macrophage Inflammatory protein (MIP-1), Glutamate, or a caspase 3 (CASP3), or any combination thereof. In another embodiment, following said administration said biological sample comprises increased levels of at least one neurotrophic factor and decreased levels of at least one inflammatory factor or pro-apoptotic factor or factor that influence inflammatory factors compared with a biological sample from a control subject.

In some embodiments, following said administration said biological sample comprises increased levels of at least one miRNA in non-responders compared with a biological sample from a control subject. In another embodiment, the miRNA is miR-126-3p. In another embodiment, following said administration said biological sample comprises increased levels of at least one miRNA and decreased levels of at least one miRNA compared with a biological sample from a control subject.

Neurotrophic factors-secreting human mesenchymal stromal stem cells may be administered by any method known to one of skilled in the art. Transplantations of neurotrophic factors-secreting human mesenchymal stromal stem cells are described, for example, in Gothelf et al., 2014, Clin Transl Med., vol. 3, page 21; Petrou et al., 2016 JAMA Neurol. Jan 11:1-8; Petrou et al. 2014; Muscle & Nerve. 49(3):455-457; WO2014/024183; WO 2015/121859 which are incorporated by reference herein in their entirety.

In another embodiment, provided herein are methods of use of a composition comprising a cell population present in an amount therapeutically effective to treat amyotrophic lateral sclerosis (ALS) in a subject, wherein said cell population comprising modified mesenchymal stem cells capable of secreting at least one neurotrophic factor (NTF).

The composition described herein may be administered via any suitable method known to one of skilled in the art. Examples of such method include, but are not limited to, intrathecal, intramuscular, intradermal, intraperitoneal, intravenous, subcutaneous, and oral routes. Administration may also include systemic or local administration of the composition disclosed herein.

The administration may also encompass surgically administering, implanting, inserting, or injecting the MSC-NTF cells or populations thereof, into a subject. The cells can be located intrathecally, subcutaneously, intramuscularly, in the central nervous system (CNS), or located at another body location, which allow the cells to perform their intended function. Suitable sites for administration may be readily determined by a medical professional.

In one embodiment, the cell population is administered intrathecally into the CSF of the subject. In another embodiment, the cell population is administered into the muscles of the subject. In a further embodiment, administration comprises administering to the cerebrospinal fluid. In still a further embodiment, administration comprises administering to the central nervous system of the subject. In another embodiment, administration comprises administering to the cerebrospinal fluid or the central nervous system, or any combination thereof of the subject.

A skill artisan would appreciate that the term "central nervous system" may encompass the brain and the spinal cord. In some embodiments, administration comprises administering to the brain. In some embodiments, administration comprises administering to the spinal cord. In some embodiments, administration comprises administering to the brain and to the spinal cord.

In one embodiment, administration comprises intramuscular (IM) injection or intrathecal (IT) injection, or a combination thereof.

In one embodiment, IM injection is at a dose of about 1 x 10⁶ MSC-NTF cells per injection. In another embodiment, IM injection is at a dose of about 2 x 10⁶ MSC-NTF cells per injection. In another embodiment, IM injection is at a dose of about 3 x 10⁶ MSC-NTF cells per injection. In another embodiment, IM injection is at a dose of about 1-2 x 10⁶ MSC-NTF cells per injection. In another embodiment, IM injection is at a dose of about 2-3 x 10⁶ MSC-NTF cells per injection. In another embodiment, IM injection is at a dose of about 1-3 x 10⁶ MSC-NTF cells per injection.

In another embodiment, IM administration comprises multiple injections at the same time point. One skilled in the art would appreciate that multiple injections at the same time point may encompass injections given one following the other at a given time point. In another embodiment, IM administration comprises about 20 injections. In another embodiment, IM administration comprises about 21 injections. In another embodiment, IM administration comprises about 22 injections. In another embodiment, IM administration comprises about 23 injections. In another embodiment, IM administration comprises about 24 injections. In another embodiment, IM administration comprises about 25 injections. In another embodiment, IM administration comprises about 26 injections. In another embodiment, IM administration comprises about 27 injections. In another embodiment, IM administration comprises about 28 injections. In another embodiment, IM administration comprises about 29 injections. In another embodiment, IM administration comprises about 30 injections.

In another embodiment, the dosage of multiple IM injections comprises a dose of about 24 x 10⁶ MSC-NTF cells. In another embodiment, the dosage of multiple IM injections comprises a dose of about 30 x 10⁶ MSC-NTF cells. In another embodiment, the dosage of multiple IM injections comprises a dose of about 36 x 10⁶ MSC-NTF cells. In another embodiment, the dosage of multiple IM injections comprises a dose of about 40 x 10⁶ MSC-NTF cells. In another embodiment, the dosage of multiple IM injections comprises a dose of about 42 x 10⁶ MSC-NTF cells. In another embodiment, the dosage of multiple IM injections comprises a dose of about 44 x 10⁶ MSC-NTF cells. In another embodiment, the dosage of multiple IM injections comprises a dose of about 46 x 10⁶ MSC-NTF cells. In another embodiment, the dosage of multiple IM injections comprises a dose of about 48 x 10⁶ MSC-NTF cells. In another embodiment, the dosage of multiple IM injections comprises a dose of about 50 x 10⁶ MSC-NTF cells. In another embodiment, the dosage of multiple IM injections comprises a dose of about 56 x 10⁶ MSC-NTF cells. In another embodiment, the dosage of multiple IM injections comprises a dose of about 60 x 10⁶ MSC-NTF cells.

In another embodiment, the dosage of multiple IM injections comprises a dose of about 24-60 x 10⁶ MSC-NTF cells. In another embodiment, the dosage of multiple IM injections comprises a dose of about 24-30 x 10⁶ MSC-NTF cells. In another embodiment, the dosage of multiple IM injections comprises a dose of about 30-36 x 10⁶ MSC-NTF cells. In another embodiment, the dosage of multiple IM injections comprises a dose of about 36-42 x 10⁶ MSC-NTF cells. In another embodiment, the dosage of multiple IM injections comprises a dose of about 42-48 x 10⁶ MSC-NTF cells. In another embodiment, the dosage of multiple IM injections comprises a dose of about 44-50 x 10⁶ MSC-NTF cells. In another embodiment, the dosage of multiple IM injections comprises a dose of about 46-52 x 10⁶ MSC-NTF cells. In another embodiment, the dosage of multiple IM injections comprises a dose of about 48-54 x 10⁶ MSC-NTF cells. In another embodiment, the dosage of multiple IM injections comprises a dose of about 54-60 x 10⁶ MSC-NTF.

In one embodiment, an IT injection comprises a dose of about 80 x 10⁶ MSC-NTF cells. In another embodiment, an IT injection comprises a dose of about 100 x 10⁶ MSC-NTF cells. In another embodiment, an IT injection comprises a dose of about 105 x 10⁶ MSC-NTF cells. In another embodiment, an IT injection comprises a dose of about 110 x 10⁶ MSC-NTF cells. In another embodiment, an IT injection comprises a dose of about 115 x 10⁶ MSC-NTF cells. In another embodiment, an IT injection comprises a dose of about 120 x 10⁶ MSC-NTF cells. In another embodiment, an IT injection comprises a dose of about 125 x 10⁶ differentiated mesenchymal cells. In another embodiment, an IT injection comprises a dose of about 130 x 10⁶ MSC-NTF cells. In another embodiment, an IT injection comprises a dose of about 135 x 10⁶ MSC-NTF cells. In another embodiment, an IT injection comprises a dose of about 140 x 10⁶ MSC-NTF cells. In another embodiment, an IT injection comprises a dose of about 150 x 10⁶ MSC-NTF cells. In another embodiment, an IT injection comprises a dose of about 200 x 10⁶ MSC-NTF cells.

In some embodiments, administration comprises a therapeutically effective number of time points. In some embodiments, injections are administered every month. In some embodiments, injections are administered every two months. In some embodiments, injections are administered every three months. In some embodiments, injections are administered as needed. In some embodiments, multiple injections are provided at each administration. In some embodiment, 2-30 injections are provide at each administration. In some embodiment, 2-20 injections are provide at each administration. In some embodiment, 20-30 injections are provide at each administration. In some embodiment, 10-20 injections are provide at each administration. In some embodiment, 2, 3, 4, 5, 6, 7, 8, 9, 10, 11, 12, 13, 14, 15, 16, 17, 18, 19, 20, 21, 22, 23, 24, 25, 26, 27, 28, 29, or 30 injections are provide at each administration.

In one embodiment, an IT injection comprises a dose of about 80-140 x 10⁶ MSC-NTF cells. In another embodiment, an IT injection comprises a dose of about 100-125 x 10⁶ MSC-NTF cells. In one embodiment, an IT injection comprises a dose of about 125-140 x 10⁶ MSC-NTF cells. In another embodiment, an IT injection comprises a dose of about 150-200 x 10⁶ MSC-NTF cells.

In another embodiment, an IT injection comprises a dose of about 125 x 10⁶ differentiated mesenchymal cells, injected every two months, wherein each administration comprises 3 injections.

In one embodiment, administration comprises a single time point. One skilled in the art would appreciate that treatment may encompass follow-up administration of MSC-NTF cell as described herein. In another embodiment, administration comprises at least two time points. In another embodiment, administration comprises two time points. The timing of administration of a second or further time point, may encompass analysis of biological samples taken from a subject and analyzed for NTF and /or inflammatory factors, as described herein. In some embodiment, administration comprising as many time points as necessary for therapeutic efficacy. In some embodiments, therapeutic efficacy may be determined based on analysis of secretion of at least one NTF and/or and at least one inflammatory factor or pro-apoptotic factor or factor that influence inflammatory factors, as described in detail herein.

In another embodiment, follow-up administration enhances the treatment of the neurodegenerative disease, for example ALS. In another embodiment, a repeat dose comprises a second administration at about 8 to 12 weeks following the initial treatment. In another embodiment, a repeat dose comprises a second administration at about 7 weeks. In another embodiment, a repeat dose comprises a second administration at about 8 weeks. In another embodiment, a repeat dose comprises a second administration at about 9 weeks. In another embodiment, a repeat dose comprises a second administration at about 10 weeks. In another embodiment, a repeat dose comprises a second administration at about 11 weeks. In another embodiment, a repeat dose comprises a second administration at about 12 weeks. In another embodiment, a repeat dose comprises a second administration at about 13 weeks. In another embodiment, a repeat dose comprises a second administration at about 14 weeks. In another embodiment, administration comprises repeat administrations at about at least three time points. In another embodiment, administration comprises repeat administrations at about at least four time points. In another embodiment, administration comprises repeat administrations at about at least five time points. In another embodiment, administration comprises repeat administrations at more than 5 time points. In another embodiment, administration comprises repeat administrations at more than 10 time points. In another embodiment, administration comprises a repeat dose at follow-up time points for a duration of a disease or disorder.

The neurodegenerative disease or condition treated by the methods of use described herein, have been described above. In one embodiment, a neurodegenerative disease or condition treated by the methods of use described herein include, but is not limited to amyotrophic lateral sclerosis (ALS) and its associated diseases and conditions.

As used herein, the terms "treat" and "treatment" may encompass therapeutic treatment, including prophylactic or preventative measures, wherein the object is to prevent or slow down (lessen) an undesired physiological change associated with a disease or condition. Beneficial or desired clinical results include, but are not limited to, alleviation of symptoms, diminishment of the extent of a disease or condition, stabilization of a disease or condition (*i.e.*, where the disease or condition does not worsen), delay or slowing of the progression of a disease or condition, amelioration or palliation of the disease or condition, and remission (whether partial or total) of the disease or condition, whether detectable or undetectable. "Treatment" can also mean prolonging survival as compared to expected survival if not receiving treatment. Those in need of treatment include those already with the disease or condition as well as those prone to having the disease or condition or those in which the disease or condition is to be prevented.

In another embodiment, provided herein is a method for diagnosing amyotrophic lateral sclerosis (ALS) in a subject, the method comprising: obtaining a biological sample from said subject; and detecting the level of a chitinase 1 (CHIT1). The level of said CHIT1 in the range of, for example, 1,600-107,600 pg/ml may indicate that the subject has said ALS disease. Any biological sample can be used. In another embodiment, the level of CHIT is in the range of 500 pg/ml-500,000 pg/ml. In another embodiment, the level of CHIT is in the range of 500 pg/ml-400,000 pg/ml. In another embodiment, the level of CHIT is in the range of 500 pg/ml-300,000 pg/ml. In another embodiment, the level of CHIT is in the range of 500 pg/ml-200,000 pg/ml. In another embodiment, the level of CHIT is in the range of 1000 pg/ml-300,000 pg/ml. In another embodiment, the level of CHIT is in the range of 1000 pg/ml-200,000 pg/ml. In another embodiment, the level of CHIT is in the range of 1,500 pg/ml-200,000 pg/ml. In another embodiment, the level of CHIT is in the range of 1,500 pg/ml-150,000 pg/ml.

In some embodiments, the biological sample is a cerebrospinal fluid (CSF) sample from a subject. In some embodiments, the biological sample is a blood sample or plasma from a subject. In some embodiments, the biological sample is a urine sample from a subject. Based on the detection of said CHIT1, ALS in a subject can be treated.

The term "subject" includes but is not limited to a human. The methods of treatment described herein can be used to treat any suitable mammal, including primates, such as monkeys and humans, horses, cows, cats, dogs, rabbits, and rodents such as rats and mice. In a particular embodiment, the mammal to be treated is human.

All patents, patent applications, and scientific publications cited herein are hereby incorporated by reference in their entirety.

The following examples are presented in order to more fully illustrate embodiments disclosed herein. They should in no way be construed, however, as limiting the broad scope disclosed herein.

### EXAMPLES

### EXAMPLE 1

### Isolation and Differentiation of Human BM-MSC

### Preparation of Differentiated human BM-MSC

*Objective:* To produce human bone marrow-mesenchymal stem cells that secrete neurotrophic factors.

### Methods:

Methods for isolation of human bone marrow-mesenchymal stem cells (BM-MSC) are well known in the art and fully described in International Patent Application Publication Nos. WO 2015/121859 and WO 2014/024183, which are incorporated herein, in their entirety.

Bone marrow samples (30-60 ml) were collected into Heparin-containing tubes from the posterior iliac crest of adult human donors undergoing bone marrow aspiration in the course of diagnostic procedures. Bone marrow aspirates were diluted 1:1 with HBSS and mononuclear cells were separated by density centrifugation (1000xG for 20 min), over Ficoll (Ficoll-Paque PREMIUM) containing tubes. The mononuclear cell fraction was collected and washed in DMEM. Cells were re-suspended in Growth Medium containing 10% Platelet lysate (PM), counted by the Trypan blue exclusion dye and seeded at a concentration of 150,000-300,000 cells/cm² in 225 cm² tissue culture flasks. Flasks were incubated in a 37°C humidified incubator with 5% CO₂.

PM growth medium consisted of Dulbecco's Modified Eagle's Medium low glucose (Sigma, Aldrich), supplemented with L-Glutamine solution 200 mM (Sigma, Aldrich), Sodium Pyruvate solution 100 mM (Sigma, Aldrich), 2 IU/ml Heparin (APP Pharmaceuticals), and 10% platelet lysate. 16-24 hrs later PM medium was aspirated to remove non-adherent cells from the flask, adherent cells were washed gently with 10 ml of DMEM, and 30 ml of fresh PM were added to the flask. hMSC cells were allowed to proliferate for 12-15 days in PM medium, which was replaced twice weekly. After 12-15 days or when the flask reached confluence. The cells were harvested by removing all growth medium and incubating in TrypLE.™ solution (Invitrogen) for 5 min in a 37°C. incubator. Cells were then washed in DMEM, counted, resuspended in PM medium and seeded in CellStacks at a density of 1000-3000 cells/cm².

MSC cultures were passaged twice weekly by detachment of the sub-confluent cell layer with TrypLE.™ solution (Invitrogen). Cells were transplanted after 2-4 passages. Accordingly, the cells were passaged for a minimum of two weeks in PM prior to induction of differentiation.

Neurotrophic factor (NTF) secretion was induced by incubating the MSC for three days in Dulbecco's Modified Eagle's Medium low glucose (Sigma, Aldrich), supplemented with 1mM dibutyryl cyclic AMP (cAMP), 20 ng/ml human Basic Fibroblast Growth Factor (hbFGF), 5 ng/ml human platelet derived growth factor (PDGF-AA), and 50 ng/ml human Heregulin β1. The culture was maintained in differentiation medium for 3 days until harvesting.

hBM MSC-NTF cells (i.e., hMSC that had been induced to secrete NTF) were stable for about 5 to 72 hours at 2-8°C.

### Preparation of Platelet Lysate

Platelet lysate may be prepared using any method known in the art. For example, in one embodiment, a platelet lysate may be prepared using a freeze-thaw protocol as provided below.

Platelet Rich Plasma (PRP) may be from Blood Bank donations determined free of infectious agents (i.e. HIV, HTLV, HCV, HBsAg). PRP containing bags were stored at -80°C and thawed in a 37°C water bath. After thawing, the Platelet Rich Plasma of multiple donors was pooled, mixed and centrifuged at 14000xG for 10 minutes to remove platelet particles and membranes. The Platelet lysate supernatant was then collected and frozen at -80°C until use. The Platelet lysate was tested for Endotoxin, Haemoglobin, pH, Total protein, Albumin, Osmolality Sterility and Mycoplasma.

### EXAMPLE 2

### Characterization of differentiated human BM-MSC (hBM MSC-NTF)

*Objective:* To characterize differences between bone marrow MSC and the differentiated bone marrow MSC prepared in Example 1.

### Methods:

### Cells

Cells were obtained from healthy donors or from Amyotrophic lateral sclerosis (ALS) patients.

### Gene Expression-mRNA qPCR measurements

The expression of several genes was compared in bone marrow (BM) MSC and BM derived MSC-NTF cells using Quantitative reverse transcription Polymerase Chain Reaction (RNA qPCR). Briefly, target genes were normalized to the geometric mean of Beta 2 microglobulin (*B2M*), Elongation Factor 1 alpha (*EF1α*), and ribosomal Protein L17 (*RPL17*) which were chosen from a panel of published normalizing genes, since they were found to be equivalently expressed in MSC and MSC-NTF cells. In the RNA qPCR the crossing point (Cp) is the cycle at which fluorescence from the amplification exceeds the background fluorescence. A lower Cp value correlates with a higher expression of the gene being analyzed. Samples with qPCR Cp >35 were considered as not detected and for calculations were given a Cp value of 35. P-value was calculated using a 2-tailed paired t-test. Healthy donors were D12, D26, D22, D23, and D24. ALS patients were 07-RI, 08-RS, 11-AG, and 12-NM.

### Secretion measurements

Secretion from MSC and MSC-NTF of target compounds and polypeptides was measured using enzyme-linked immunosorbent assays (ELISA), for example Quantikine® (an example of an ELISA kit) or Ray Bio commercial assay kits (an example of a multiplex assay system) as are known and available.

### Results

Gene expression was analyzed by measuring mRNA expression in MSC and MSC-NTF from both healthy donors and ALS patients. Genes analyzed included cell cycle genes (such as TOP2A), secreted genes (BMP2, LIF, FGF2, WNT5A, AREG, HGF, BDNF), mesenchymal genes (MEST), secretion-related genes (PCSK1, RAB27B, SNAP25), glutamate transporter genes [SLC1A1 (EAAC1), and SLC1A3 (GLAST)], neuronal genes TUBB3 (TUJ1), and SLC16A6.

Results showed significant decrease (<0.0001) in *Top2A* expression, in MSC-NTF vs MSC of normal donors suggesting the MSC-NTF cells are in cell cycle arrest (**Figure 1A**). **Figure 1B** shows the per sample fold change, wherein there was a 71 fold decrease of expression of *Top2A* expression (MSC-NTF/MSC). (P-value = 2.9x10⁻⁷ n=9)

Significant increase in BMP2 (53.7 fold) and LIF (6.8 fold) expression was observed in MSC-NTF vs MSC of normal donors and ALS patients, demonstrating the MSC-NTF have increased expression of the neurotrophic factor gene LIF (**Figures 2A & 2B****, and** **Figures 3A & 3B**). Decrease in FGF2 expression was observed in some donors' MSC-NTF cells (cells derived from patients were not tested) (**Figures 4A & 4B**). The P-value for these results were 9.73x10⁻⁹ (n=9) for BMP2, 6.9x10⁻⁴ (n=9) for LIF, and 0.015 (n=5) for FGF2. The increased expression of the BMP2 gene correlated well with the increased BMP-2 secretion observed in MSC-NTF cells, as compared to MSC cells, from donors and from ALS patients in the Phase 2a clinical trial (**Figures 17A & 17B**). Similarly, the increased LIF expression correlated with the increased secretion of the neurotrophic factor, LIF, observed in MSC-NTF cells (14 fold as compared to MSC) in the vast majority of normal donors and ALS patients in the Phase 2a and Phase 2 clinical trials (**Figures 19A** **&19B**).

Significant increase in WNT5A (2.4 fold change), expression and AREG (Amphiregulin, a member of the epidermal growth factor family, 20.1 fold change) expression was observed in MSC-NTF vs MSC of normal donors (AREG) and patients (WNT5A) (**Figures 5A & 5B and Figures 6A & 6B****,** respectively). The P-value for these results were 0.001 (n=9) for WNT5A and 0.023 (n=5) for AREG.

Similarly, significant increase in HGF (-2.3 fold change) expression and BDNF (-1.5 fold change) expression in MSC-NTF vs MSC of normal donors and ALS patients was observed (**Figures 7A & 7B** and **Figure 8A & 8B****,** respectively). The P-value for these results were 0.042 (n=9) for HGF and 0.040 (n=9) for BDNF.

Analysis of the mesenchymal gene, MEST, showed significant decrease (0.14 fold change; P-value of 2.1x10⁻⁴ (n=9)) in MEST (Mesoderm-specific transcript) expression in MSC-NTF vs MSC of normal donors and patients (Figures 9A and 9B).

Expression of secretion related genes showed an increase in PCSK1 (Proprotein convertase 1) and RAB26B in MSC-NTF vs MSC of patients and healthy donors (Figures 10A & 10B (P-value=9.8x10⁻⁷ n=9) and **Figures** 11A & 11B (P-value =0.001 n=9), respectively). Increase in SNAP25 expression was observed in donor's MSC-NTF vs MSC, and was not measured in patients (Figures 12A & 12B; P-value=6.77x10⁻⁴ n=5). PCSK1 and SNAP25 were not detected (Cp>35) in any of the MSC samples, therefore, an arbitrary value of Cp=35 was given for the fold-change calculations for these genes.

Expression of glutamate transporter genes SLC1A1 and SLC1A3 showed increase in SLC1A3 (GLAST- an astrocytic glutamate transporter) expression (7.9 fold change) (**Figures 14A & 14B****;** P-value=0.007 n=9), and decreased expression of SLC1A1 (EAAC1 expression - a neuronal glutamate transporter) (1.66 fold change) (**Figures 13A & 13B****;** P=0.032 n=5). These results correlated well with the data showing increased GLAST expression in MSC-NTF from ALS patients compared with MSC from the same donors (**Figure 14**). Further, in most of the patients, MSC-NTF cells secretes less glutamate as compared to the MSC cells from which they are derived (**Figure 21**). Since GLAST is a transporter that removes glutamate from the extracellular space, it's increased expression may explain the decrease of glutamate in the MSC-NTF cells culture supernatants, as compared to the MSC cells culture supernatants.

Analysis of the neuronal gene TUBB3 showed TUBB3 expression was down regulated (by 3.4 fold (**Figures 15A & 15B**; P-value=0.027 n=5) in MSC-NTF cells of normal donors. In addition, analysis of SCL16A6 showed expression was upregulated by 29.6 fold in MSC-NTF vs. MSCs (**Figures 16A** **&16B;** P-value=0.031 n=5). Interestingly, SLC16A6 expression was found to be increased in many MSC manipulations described in the literature, yet it was not expressed (Cp>35) in any MSC sample.

Analysis of TSG-6 secretion showed that TSG-6 was found to be secreted to the culture supernatant of MSC-NTF but not that of MSC cells for most ALS patients (**Figure 20**).

### G-CSF

For many years granulocyte colony-stimulating factor (G-CSF) has been considered a highly specific hematopoietic growth factor. More recent studies have shown the presence of the G-CSF/G-CSF-receptor (G-CSFR) system in the brain and roles in neuroprotection, neural tissue repair as well as improvement in functional recovery have been described. In a mouse model of ALS, G-CSF significantly improved motor performance and motor-neuron survival, and reduced denervation atrophy. Based on pre-clinical findings several smaller studies in ALS patients have been initiated with promising outcomes.

The secretion of G-CSF was evaluated in the culture supernatant of MSC-NTF cells of ALS patients and normal donors as compared to the MSC of the same subject prior to differentiation using an ELISA assay for G-CSF (R&D Systems). The results show specific productivity of G-CSF secretion of pg/10⁶ cells in MSC-NTF cells.

There was no detectable secretion of G-CSF in the MSC while there were significant amounts of G-CSF secreted by the MSC-NTF cells (**Figures 18A and 18B**).This increased expression and secretion of neurotrophic factors, measurements showing increased G-CSF secretion in MSC-NTF cells (as compared to MSC) was observed in most (but not all) normal donors and ALS patients in two clinical trials (**Figures 18A & 18B**). Similarly, there was increased secretion of LIF (**Figures 19A** **and** **19B**) and TSG-6 (**Figure 20**) in MSC-NTF as compared with MSC obtained from ALS patients.

### Summary

The increase in secretion-associated genes (PCSK1 and RAB26B suggest that the differentiation process induces secretory mechanisms that are reflected in the detection of multiple secreted neurotrophic factors such as VEGF, HGF, LIF, BMP2, etc etc.. The decrease in MEST suggests a change from the naive MSC phenotype following the differentiation process. The decrease in the cell-cycle related gene TOP2A, correlates with the cell-cycle arrest and reduced proliferation of the MSC-NTF cells and their differentiation.

### EXAMPLE 3

### Modulation Of Neurotrophic And Inflammatory Factors In The CSF Of ALS Patients Treated With MSC-NTF Cells

### Objective:

The primary objective of this study was to evaluate the safety of transplantation of expanded autologous MSC-NTF cells described in Examples 1 and 2.

### Materials and Methods

### BM-MSC-NTF cells (NurOwn®)

MSCs that have enhanced secretion of neurotrophic factors (NTFs) offer a novel method for delivering multiple NTFs to patients suffering from neurodegenerative diseases, such as amyotrophic lateral sclerosis (ALS) patients, while simultaneously leveraging the potential therapeutic benefits of MSCs, such as immunomodulatory effects. Culture methods have been developed that can induce MSCs to secrete enhanced levels of various neurotrophic factors (NTF). NTFs that may be secreted from BM-MSC have been described in details above including glial derived neurotrophic factor (GDNF), vascular endothelial growth factor (VEGF), leukemia inhibitory factor (LIF), Granulocyte Stimulating factor (G-CSF), BMP-2, TSG-6 and hepatocyte growth factor (HGF) (*See also* Example 2 *Results*). NTF-secreting MSCs (MSC-NTF cells or NurOwn®) are an autologous therapy generated from bone marrow derived MSCs (*See* Example 1). In one embodiment, the terms "MSC-NTF" and "NurOwn" may be used interchangeably having all the same meanings and qualities.

Briefly, the production of MSC-NTF cells begins with isolation of MSCs from the mononuclear fraction of the patients' bone marrow aspirate, *ex vivo* propagation, and induction under proprietary culture conditions that induce NTF secretion. MSC-NTF cells have been administered in animals via intramuscular (IM), intrathecal (IT), and intracerebroventricular (ICV) administration, and to ALS patients via IM and IT administration alone or in combination..

### Double-Blind Placebo-Controlled clinical trial

A phase 2 multicenter double-blind placebo-controlled study in ALS patients entitled 'A Phase 2 Randomized, Double-Blind, Placebo-Controlled Multicenter Study of Transplantation of Autologous Mesenchymal Stem Cells Secreting Neurotrophic Factors in Patients with Amyotrophic Lateral Sclerosis has been recently completed at three major US medical centers (ClinicalTrials.gov Identifier: NCT02017912). At completion of the study the Data and Safety Monitoring Board (DSMB) reviewed safety data and concluded that the treatment was tolerated extremely well and there were no serious adverse events related to the therapy. The safety profile shows that ALS can be treated by the methods described herein.

### Study endpoints

The primary endpoint of the study was aimed at evaluating the safety and tolerability of transplantation of expanded autologous MSC-NTF cells administered on a single occasion via combined intrathecal (IT) administration and 24 intramuscular (IM) injections given into the right biceps and triceps muscles in patients with ALS.

The secondary endpoints were efficacy, comparing the change in disease progression from the pre-transplantation period to the post-transplantation period between the treatment and placebo groups as determined by the ALS Functional Rating Score Revised (ALSFRS-R, a validated outcome measure of ALS disease progression) and Slow Vital Capacity (SVC) a pulmonary function. Secondary endpoints were evaluated in treatment and placebo groups through 24 weeks post-transplantation time period, relative to the 12-16 week baseline period before transplantation. Dulbecco Modified Eagle Medium (DMEM) was used as placebo.

### Exploratory endpoints:

To compare the slopes of decline of HHD, and electrical impedance myography (EIM, for patients in whom this optional outcome measure is available) against placebo as well as during the 12-16-week pre-transplant monitoring period relative to the 24-week post-transplant follow-up period.

To determine feasibility of succeeding in blinding patients and caregivers to the treatment, in anticipation of a requirement to include a placebo group in a Phase III efficacy study.

To examine the cerebrospinal fluid (CSF) of ALS patients at Baseline and 2 weeks after treatment for the presence of neurotrophic factors and possible ALS biomarkers.

### Study Overview

Participants who met eligibility criteria were evaluated at a subsequent visit during which eligibility was confirmed. Subsequently participants were randomized and the bone marrow aspiration performed. MSC-NTF cell transplantation occurred three to four weeks following bone marrow aspiration. For transplantation, all participants were admitted to the hospital for 48 hours of monitoring. Subsequent outpatient study visits occurred two weeks after transplantation and then every four weeks for six months (**Figure 22A**).

### Methods

The study protocol was approved by the FDA and by the institutional review board (IRB) of each participating site. All participants signed informed consent. The study was conducted in accordance with Good Clinical Practice. (ClinicalTrials.gov identifier NCT02017912).

### Participant Selection Criteria

At screening, eligible participants had a diagnosis of possible, probable, laboratory-supported, probable, or definite ALS by El Escorial Criteria, ALSFRS-R ≥30, vital capacity (VC) ≥65% of the predicted normal value for height, age and gender, and symptom onset duration of greater than 1 year and less than or equal to 2 years. Participants were either not receiving riluzole or were on a stable dose for ≥ 30 days, had the ability to lie flat for the IT cell transplant procedure, had at least some limb weakness due to ALS, and were residents of the United States. Potential participants were excluded for the following reasons: use of mechanical ventilation; presence of feeding tube; prior treatment with stem cells; pregnancy; exposure to investigational agents or immunosuppressive therapy within 4 weeks of screening; active autoimmune disease or infection (including Hep B, Hep C or HIV), cancer within the previous five years; or unstable psychiatric or medical condition or clinically significant abnormal safety laboratory values.

### Participant Evaluations

Fifty-nine participants were screened for this trial. There were eight screen failures, and during the three-month run-in period, three participants discontinued the study prior to randomization. Forty-eight participants were randomized of which 43 completed follow-up (**Figure 22C**).

Treatment arms showed similar baseline characteristics. The majority of the treated participants were male (35 participants, 72.9%) and white (48 participants, 100%). The mean age of the participants was 51.1 years (range: 26 to 71 (Tables 1A and 1B below).

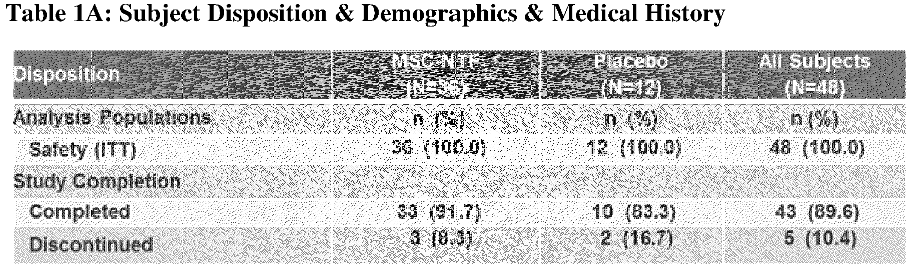

**Table 1B: Summary of Demographics and Baseline Characteristics by Treatment group***

| | **MSC-NTF** | **Placebo** | **All Participants** |
|---|---|---|---|
| | **(N=36)** | **(N=12)** | **(N=48)** |
| | **n (%)** | **n (%)** | **n (%)** |
| Sex | | | |
| Male | 25 (69.4) | 10 (83.3) | 35 (72.9) |
| Female | 11 (30.6) | 2 (16.7) | 13 (27.1) |

| **Age (years)** | | | |
|---|---|---|---|
| Mean (SD) | 50.3 (11.90) | 53.5 (9.11) | 51.1 (11.27) |

| **El Escorial Criteria** | | | |
|---|---|---|---|
| Possible | 3 (8.3) | 1 (8.3) | 4 (8.3) |
| Laboratory-Supported Probable | 5 (13.9) | 1 (8.3) | 6 (12.5) |
| Probable | 16 (44.4) | 7 (58.3) | 23 (47.9) |
| Definite | 12 (33.3) | 3 (25.0) | 15 (31.3) |

| **ALS Medical History: Months Since Diagnosis** | | | |
|---|---|---|---|
| Mean (SD) | 9.00 (5.578) | 9.01 (4.637) | 9.00 (5.311) |

| **ALS Medical History: Months Since First Symptom** | | | |
|---|---|---|---|
| Mean (SD) | 17.62 (3.796) | 16.75 (3.104) | 17.40 (3.624) |

| | | | |
|---|---|---|---|
| * Abbreviations: ALS = Amyotrophic lateral sclerosis; SD = standard deviation Data are presented as n and percentages or means (±SD). Abbreviations: ALSFRS-R= ALS functional rating scale revised. Percentages are based on the number of participants (N) in a given treatment group for the population being analyzed. | | | |

After providing informed consent, each patient underwent approximately 12-16 week baseline period monthly visits during which ALS Functional Rating Scale (ALSFRS) and revised ALSFRS-R (ALSFRS-R) scores and slow vital capacity SVCs were obtained. During this period of time the patients' bone-marrow was harvested and mesenchymal stromal cells isolated and expanded, as described above in Example 1.

Briefly, patient bone marrow was aspirated about 9-11 weeks following the first screening visit. The MSC isolation and cell propagation process lasted about 3-5 weeks and was followed by MSC-NTF cells transplantation.

Twelve to 16 weeks after screening, patients underwent transplantation with their autologous MSC-NTF or matching placebo; autologous MSC-NTF or placebo were administered both intrathecally (IT) as well as intramuscularly (IM) as 24 IM injections given into the right biceps and triceps muscles, all at a single study visit (**Figure 22B**). In each case, placebo was the excipient matching the excipient in which the MSC cells were suspended.

Dosages of autologous MSC-NTF administered were as follows: each of the 24 IM administrations contained 2 x 10⁶ MSC-NTF in 200µl (cell concentration was 10 x 10⁶ cells/ml), and 100-125 x 10⁶ MSC-NTF in 4 ml were used for the IT administration. Thus, the MSC-NTF cell dose was: 100-125 x 10⁶ cells by IT administration and a total of 48 x 10⁶ cells by IM administration.

Following treatment patients were followed in up to six monthly visits (24-26 weeks from transplantation date; assessed at 2, 4, 8, 12, 16, and 24 weeks) at which the ALS Functional Rating Scale (ALSFRS) and revised ALSFRS-R (ALSFRS-R) and slow vital capacity (SVC) were obtained, along with vital signs, laboratory tests and recording of concomitant medications and adverse events (AEs, see scheme in **Figure 1A**).

In the course of the study cerebrospinal cord fluid (CSF) samples were collected from all patients pre-treatment on the day of administration (at study Visit 5) and 2 weeks post treatment (at study Visit 6) aiming to identify the presence of secreted neurotrophic factor biomarkers and their correlation to treatment.

### Randomization and Blinding

This was a randomized double-blind placebo-controlled study. Participants were stratified by site and randomly assigned at a 3:1 ratio to MSC-NTF cells or matching placebo. To maintain blinding, bone marrow was aspirated from all participants and in addition participants randomized to placebo were administered excipient at the time of the transplantation procedure.

### Bone Marrow Aspiration

All participants, regardless of randomization group, underwent bone marrow aspiration, performed by a licensed physician using standard practice techniques. A total of 50-70 mL of bone marrow was aspirated from each participant.

### Cell Manufacturing and Transplantation

MSCs were isolated from the bone marrow, expanded and differentiated in culture to secrete NTFs using a culture based approach, as described in detail in Example 1. The MSC-NTF cells production was carried out under full environmental control, in cleanrooms compliant with good manufacturing practices (GMP).

Autologous MSC-NTF cells were released for transplantation when they fulfilled the applicable release criteria including safety, potency and identity tests.

On the day of transplantation, following the release testing, the MSC-NTF cells were provided in a ready-to-use, participant-customized, treatment package consisting of one 5 mL syringe for IT transplantation, and 24 1 mL syringes for IM transplantation. Placebo (excipient) was provided in the same number of syringe types and sizes as the cell-containing syringes.

Because the MSC-NTF cells and placebo syringes had slightly different appearance, the injections were performed by unblinded injection teams, and the packaging was not opened in the presence of the blinded treatment team. Participants were draped to obscure their view of the transplant material and procedure. Post-transplant evaluators were blinded to the treatment allocation.

### Outcome Measures

Adverse events were assessed throughout study duration. Safety labs were collected at each visit, analyzed at a central laboratory and monitored by the site investigator, medical monitor, and the drug safety monitoring board (DSMB). Clinical outcome measurements were collected at all visits by trained evaluators at each site, certified by the Outcomes and Monitoring Center for the Northeast ALS Consortium at Barrow Neurological Institute, and included slow vital capacity (SVC), ALSFRS-R, hand-held dynamometry (HHD). At the week 12 and 20 post-treatment visits ALSFRS-R was administered over the phone. Cerebrospinal fluid was collected by lumbar puncture immediately before and two weeks after transplantation starting with the ninth participant, following a protocol amendment. To assess blinding, all participants and treating physicians were asked whether they believed they had received cells or placebo at the end of the transplantation visit and at the final follow-up visit of the trial.

### CSF Collection and Analysis

Cerebral spinal fluid (CSF) was collected from ALS patients undergoing intrathecal administration of MSC-NTF cells or a placebo lacking any cells, at study visit 5 (**Figure 22A****; V5 throughout**), prior to cell administration (transplantation) and approximately two weeks later at visit 6 (Figure 22A; V6 throughout) by standard lumbar puncture.

Cerebrospinal fluid (CSF) collected prior to cell administration and two weeks post transplantation by lumbar puncture, was immediately centrifuged at 1750-g for 10 minutes, aliquoted and stored at -80 °C. Twenty-six sample pairs of treated, and nine sample pairs of placebo patients for whom pre and post-transplantation samples were available, were analyzed.

### Multiplex Analysis of CSF

Biomarkers were analyzed using a Multiplex immunoassay (customized Procarta immunoassay, Affymetrix, Santa Clara, CA, USA), according to the manufacturer's instructions. Mean fluorescence intensities of analyte-specific immunoassay bead sets were detected by a flow-based Luminex 3D suspension array system (Luminex, Austin, TX, USA).

### CSF ELISA

Chitotriosidase (CHIT-1) levels were measured using a commercially available kit per manufacturer's instructions.

In addition, ELISA assays were performed using commercially available ready-to-run ELISA kits for the detection of VEGF, HGF, and LIF (Quantikine, R&D Systems) per manufacturer's instructions.

### Multiplex analyses

Inflammatory, neurotrophic and other factors were detected in the CSF using a custom made Multiplex analysis kit (Affymetrix, eBiosience).

### Quantification of CSF microRNA

Due to the limited availability of patients' CSF, equal volumes of CSF samples of responders, non-responders and of placebo patients were pooled to evaluate miRNAs expression pre- and two weeks post-treatment (V5 and V6 respectively). To minimize pool limitations, groups of responders were defined as those responding to treatment at least 12 weeks post transplantation by an ALSFRS-R improvement of 1.5 points/month or more, and non-responders were defined as those patients with no improvement 4 weeks post transplantation.

Equal amounts of CSF from patients within each group were pooled together for a total of 180µl/pool. Each pool was supplemented with 2µg of RNA grade glycogen (Thermo Scientific, Waltham, Massachusetts) as a carrier and Molecular biology water (Sigma) were added to a final volume of 200µl. Pools were spiked with 0.03 fmole of UniSp6 (Exiqon, Denmark) and RNA was subsequently isolated using miRCURY RNA Isolation Kits - Biofluids (Exiqon, Denmark) according to manufacturer's instructions.

Reverse transcription was performed using Universal cDNA Synthesis Kit II (Exiqon, Denmark) according to manufacturer's instructions. microRNA qPCR measurements were performed by LightCycler480 ii (Roche Molecular Diagnostics, Pleasanton, California) using ExiLENT SYBR Green master mix and LNA primers (both Exiqon, Denmark). The expression of each microRNA was calculated relatively to the expression of the UniSp6 spike-in.

### Quantification of Glutamate in CSF

Two acceptable assays known in the art were used to measure glutamate- HPLC and a colorimetric assay. Both methods showed similar relative results. The colorimetric assay showed better repetitions and thus the analysis continued with this method.

### Change in ALSFRS-R score in patients on riluzole as compared to those not on riluzole

Patients who were on riluzole vs. those not on riluzole on the day of transplantation were analyzed separately to evaluate the effect of riluzole on change in ALSFRS-R score post transplantation. No noticeable effect of riluzole was observed on the ALSFRS-R total scores.

In patients taking riluzole, at all time points except at 2-weeks (≥1.0 and ≥1.5 threshold), 16-week and 24-weeks (≥2.5 threshold), percent responders were greater in MSC-NTF cells group compared to the placebo group. The change observed in ≥0.5-point score at 12-weeks favoring MSC-NTF cells over placebo group was statistically significant (38.9% vs 0; one-sided, p=0.09) (Data not shown).

In patients not taking riluzole, improvement was seen in the MSC-NTF cells group compared to the placebo at all time points except at 8-weeks (≥0.5 and ≥1.5 threshold), 12-weeks (≥1.5), 16-weeks and 24-weeks (≥0.5 and ≥1.5 threshold) and were statistically significant at 2-weeks for ≥0.5 and ≥1.0 points (one-sided, p≤0.09) and 4-weeks for ≥1.5 point (one-sided, p=0.09) **(****Figures 27A and 27B****,** and Data not shown).

In both the subgroups, patients on riluzole and patients not taking riluzole, the percent of responders were greater in the MSC-NTF cells group compared to the placebo at all time points except at 8-weeks (75% and 100% threshold) in the subgroup of patients on riluzole treatment. The changes observed in patients not taking riluzole at 2-weeks (one-sided, p=0.06) favoring the MSC-NTF cells were statistically significant.

### Statistical Analysis

The primary trial outcome was safety assessed with respect to the incidence of treatment-emergent AEs and SAEs, lab abnormalities, vital signs, ECGs and physical exams. AEs were coded using MedDRA.

Pre-specified efficacy analyses in the Statistical Analysis Plan (SAP) included comparison of ALSFRS-R slopes post-treatment vs. pre-treatment between treatment groups using model fit least-squares means as well as responder analyses comparing the MSC-NTF and placebo groups. Responder analyses were pre-specified and defined as a 20-30% improvement in post-treatment slope compared to pretreatment.

To further expand the responder analysis, various responder thresholds and definitions were used. Two methods for defining cutoffs were examined: 1) defining an exact change in ALSFRS-R slope in points per month and 2) using a percent change in ALSFRS-R post-treatment slope compared to pre-treatment slope. For each method, the post-transplantation slope was compared with the pre-transplantation slope and a range of thresholds for responders (≥0.5 to ≥2.5 points/months; and ≥25% to ≥100% change) was defined at each follow-up visit.

Three pre-specified subgroup analyses included: ALSFRS-R overall pre-treatment score change (< -2 points 'fast progressors' or ≥ -2 points 'slow progressors'; ALSFRS-R motor pre-treatment score change (< -1 points vs ≥ -1 points); and baseline SVC (≥ 70%). Secondary and exploratory objectives included analyses of SVC, HHD and CSF pharmacodynamic markers. Statistical significance for the LS means was determined if the p-value was ≤0.05 (two-sided) and for responder analyses if the p-value < 0.05 (two-sided Fisher's exact test).

The CSF data was statistically assessed for significance by either Student's t test or ANOVA analysis as applicable. A p value of less than 0.05 was considered statistically significant. CSF collection

### Results

### CSF collection

CSF collection began following submission of an amendment to the Protocol. Only pre- and post-transplant paired samples were analyzed. In total, 26 sample pairs of treated and 9 sample pairs of placebo were available for analysis.

### Neurotrophic factors in the CSF of ALS patients

NTFs were measured in the CSF samples of patients pre- and two weeks post-treatment. Levels of VEGF, HGF, and LIF were found to significantly increase in the CSF of treated but not placebo patients (range p<0.05 - p<0.001, Table 2), indicating the presence of the cells two weeks post treatment and their activity as evidenced by the continued secretion of these neuroprotective factors.

**Table 2: Post-treatment secretion of neurotropic factors by MSC-NTF cells into the CSF of ALS patients**

| | **Treated** | | | **Placebo** | | |
|---|---|---|---|---|---|---|
| NTF | **V5** | **V6** | p value | **V5** | **V6** | p value |
| | Titer (pg/ml, Average ± SEM) | Titer (pg/ml, Average ± SEM) | | Titer (pg/ml, Average ± SEM) | Titer (pg/ml, Average ± SEM) | |
| **VEGF** | 37.7 ± 3.1 | 667.5 ± 243 | 0.0159 | 30.6 ± 4.8 | 29.8 ± 4.9 | 0.4568 |
| **HGF** | 391 ± 22.7 | 498 ± 37.4 | 0.0046 | 448±48.8 | 467 ± 41 | 0.4221 |
| **LIF** | 0 | 12.33 ± 3.2 | 0.0008 | 0 | 0 | na |

A significant increase in VEGF, HGF and LIF was observed post-transplantation (**Figures 23A-23F**). Average basal levels of VEGF were 37.1±15.8pg/ml (Mean ± SEM) in treated patients and 30.6±14.4 pg/ml in placebo patients. Post-transplantation, the mean VEGF increase in the treated patients was 629.82±243.352 (p=0.016) and -0.78±0.995 (p=0.45) in placebo patients. (**Figures 23A and 23B**). CSF was found to have high basal levels of HGF (treated patients- 391.1±115.9 pg/ml; placebo patients- 448.4±146.3pg/ml). Following transplantation, HGF levels significantly increased in the MSC-NTF cells treated participants (107.18±34.444; p=0.0046) while there was minimal change in those receiving placebo (18.82±22.25; p=0.42). (**Figures 24C and 24D**). LIF was undetectable in the CSF prior to transplantation and significantly increased (12.33±3.227; p=0.0008) post-transplantation in treated participants, while there was no detectable LIF in the placebo group either pre- or post-transplantation (**Figures 23E and 23F**).

### Inflammatory factor and pro-apoptotic biomarkers in the CSF of ALS patients

A large amount of evidence points to the involvement of inflammatory processes in development and progression of ALS. The presence of inflammatory factors in the CSF was analyzed pre- and post- MSC-NTF cells treatment. A statistically significant decrease in the levels of inflammatory factors was found in the CSF of treated but not placebo patients (Table 3), indicating the presence of the cells and their continued immunomodulatory and anti-inflammatory effect two weeks post-treatment. The presence of high level of neurotrophic factors in the CSF of treated but not Placebo patients, two weeks post treatment, provides a clear indication of the persistence of the MSC-NTF cells in the CSF of the treated patients leading to the efficacy signals observed .

**Table 3: Modulation of Inflammatory factors and Caspase 3 in the CSF of ALS patients after treatment with MSC-NTF cells**

| | **Treated** | | | **Placebo** | | |
|---|---|---|---|---|---|---|
| Factor | **V5** | **V6** | p value | **V5** | **V6** | p value |
| | Titer (pg/ml, Average ± SEM) | Titer (pg/ml, Average ± SEM) | | Titer (pg/ml, Average ± SEM) | Titer (pg/ml, Average ± SEM) | |
| **CRP** | 1092±175 | 882±145 | 0.1036 | 1213±267.5 | 1037±311.5 | 0.4850 |
| **MCP-1** | 42.3±1.845 | 25±1.6 | 1.0265E-09 | 40.5±3.17 | 39.59±2.5 | 0.7082 |
| **SDF-1** | 195.6±6.1 | 149±11.7 | 0.0014 | 200.8±5 | 201±7 | 0.9592 |
| **CHIT** | 28729±5028 | 27465±4981 | 0.0310 | 30983±9536 | 33545±11382 | 0.2646 |
| **MIP-1b** | 8.1±0.73 | 8.3±1.3 | 0.8999 | 4.7±0.9 | 4.9±1 | 0.7999 |
| **Caspase-3** | 3.6±0.4 | 1.8±0.3 | 3.8592E-05 | 1.8±1.1 | 1.8±1.7 | 0.2771 |

Post-transplantation results showing the significant reduction in some of the inflammatory markers tested (MCP-1, SDF-1, and CHIT-1) observed in the MSC-NTF cells treated participants is presented in **Figures 24A, 24C, 24E** while no change was observed in the placebo group (**Figures 24B, 24D, 24F**). Basal levels of MCP-1 were 42.2±9.4 pg/ml in treated participants and 40.5±9.5 pg/ml in the placebo group. Post-transplantation, mean MCP-1 levels decreased significantly in the MSC-NTF cells treated participants (-17.29±1.83pg/ml; p<0.0001) while no significant change was observed in the placebo group (-0.89±2.29pg/ml; p=0.7). (**Figures 24A and 24B**) The post transplantation difference between MSC-NTF treated participants and the placebo group was statistically significant (p<0.0001). Average basal levels of SDF-1a were 195.6±31.1 pg/ml in MSC-NTF treated participants and 200.8±14.99 pg/ml in the placebo group. Post-transplantation, SDF-1a significantly decreased in the MSC-NTF treated participants (-46.57±12.93pg/ml; p=0.0014) while it remained unchanged in the placebo group (0.27±5.049pg/ml; p=0.96). (**Figures 24C and 24D**) The post-transplantation difference between the two groups was statistically significant (p=0.04). There was no significant post-transplantation change in the levels of MIP-1β and C-reactive protein (CRP) in either group (data not shown).

The levels of Caspase 3, one of the key mediators of apoptosis. Interestingly, Caspase-3 was significantly reduced post-transplant in MSC-NTF treated participants (-2.09±0.429; p<0.0001) but not in the placebo group (-0.91±0.922; p=0.35).

Minimal differences were observed for CRP and MIP-1β (**Figures 24G and 24H,** and **Figures 24I and 24J****,** respectively)

### Correlation between CSF Neurotrophic and inflammatory biomarker factors

Statistically significant correlations were identified between the increase in neurotrophic factors and the decrease in inflammatory factors in the CSF of treated but not placebo patients (Table 4), indicating a direct effect of the NTFs secreted by the MSC-NTF cells on the decrease in the levels of the inflammatory factors in the CSF.

**Table 4: Correlation between increased NTFs and decreased inflammatory markers ***

| **Correlation** | **Treated** | | | | **Placebo** | | | |
|---|---|---|---|---|---|---|---|---|
| | Pre-transplant | | Post-transplant | | | | Post-transplant | |
| | Correlation | p value | Correlation | p value | Correlation | p value | Correlation | p value |
| **VEGF/MCP-1** | -0.017 | 0.9326 | -0.564 | 0.0027 | -0.005 | 0.9904 | 0.214 | 0.5804 |
| **VEGF/SDF-1** | 0.236 | 0.2463 | -0.765 | <0.0001 | -0.098 | 0.8025 | -0.209 | 0.5886 |
| **HGF/SDF-1** | 0.119 | 0.5614 | -0.412 | 0.0363 | -0.388 | 0.3025 | -0.14 | 0.7194 |
| **LIF/SDF-1** | NA | NA | -0.534 | 0.0049 | NA | NA | NA | NA |
| **LIF/MCP-1** | NA | NA | -0.422 | 0.0316 | NA | NA | NA | NA |

| | | | | | | | | |
|---|---|---|---|---|---|---|---|---|
| *p values calculated by ANOVA. p values in highlighted cells are statistically significant. *NA- Not Applicable | | | | | | | | |

Two weeks post-transplant a statistically significant inverse correlation (Table 3) was observed between VEGF and MCP-1 (correlation -0.56, p=0.003, **Figures 25A and 25B**), VEGF and SDF-1a (correlation -0.77 p <0.0001), HGF and SDF-1a (correlation-0.41 p =0.004), LIF and SDF-1 (correlation-0.53 p=0.005) and LIF and MCP-1 (correlation-0.422 p =0.034) in MSC-NTF cells treated participants but not in the placebo group.

The statistically significant inverse correlation (p<0.01) detected between the increase in VEGF and the decrease in MCP-1 in the CSF post treatment with MSC-NTF cells proved for the first time *in vivo* that the biological function (mode of action) of the cells is mediated by secretion of multiple trophic factors (**Figures 25A and 25B**).

### RESPONDER ANALYSES (Efficacy Analysis)

### Mean Slope change in all participants and in pre-specified subgroup excluding slow progressors

The pre-treatment ALSFRS-R slopes were similar in the two groups: -0.7 pts./mo. (MSC-NTF arm) and -0.6 pts./mo. (placebo arm). The change in the ALSFRS-R slope post-transplant was +1.7 pts./mo.(MSC-NTF) and -0.4 pts./mo.(placebo) at two weeks (p=0.110), +0.6 pts./mo.(MSC-NTF) and -0.03 pts./mo.(placebo) at four weeks (p=0.368). After eight weeks, the change in slope compared to pre-treatment slope was similar in each arm (**Figure 26A**).

### Change in ALSFRS-R Total Score

The results of change in ALSFRS-R total score for the population excluding slow progressors is presented in Table 7. The LS mean change from baseline showed an improvement in the MSC-NTF cells group compared to the placebo at the 2-week (LS mean: 0.90 vs -1.20), 4-week (LS mean: 0.50 vs -1.00) and 16 week (LS mean: -3.40 vs -7.80) follow-up time points. Statistically significant improvement was observed based on the LS mean difference favoring the MSC-NTF cells group at the 2 week, 4 week, and 16 week follow-up time point (p=0.02, 0.10, and 0.15, respectively). Graphical representation of the LS mean change in ALSFRS-R total score for subjects excluding slow progressors is presented in Figures **26C, 26D****, and 27E**.

In the pre-specified subgroup excluding slow progressors (i.e., excluding those with a decline of less than 2 points in ALSFRS-R scores during the 3-month period between screening and baseline), there were 15 participants (42%) in the MSC-NTF arm and six (50%) in the placebo arm. In an analysis of this subgroup, the pre-treatment ALSFRS-R slopes were comparable: MSC-NTF arm -1.5 and placebo arm -1.2 (p=0.232). Comparison of LS means of the post-transplant ALSFRS-R slope minus the pre-transplantation slope between the MSC-NTF and placebo groups demonstrated a significant improvement in the MSC-NTF group at 2 and 4 weeks (+3.3 vs -1.3; p=0.021, and+2.0 vs -0.1; p=0.033, respectively) and a continued trend for improvement in the MSC-NTF group at all remaining time points (**Figure 26B**).

In an analysis of the correlation between the neurotrophic factors secreted by the MSC-NTF cells into the CSF and the clinical response of the patients treated, as evaluated by the ALSFRS-R score (a validated outcome measure of ALS disease progression) it was found that the VEGF is significantly increased in the CSF of those patients responding to treatment as determined by the 50% improvement in post-transplant as compared to pre-transplant ALSFRS-R slope at 8 weeks post-transplant (**Figures 23A-23B****,** and **23G**).
The correlation between the increased levels of the other neurotrophic factors, the reduction in the inflammatory factor levels in the CSF and the clinical response, suggests a clear trend towards a more evident change in the responding vs the non-responding patient population (**Figure 24G**). No change in the levels of neurotrophic or inflammatory factors was detected in the CSF of placebo patients (**Figure 24G**).

### ALSFRS-R Responder Analysis, Intent-to-Treat Population

The pre-specified responder analysis examined both percentage improvements and absolute point improvement per month in post treatment ALSFRS-R slope compared to pre-treatment slope. The first of these looked at patients who achieved 25%, 50%, 75% and 100% improvement. Neurologists view a 25% improvement in slope as at least somewhat clinically meaningful, and a 50% improvement in slope as very clinically meaningful (Castrillo-Viguera et al, Amyotrophic Lateral Sclerosis 2010; 11: 178-180).

Across all definitions of "responder" and at all except one time point studied, a higher percentage of MSC-NTF cells - treated (NurOwn-treated) subjects were responders compared to placebo. As an example, at week 12 (post-treatment), 40% of subjects in the MSC-NTF cells (NurOwn) arm, versus just 17% of placebo subjects, experienced an improvement in the ALSFRS-R slope post-treatment compared to pre-treatment of at least 50%.

### Change in slow vital capacity (SVC) Slope-Responder Analysis

The frequency and percentage of patients with at least 10%, 15%, 20%, 25%, 30%, 50%, 75%, and 100% change (improvement) in slope was compared between the treatment groups using Fisher's Exact test. Only results at 25%, 50%, 75% and 100% thresholds are summarized.

The proportion of patients who were responders based upon percent improvement in post-transplantation as compared to pre-transplantation slope in SVC, for the 2-weeks through 24-week follow-up period was measured. At a high threshold of ≥100% improvement, a higher percentage of MSC-NTF cells treated patients were responders compared to the placebo at the 4-weeks through 24-weeks follow-up time points. Across responders with at least 25%, 50%, 75%, and 100% improvement and at all time points except at the 2-weeks and 24-weeks (at the 50% threshold), a higher percentage of the MSC-NTF cells treated patients were responders compared to the placebo and the difference was statistically significant for the 50% threshold at the 16-week follow-up time point (one-sided, p=0.09). The changes observed at other time points were not statistically significant. The graphical representation of percentage of patients with ≥100% improvement in SVC scores is presented in **Figure 27C****.**

### Responders with a positive change of ≥ 1.5 points/month in the ALSFRS-R slope post-transplant compared to pre-transplant

When response to treatment was evaluated based on the absolute point improvement/month in ALSFRS-R post treatment slope compared to pre-treatment slope over time, again there was strong evidence of a MSC-NTF (NurOwn) treatment effect.

A higher proportion responders, defined by a positive change of ≥ 1.5 points/month in the ALSFRS-R slope post-transplant compared to pre-transplant, were observed in the MSC-NTF cells group compared to placebo at all time points. The difference was significant at Week 4 (MSC-NTF 47%, placebo 9%, p=0.033, **Figure 27A**).

In the subpopulation excluding slow progressors, a higher proportion of responders (defined as ≥ 1.5 points/month improvement) were observed in the MSC-NTF group compared to the placebo group at all time points; at 2 weeks (73% vs 20%), 4 weeks (80% vs 0%), 8 weeks (53% vs 20%), 12 weeks (53% vs 0%), 16 weeks (47% vs 0%), 24 weeks (27% vs 0%). The difference was significant at week 4 (p=0.004) and week 12 (p=0.046, **Figure 27B**).

### Responders with ≥100% Improvement in the ALSFRS-R Slope Post-Transplant Compared to Pre-Transplant

A higher proportion of responders, defined as those whose disease progression halted or whose ALSFRS-R actually stabilized or improved (≥100% improvement in the ALSFRS-R slope post-transplant compared to pre-transplant) were observed in the MSC-NTF cells treated group, at all follow-up visits, except at Week 8 (**Figure 28A**).

Given that MSC-NTF cells (NurOwn) is thought to slow disease progression, a pre-specified subgroup was defined in order to exclude subjects whose disease was progressing slowly (defined by an ALSFRS-R slope of -0.7 or higher during the pre-treatment phase). These subjects would be less likely to have a detectable benefit from MSC-NTF cells (NurOwn). The more rapidly progressing subgroup, comprising approximately half of the subjects in the study, showed a marked benefit from MSC-NTF cells (NurOwn) treatment; In the subpopulation excluding slow progressors, a higher proportion of responders were observed in the MSC-NTF group compared to the placebo group at all follow-up visits; at 2 weeks (93% vs 20%), 4 weeks (80% vs 20%), 8 weeks, (53% vs 20%), 12 weeks (47% vs 17%), 16 weeks (40% vs 0%), 24 weeks (27% vs 0%). The difference was statistically significant at Week 2 (p=0.005) and Week 4 (p=0.031) (Figure 28B).

The **Figure 28A** shows the benefit seen in MSC-NTF cells (NurOwn) treated patients; responders were defined using a very high threshold of 100% improvement in the slope post-treatment compared to the pre-treatment slope, meaning a subject needed to have stable disease or improve to be defined as a responder. The **Figure 28A** demonstrates both the near-term and long-term benefit of MSC-NTF cells (NurOwn) treated subjects as compared to placebo.

### Correlation between MCP-1 and post treatment ALSFRS-R slope

A statistically significant correlation was observed between the decrease in MCP-1 levels two weeks post-transplantation and slower disease progression post-transplantation as compared to the pre-transplantation slope, Significant correlations (p<0.05) were identified at 4, 12, 16 and 24 weeks post-treatment (**Figures 29A** **and** **29B****,** correlation at 12 weeks) suggesting that higher levels of MCP-1 levels may contribute to ALS disease progression.

### Biomarkers

In summary of the data showing the changes in NTF and inflammatory factors, it was found that there was a significant increase in the levels of NTFs in the CSF of MSC-NTF treated patients. No significant change in the levels of NTFs were observed in the CSF of placebo patients.

Inflammation in the CNS and the systemic circulation is considered to be a key factor in the pathogenesis of ALS. A significant post-transplantation reduction in some of the inflammatory markers tested (MCP-1 and SDF-1) were observed in the MSC-NTF cells treated patients while no change was observed in the placebo group, suggesting that the NTFs secreted by the cells modulate the levels of these inflammatory factors.

Furthermore, a statistically significant correlation was found between the increase in NTFs and the decrease in inflammatory factors in the MSC-NTF cells treated group while no correlation was found between any of these biomarkers in the placebo group suggesting a direct effect of the treatment on the inflammatory markers.

In addition, a statistically significant inverse correlation between the ALSFRS-R score and the CSF levels of CHIT, an enzyme synthesized by microglia or infiltrating macrophages found to play a role in neuroinflammation, was identified, suggesting it could be developed as a putative biomarker both for the diagnosis of ALS and for following its progression. In fact, normal levels of CHIT-1 in the CSF are reported to be in the range of 80 - 1,250 pg/ml (mean 982±245, Varghese et al. 2013), while in ALS patients, the baseline levels were found to be about twenty fold higher, in the range of 1,600-107,600 pg/mL. The mean change in levels of NTFs and potential ALS inflammatory biomarkers (CHIT, CRP, MCP-1, MIP-1 β, SDF-1) and of Caspase- 3 (a pro-apoptotic factor) from Visit 5 (pre-transplantation) to Visit 6 (post-transplantation) was measured (data not shown).

### Modulation of miRNA expression in the CSF

It was determined that specific miRNA have different levels of expression in MSC-NTF cells. In some embodiments, miR-34a and miR-132 expression is elevated in MSC-NTF cells compared with MSC (See WO2014/024183 incorporated herein in full). In some embodiments, miR-376a, miR-19b, and miR-146a are expressed in MSC-NTF cells (See WO2014/024183 incorporated herein in full). In some embodiments, miR-126 and miR-9 are not expressed or have very low expression in MSC-NTF cells (See WO2014/024183 incorporated herein in full).

Due to the limited amount of patients' CSF, samples of homogeneous groups of responder, non-responder and of placebo patients were pooled to evaluate miRNAs levels pre- and two weeks post-treatment (V5 and V6 respectively). Specifically, individual CSF samples were pooled as following: Responders V5 (n=6); Responders V6 (n=6); Non-responders V5 (n=9); Non-responders V6 (n=9); Placebo V5 (n=9); and Placebo V6 (n=9).

The expression of miR-34a, miR-132, miR-19b, miR376a-3p, and miR-146a-5p that are highly expressed in MSC-NTF cells (Data not shown) were measured. Expression measurements were made relative to a non-human exogenous miRNA spiked into the sample for normalization. The non-human miRNA was added in a fixed (constant amount) to an equal (same) volume of each CSF sample to be analyzed before RNA extraction from the CSF and was used to normalize the expression of the target miRNA to be analyzed. These miRNAs were found to be increased post treatment in the CSF of MSC-NTF treated but not placebo patients (**Figures 30A, 30B****,** **30C, 30D****, and** **30E**). Interestingly, miR-34a, miR376a -3pand miR-132 basal levels are notably lower in non-responder as compared to responder patients (**Figures 30A, 30B****, and** **30D****).**

In addition, the expression of miRNAs known to play a role in ALS was measured, including miR-9, miR-155 and miR-577. miR-155 and miR-577 were undetectable in the CSF of ALS patients. miR-9-5p which is not expressed in MSC-NTF cells, did not significantly change in either group following treatment (**Figure 30F**). In addition, miR-126, which has low or no expression in MSC-NTF cells, showed increased expression in non-responders (**Figure 30G**).

The results show different expression of some of the miRNAs in responder and non-responder patients.

Conclusions based on the miRNA data presented above include that (1) Increased expression levels of miRNAs known to be secreted by MSC-NTF, which was observed only in treated patients, is further evidence of a biological function of the MSC-NTF cells; and (2) Modulated expression of the miRNAs suggest anti-inflammatory and neuro-supportive effects.

### Glutamate Concentrations in the CSF

The glutamate concentrations of the CSF in placebo and MSC-NTF samples were analyzed two weeks before (V5) and two weeks after (V6) administration of cells. **Figure 31** shows significant elevation in glutamate in the CSF of patients treated with MSC-NTF cells, as detected using HPLC and colorimetric assays. No change was observed in the placebo CSF samples.

Further, the table presented in **Figure 32** highlights the seven (7) treated patients that had the most significant fold change in glutamate after treatment (524,605,608, 613,702,711,715). All of these patients were responders at least at the 2 week time point, wherein patients 605, 613 and 711 were responders at all time points, and patients 715 and 608 were responders up to 16 weeks. It is unclear if the Glutamate was released from the transplanted cells or from the patient himself, however it seems that the glutamate elevation was not toxic.

The lack of glutamate results for all patients was due to the relatively small amount of CSF were left for some of the patients.

### Slow Vital Capacity

Analysis of changes in post-treatment slope compared to pre-treatment slope and responder analyses with various thresholds showed no significant differences between the two-treatment groups. Excluding slow progressors did not impact the analysis.

### Hand Held Dynamometry (HHD)

Comparison of muscle strength in the IM transplanted arm to the non-injected arm, as measured by HHD, did not demonstrate significant side-to-side difference in HHD score slopes over 24 weeks.

### SAFETY AND TOLERABILITY

The trial met its primary endpoints for safety and tolerability. Forty-three participants (90%) completed the follow-up period with in-person visits (33 MSC-NTF; 10 Placebo). Five participants discontinued in-person follow-up; of these, two completed the trial with remote follow-up by telephone (**Figure 22C**). There were no deaths during the study, no treatment-related SAEs and no AEs that led to study dropout. Two participants in the MSC-NTF treatment arm underwent tracheostomy placement for respiratory failure following placement of a feeding tube, approximately four months post treatment.

Adverse events related to the delivery of the MSC-NTF or placebo were transient and included headache (75% MSC-NTF; 50% Placebo), fever (33% MSC-NTF; 0% Placebo), back pain (72% MSC-NTF; 8% Placebo) and injection site bruising (30.6% MSC-NTF; 25% Placebo, Table 5).

**Table 5: Overall Summary of Adverse Events (AE) and Serious Adverse Events (SAE) by Treatment Group - Safety population***

| **TEAEs** | **MSC-NTF** | **Placebo** |
|---|---|---|
| | **N=36** | **N=12** |
| | **n (%)** | **n (%)** |
| Number of TEAEs | 585 | 109 |
| Number of Participants with at least one TEAE | 36 (100%) | 12 (100%) |
| Number of Treatment-Related TEAEs [1] | 197 | 32 |
| Number of Participants with at least one Treatment-Related TEAE | 35 (97.2%) | 9 (75.0%) |
| Number of Treatment-Related Serious TEAEs | 0 | 0 |
| Number of Participants with at least one Treatment-Related Serious TEAE | 0 | 0 |
| Number of Participants with TEAEs by Maximum Severity | | |
| Mild | 36 (100%) | 12 (100%) |
| Moderate | 34 (94.4%) | 10 (83.3%) |
| Severe | 3 (8.3%) | 1 (8.3%) |
| Potentially Life-Threatening | 1 (2.8%) | 0 |

| Number of SAEs | 14 | 2 |
|---|---|---|
| Number of Participants with at least one SAE | 9 (25%) | 2 (16.6%) |
| Number of Participants with at least one Treatment-Emergent SAE | 8 (22.2%) | 1 (8.3%) |
| Number of Participants with Treatment-Related SAE | 0 | 0 |
| Number of Participants with TEAEs Resulting in Treatment Withdrawal | 0 | 0 |
| Number of Participants with TEAEs Resulting in Withdrawal from Study | 0 | 0 |
| Number of Participants with TEAEs Resulting in Death | 0 | 0 |

| | | |
|---|---|---|
| * Abbreviations: SAE = Serious adverse events; TEAE = Treatment emergent adverse events Percentages are based on the number of participants (N) in a given treatment group for the population being analyzed. An adverse event is considered a TEAE if the start date/time of the adverse event is on or after the date/time of initiation of cell transplantation or if the severity worsens after the initiation of cell transplantation. [1] Treatment-Related TEAEs are TEAEs that are considered to have probable, possible, or definite relationship to the study drug. | | |

AEs and SAEs are summarized in **Table 6** by system organ class. Over the course of the study, 11 participants (9/36 in the MSC-NTF cells group (25%) and 2/12 in the placebo group (17%)) developed 16 SAEs (**Table 7**). Two SAEs occurred after study entry but prior to study treatment. All treatment emergent serious adverse events (TE-SAEs) were related to ALS disease progression and no treatment-related serious adverse events (TR-SAEs) were observed during the study (TE-SAEs that are considered to have possible, probable, or definite relationship to the study drug). No clinically significant abnormalities were identified on safety lab tests (blood hematology, chemistries, urinalysis) during the study.

**Table 6: Percentage of Participants with Treatment Emergent Adverse Events by Trial Arm and Type (> 15% in either group)**

| **Adverse Event** | **MSC-NTF (%)** | **Placebo (%)** |
|---|---|---|
| Headache and Procedural Headache | 80.6 | 66.7 |
| Back Pain | 72.2 | 8.3 |
| Pyrexia | 33.3 | 0 |
| Arthralgia | 33.3 | 0 |
| Injection Site Pain | 27.8 | 8.3 |
| Constipation | 25 | 8.3 |
| Pain in Extremity | 22.2 | 0 |
| Neck Pain | 19.4 | 0 |
| Myalgia | 16.7 | 0 |
| Cough | 16.7 | 0 |
| Nausea | 16.7 | 0 |

**Table 7: Number and Percentage of Participants with Treatment Emergent Serious Adverse Events by Trial Arm and Type**

| | MSC-NTF (N=36) | | Placebo (N=12) | |
|---|---|---|---|---|
| Treatment Emergent Serious Adverse Event (TE-SAE) | # of Events | n (%) | # of Events | n (%). |
| Any TE-SAE | 14 | 8 (22.2) | 1 | 1 (8.3) |
| Dysphagia | 6 | 6 (16.7) | 1 | 1 (8.3) |
| Stoma Site Pain | 1 | 1 (2.8) | 0 | 0(0) |
| Hypophagia | 1 | 1 (2.8) | 0 | 0(0) |
| Breast Hyperplasia | 1 | 1 (2.8) | 0 | 0(0) |
| Dyspnea/Respiratory Fatigue | 3 | 3 (8.4) | 0 | 0(0) |
| Acute Respiratory Failure | 2 | 2(5.6) | 0 | 0(0) |

MSC-NTF cells (NurOwn) were found to be safe and well tolerated with the majority of adverse events being mild or moderate and transient. (See Tables 6 and 7 above)

There were no deaths reported in the study and no patients discontinued participation because of an adverse event. All patients in both active treatment and placebo groups experienced at least one treatment-emergent adverse event. Adverse events tended to be mild-to-moderate in intensity in both groups. Treatment-related adverse events, as determined by the blinded investigator, occurred slightly more frequently in active-treated patients than in placebo-treated patients, 97.2% vs. 75.0%. The largest differences in frequencies were for the localized reactions of injection site pain and back pain, and systemic reactions of pyrexia, headache, and arthralgia. The pattern of adverse events is consistent with a transient reaction to the transplantation. These adverse events were minor in nature and self-limiting. Post-therapy serious adverse events (SAEs) tended to occur more frequently in active-treatment patients (8/36, 22.2%) than in placebo patients (1/12, 8.3%). Most SAEs were related to progression of the underlying ALS, most commonly dysphagia. No SAEs were related to study treatment. The risk-benefit ratio for MSC-NTF cells remains positive.

### Adequacy of Blinding

Eighty-five percent of the participants in the treatment arm and 82% in the placebo arm thought that they had received MSC-NTF cells when asked immediately post treatment, while at the end of the trial 51% and 50% respectively in each treatment arm felt they had received MSC-NTF. Immediately post-transplant, investigators thought that 89% of participants in the active treatment arm and 50% in the placebo arm had received MSC-NTF. Investigator opinion remained similar at the end of the study (82% and 60% respectively).

### Conclusions

Autologous MSC-NTF cells transplanted intrathecally to ALS patient actively secrete NTFs *in vivo* into the CSF, leading to a significant reduction the levels of inflammatory factors in the CSF of the treated patients. No change in levels of NTFs nor of inflammatory factors was detected in the CSF of placebo patients.

The secretion of NTFs was also correlated to the change in disease progression as determined by the correlation between the increased secretion of NTFs from the cells, the decrease in inflammatory factor two weeks post-transplant, and the post-transplant improvement in ALSFRS-R slope. Inflammatory factors are in fact considered to be involved in the pathology of ALS.

Further, the primary objective was reached, demonstrating that the MSC-NTF cells were safe and well tolerated.

As the data from this example show, single administration of MSC-NTF cells (NurOwn®) produced a clinically meaningful beneficial response in terms of both the ALSFRS-R rating scale and CSF biomarkers.

In the intention to treat (ITT) population, across all definitions of "responder", a higher percentage of MCS-NTF cells (NurOwn®)-treated subjects were responders compared to placebo, at all except one time-point studied out to 24 weeks.

In a pre-specified subgroup analysis that excluded slowly progressing subjects, MCS-NTF cells (NurOwn®)-treated subjects showed marked outperformance compared to placebo-treated subjects.

Increased levels of growth factors in the CSF and decreased inflammatory markers observed after two weeks are further evidence of a biological effect.

### EXAMPLE 4

### Chitotriosidase as a biomarker for ALS

### Correlation between Chitotriosidase (CHIT1) and ALSFRS-R

Example 3 above, describes a Phase II Study of MSC-NTF cells (NurOwn®) in Patients with ALS. As described above, patients were randomized to receive NurOwn® cells administered via combined intramuscular and intrathecal injection (n= 36), or placebo (n=12). They were followed monthly for approximately three months before transplantation and six months following transplantation. CSF was collected from ALS patients immediately prior to transplant (at Visit 5) and 2 weeks post-transplant (at Visit 6).

### Methods and Results

ELISA commercial kits with a wide detection range were used to detect CHIT-1 in the CSF of study subjects from Example 3. A statistically significant inverse correlation between baseline (pre-treatment, visit 5) levels of CHIT-1 and ALS-FRS-R (a validated outcome measure of ALS disease progression) was observed (**Figure 33**).

Furthermore, a significant inverse correlation was observed between pre-treatment CHIT-1 levels and the rate of progression of ALS as evidenced by the change in ALSFRS-R slope (**Figure 34**) and Slow vital Capacity (SVC, a measure of pulmonary function, **Figure 35**) progression during the 3 months period prior to treatment (i.e.; pre-treatment slope).

### Conclusions

There is currently no biomarker that can be used to diagnose and follow ALS disease progression. The results show that Chitotriosidase (CHIT-1) levels in the CSF can be used as a bio-marker for ALS disease diagnosis and progression.

In fact normal levels of CHIT-1 in the CSF are reported to be in the range of 80 - 1,250 pg/ml, with a mean of 982 ± 245 (n = 11) while in ALS patients, the levels found were twenty-fold higher in the range of about 1,600-107,600 pg/ml ± with a mean of 30,171 ± 25,897 (n = 34).

### EXAMPLE 5

### Adipose derived MSC-NTF cells

*Objective:* To evaluate whether mesenchymal stem cells (MSC) isolated from other tissues could be induced to become MSC-NTF cells.

### Methods & Results:

MSC were isolated from adipose tissue of healthy donors (ATCC) propagated in platelet growth medium (PM) and induced to secrete neurotrophic factors (NT) such as Glial Cell Derived Neurotrophic Factor (GDNF), vascular endothelial growth factor (VEGF), and hepatocyte growth factor (HGF) by the method used for inducing secretion of NTFs from BM derived MSC-NTF cells (See, for example, Example 1).

The secretion of GDNF, VEGF, and HGF of from adipose derived MSC-NTF (AD-MSC-NTF) cells that were induced to differentiate from adipose derived MSC (AD-MSC) was measure and was within the range previously obtained from BM-MSC-NTF cells of healthy donors (Table 8). The NTFs of AD-MSC-NTF cells were secreted 3 to 43 fold as compared to MSC from the same subject prior to differentiation.

Adipose derived MSC-NTF cells were characterized by flow cytometry and found to express all bone marrow (BM) derived MSC-NTF cells' characteristic positive markers while they did not express any of the negative markers (CD19, CD34, CD14, CD3, CD45, HLA-DR). CD90, CD73, and CD44 were expressed in >96% of adipose derived MSC-NTF cells. CD105 was expressed in at least 91.8% of the cells (Table 9). The expression of all negative markers was below 1.83% (Table 9). Also CD49a expression was similar to that shown for BM derived MSC and MSC-NTF cells (Table 9).

**Table 8: Specific productivity of thawed AD-MSC-NTF cells as compared to donor BM-MSC**

| | **AD-MSC P8** | | | **AD-MSC P5** | | | **BM-MSC donors** | | |
|---|---|---|---|---|---|---|---|---|---|
| | **MSC (pg/10⁶ cells)** | **MSC-NTF (pg/10⁶ cells)** | **Fold Induction** | **MSC (pg/10⁶ cells)** | **MSC-NTF (pg/10⁶ cells)** | **Fold Induction** | **Fold Induction** | **Min** | **Max** |
| **GDNF** | 4 | 173 | 43.25 | 24 | 110 | 4.58 | 4.5 (n=12) | 1.5 | 8.9 |
| **VEGF** | 3738 | 12906 | 3.45 | 2603 | 18783 | 7.22 | 5.3 (n=8) | 1.8 | 18.6 |
| **HGF** | 15824 | 60639 | 3.83 | 11562 | 82036 | 7.1 | 8.6 (n=6) | 3 | 18.6 |

**Table 9: Phenotype of AD-MSC-NTF cells**

| **Experiment** | **AD-MSC vs**. **AD-MSC-NTF** | | | |
|---|---|---|---|---|
| **Passage** | **P8** | | **P5** | |
| **Cell Type** | **MSC** | **MSC-NTF** | **MSC** | **MSC-NTF** |
| **Surface Marker** | **% positive** | **% positive** | **% positive** | **% positive** |
| **CD73** | 97.18 | 98.22 | 96.05 | 97.54 |
| **CD90** | 98.22 | 98.18 | 97.9 | 97.65 |
| **CD105** | 94.36 | 93.12 | 91.8 | 92.47 |
| **CD44** | 96.54 | 97.71 | 96.83 | 96.54 |
| **CD49a** | 30.32 | 62.79 | 55.19 | 73.84 |
| **CD19** | 0.08 | 0.08 | 0.03 | 0 |
| **CD34** | 0.02 | 0.84 | 0.07 | 1.83 |
| **CD14** | 0 | 0 | 0 | 0 |
| **CD3** | 0 | 0 | 0.02 | 0.06 |
| **CD45** | 0 | 0.03 | 0.02 | 0.07 |
| **HLA-DR** | 0 | 0.01 | 0 | 0.01 |

### Modulation of CD44 and CD73 MFI of AD-MSC and AD-MSC-NTF cells

Modulation of CD44 and CD 73 Mean Fluorescence intensity (MFI) was similar to that observed with BM derived MSC and MSC-NTF cells (**Figure 36**). Table 10 presents the MFI of the different peaks.

**Table 10**

| | **MFI** | | **Ratio** |
|---|---|---|---|
| | **MSC** | **MSC-NTF** | |
| **CD44** | 136 | 34.7 | 0.26 |
| **CD73** | 88.5 | 155 | 1.75 |

CD44 Mean Fluorescence intensity (MFI) was down-regulated from 136 in AD-MSC prior to differentiation to 34.7 in AD-MSC-NTF (a ratio of 0.26, **Figure 1**), and CD73 was up-regulated from 88.5 in AD-MSC prior to differentiation to 155 in AD-MSC-NTF (a ratio of 1.75, **Figure 36**). These ratios are in line with those obtained for bone marrow derived MSC and MSC-NTF cells analyzed on the same day and under the same experimental conditions (a ratio of 0.59 and 1.8 respectively).

*Conclusion:* MSC derived from adipose tissue can be induced to differentiate into MSC-NTF secreting cells having the same characteristics as those derived from Bone Marrow.

### EXAMPLE 6

### Dental Pulp stem cells derived NTF cells

*Objective:* To evaluate whether mesenchymal stem cells (MSC) isolated from dental pulp could be induced to become MSC-NTF cells.

### Methods & Results:

Dental Pulp derived Stem Cells (DPSC) were purchased from a commercial vendor (Lonza) adapted to grow in PM (See Example 1) in the absence of antibiotics and induced into DPSC-NTF secreting cells. DPSC population could be propagated in PM without antibiotics with a population doubling of 0.91 in average.

DPSC were then induced to secrete NTFs by the method used for inducing secretion of NTFs from BM derived MSC-NTF cells (See Example 1 and disclosure therein).

Surface marker expression was similar to that of BM derived MSC-NTF cells and all MSC positive markers were expressed in >95% of cells propagated in PM (Table 11).

**Table 11: DPSC vs. DPSC-NTF phenotype**

| **Surface marker** | **DPSC P5 % Positive** | **DPSC-NTF P5 % Positive** |
|---|---|---|
| **CD73** | 98.94 | 99.37 |
| **CD90** | 98.91 | 99.13 |
| **CD105** | 95.93 | 96.22 |
| **CD44** | 91.96 | 98.19 |
| **CD49a** | 66.69 | 92.88 |
| **CD3** | 0 | 0.01 |
| **CD14** | 0.01 | 0.04 |
| **CD19** | 0.01 | 0.06 |
| **CD34** | 0 | 0.08 |
| **CD45** | 0 | 0 |
| **HLA-DR** | 0.01 | 0.04 |

Surface marker CD49a was also expressed in >90% of MSC-NTF cells as compared to 67% of MSC as in BM derived MSC and MSC-NTF cells (Table 12).

**Table 12: CD44 and CD73 Mean Florescence Intensity (MFI)**

| | **MFI** | | |
|---|---|---|---|
| | **DPSC** | **DPSC-NTF** | **Ratio** |
| **CD44** | 62.8 | 77.1 | 1.23 |
| **CD73** | 104 | 156 | 1.50 |

At the end of differentiation, GDNF, VEGF and HGF secretion was determined by ELISA. The results show that DPSC-NTF cells demonstrate a higher secretion of all NTFs, as compared to DPSC, by at least 2.27 fold (Table 13). The results showed that the fold induction of GDNF, VEGF and HGF of DPSC was within the range of previously obtained values from fresh BM-MSC of healthy donors (Table 13).

**Table 13: Specific productivity of DPSC and fresh donor BM-MSC**

| | **DPSC (pg/10⁶ cells)** | **DPSC-NTF (pg/10⁶ cells)** | **Fold Induction** | **BM-MSC donors** | | |
|---|---|---|---|---|---|---|
| | | | | **Fold Induction** | **Min** | **Max** |
| **GDNF** | 286 | 649 | 2.27 | 4.8 (n=20) | 1.5 | 9.4 |
| **VEGF** | 5,133 | 24,780 | 4.83 | 4.9 (n=9) | 1.5 | 18.6 |
| **HGF** | 23,317 | 62,845 | 2.70 | 6.78 (n=10) | 2.4 | 18.6 |

While certain features of the disclosure have been illustrated and described herein, many modifications, substitutions, changes, and equivalents will now occur to those of ordinary skill in the art. It is, therefore, to be understood that the appended claims are intended to cover all such modifications and changes as fall within the true spirit of the disclosure.

Embodiments of the invention, corresponding to the original claims of PCT/IL2017/050801 (published as WO 2018/015945 A2), include the following:
1. A method of treating a neurodegenerative disease in a subject in need thereof, the method comprising administering to said subject a therapeutically effective amount of a cell population of mesenchymal stem cells (MSC) cells that have been induced to secrete at least one neurotrophic factor (NTF), wherein said cell population comprises MSC-NTF cells, thereby treating said neurodegenerative disease in said subject.
2. The method of embodiment 1, wherein said neurodegenerative disease comprises Amyotrophic Lateral Sclerosis (ALS); Frontotemporal Dementia (FTD); Parkinson's disease; Multiple System Atrophy (MSA); Huntington's disease; Alzheimer's disease; Rett Syndrome; lysosomal storage diseases; "white matter disease" or glial/demyelination disease, including Sanfilippo, Gaucher disease; Tay Sachs disease (beta hexosaminidase deficiency); multiple sclerosis (MS); Neuromyelitis Optica (NMO), NMO spectrum disease, brain injury or trauma caused by ischemia, accidents, or environmental insult; stroke; cerebral palsy (CP); autism and autism spectrum disorder; spinal cord damage; or ataxia; or any combination thereof.
3. The method of any one of embodiments 1-2, wherein said mesenchymal stem cells comprise:
   (a) bone marrow mesenchymal stem cells, adipocyte mesenchymal stem cells, dental pulp mesenchymal stem cells placenta mesenchymal stem cells, synovial membrane mesenchymal stem cells, peripheral blood mesenchymal stem cells, periodontal ligament mesenchymal stem cells, endometrium mesenchymal stem cells, umbilical cord mesenchymal stem cells, or umbilical cord blood mesenchymal stem cells;
   (b) cells autologous with said subject; or
   (c) cells allogeneic with said subject;
   or any combination thereof.
4. The method of any one of embodiments 1-3, wherein said MSC-NTF comprise:
   (a) non-genetically modified human cells; or
   (b) mesenchymal stem cells induced *ex vivo* to express and secrete at least one neurotrophic factor (NTF);
   or any combination thereof.
5. The method of any one of embodiments 1-4, wherein basal secretion of said at least one NTF from said MSC-NTF cells is greater than a basal secretion of said NTF in a non-differentiated mesenchymal stem cell from the same subject.
6. The method of any one of embodiments 1-5, further comprising a step of assaying a biological sample from said subject prior to and following said administration.
7. The method of embodiment 6, wherein said biological sample comprises blood, serum, urine, or cerebrospinal fluid (CSF).
8. The method of any one of embodiments 1-7, wherein following said administration, said biological sample comprises increased levels of at least one neurotrophic factor (NTF) compared with a control biological sample.
9. The method of any one of embodiments 1-8, wherein said neurotropic factor (NTF) is selected from the group comprising a vascular endothelial growth factor (VEGF), a hepatocyte growth factor (HGF), a leukemia inhibitory factor (LIF), Granulocyte Stimulating factor (G-CSF), a Brain-derived neurotrophic factor (BDNF), a Tumor necrosis factor-inducible gene 6 protein (TSG-6; also known as TNF-stimulated gene 6 protein), Bone morphogenetic protein 2 (BMP2), Fibroblast Growth Factor 2 (FGF2), or a Neublastin, or any combination thereof.
10. The method of any one of embodiments 1-9, wherein following said administration, said biological sample comprises decreased levels of at least one inflammatory factor or pro-apoptotic factor or factor that influence inflammatory factors compared with a control biological sample.
11. The method of embodiment 10, wherein said inflammatory factor or pro-apoptotic factor or factor that influence inflammatory factors is selected from the group comprising a chitinase 1 (CHIT1), a C-reactive protein (CRP), a monocyte chemotactic protein 1 (MCP1), a stromal derived factor 1 (SDF-1), Macrophage Inflammatory protein (MIP)-1b, Glutamate, or a caspase 3 (CASP3), or any combination thereof.
12. The method of any one of embodiments 1-11, wherein gene expression of at least one biomarker is modulated in said MSC-NTF cells compared with control MSC cells.
13. The method of embodiment 12, wherein said biomarker comprises any one of TOP2A, FGF2, MEST, SLC1A1, or TUBB3, or a combination thereof, wherein said modulated gene expression comprises decreased gene expression.
14. The method of any one of embodiments 12-13, wherein said biomarker comprises any one of BMP2, LIF, WNT5A, AREG, HGF, BDNF, PCSK1, RAB27B, SNAP25, SLC1A3, or SLC16A6, or a combination thereof, wherein said modulated gene expression comprises increased gene expression.
15. The method of any one of embodiments 1-14, wherein following said administration said biological sample comprises increased levels of at least one neurotrophic factor and decreased levels of at least one inflammatory factor or pro-apoptotic factor or factor that influence inflammatory factors compared with a control biological sample.
16. The method of any one of embodiments 1-15, wherein said administration comprises administering to
   (a) the cerebrospinal fluid or the central nervous system of the subject; or
   (b) intramuscular (IM) injection or intrathecal (IT) injection, or a combination thereof; or any combination thereof.
17. The method of embodiment 16, where said IM injection is at a dose of about 2 x 10⁶ MSC-NTF cells per injection, and wherein said IT injection is at a dose of about 100-125 x 10⁶ MSC-NTF cells.
18. The method of embodiment 17, where said administration comprises multiple IM injections, wherein said multiple injections comprise a dose of about 48 x 10⁶ MSC-NTF.
19. The method of any one of embodiments 1-18, wherein said administration comprises a therapeutically effective number of time points.
20. The method of any one of embodiments 2-19, further comprising a step of detecting a biomarker associated with said ALS, or a biomarker that identifies progression of ALS, or a combination thereof.
21. The method of embodiment 20 wherein said biomarker comprises chitinase 1 (CHIT1), MCP-1, VEGF, miR-34a, miR-376-a, or miR-132, or any combination thereof.
22. A composition comprising a cell population present in an amount therapeutically effective to treat neurodegenerative disease in a subject, said cell population comprising mesenchymal stem cells (MSC) cells that have been induced to secrete at least one neurotrophic factor (NTF), wherein said cell population comprises MSC-NTF cells.
23. The composition of embodiment 22, wherein said neurodegenerative disease comprises Amyotrophic Lateral Sclerosis (ALS); frontotemporal dementia (FTD); Parkinson's disease; Multiple System Atrophy (MSA); Huntington's disease; Alzheimer's disease; Rett Syndrome; lysosomal storage diseases; "white matter disease" or glial/demyelination disease, including Sanfilippo, Gaucher disease; Tay Sachs disease (beta hexosaminidase deficiency); multiple sclerosis (MS); Neuromyelitis Optica (NMO); NMO spectrum disease; brain injury or trauma caused by ischemia, accidents, or environmental insult; stroke; cerebral palsy (CP); autism and autism spectrum disorder; spinal cord damage; or ataxia; or any combination thereof.
24. The composition of any one of embodiments 22-23, wherein said mesenchymal stem cells comprise
   (a) bone marrow mesenchymal stem cells, adipocyte mesenchymal stem cells, dental pulp mesenchymal stem cells, placenta mesenchymal stem cells, synovial membrane mesenchymal stem cells, peripheral blood mesenchymal stem cells, periodontal ligament mesenchymal stem cells, endometrium mesenchymal stem cells, umbilical cord mesenchymal stem cells, or umbilical cord blood mesenchymal stem cells;
   (b) cells autologous to said subject; or
   (c) cells allogeneic to said subject; or
   any combination thereof.
25. The composition of any one of embodiments 22-24, wherein said MSC-NTF comprise
   (a) non-genetically modified human cells; or
   (b) mesenchymal stem cells induced *ex vivo* to express and secrete at least one neurotrophic factor (NTF);
   or a combination thereof.
26. The composition of any one of embodiments 22-25, wherein a basal secretion of said at least one NTF is greater in said MSC-NTF cells compared with a basal secretion of said at least one NTF in a non-differentiated, mesenchymal stem cell.
27. The composition of any one of embodiments 22-26, wherein said at least one NTF is selected from the group comprising a vascular endothelial growth factor (VEGF), a hepatocyte growth factor (HGF), a leukemia inhibitory factor (LIF), Granulocyte Stimulating factor (G-CSF), a Brain-derived neurotrophic factor (BDNF), a Tumor necrosis factor-inducible gene 6 protein (TSG-6; also known as TNF-stimulated gene 6 protein), Bone morphogenetic protein 2 (BMP2), Fibroblast Growth Factor 2 (FGF2), or a Neublastin, or any combination thereof.
28. A method for detecting CHIT1 in a subject, the method comprising: obtaining a biological sample from said subject; detecting the level of a chitinase 1 (CHIT1) in the sample, wherein the level of said CHIT1 is in the range of 500-300,000 pg/ml.
29. The method of embodiment 28, wherein said biological sample comprises a cerebrospinal fluid (CSF) sample, a urine sample, a blood sample, or a serum sample, from said subject.
30. The method of any one of embodiments 28-29, wherein said detecting further comprising detecting MCP-1 in said subject.
31. The method of any one of embodiments 28-30, wherein said subject has ALS disease and said detecting determines progression of ALS.
32. A method of diagnosis of amyotrophic lateral sclerosis (ALS) in a subject, the method comprising: obtaining a biological sample from said subject; detecting the level of a chitinase 1 (CHIT1) in the sample, wherein the level of said CHIT1 in the range of 500-300,000 pg/ml indicates that said subject has said ALS disease.
33. The method of embodiment 32, wherein said biological sample comprises a cerebrospinal fluid (CSF) sample, a urine sample, a blood sample, or a serum sample, from said subject.
34. The method of any one of embodiments 32-34, wherein said detecting further comprises detecting MCP-1 in said subject, and wherein the level of MCP-1 indicates that said subject has said ALS disease.
35. The method of any one of embodiments 32-34, wherein said diagnosis determines progression of ALS.
36. A method for treating amyotrophic lateral sclerosis (ALS) in a subject, the method comprising: obtaining a biological sample from said subject; detecting the level of a chitinase 1 (CHIT1), wherein the level of said CHIT1 in the range of 1,600-107,600 pg/ml indicates that said subject has said ALS disease; and based on the detection of said CHIT1, treating said ALS in said subject.
37. The method of embodiment 36, wherein said biological sample comprises a cerebrospinal fluid (CSF) sample, urine, a blood sample, or a serum sample from said subject.
38. The method of any one of embodiments 36-37, wherein following said treating said method increases the level of VEGF, HGF, LIF, G-CSF, BDNF, TSG-6, miR-34a, miR-132, miR-19, miR376-a, or miR-146a-5p, or any combination thereof in a biological sample of said subject compared with a level in a control biological sample.
39. The method of any one of embodiments 36-38, wherein said method decreases the level of CHIT1, CRP, MCP1, SDF-1, Macrophage Inflammatory protein (MIP)-1b, Glutamate, or CASP3, or any combination thereof in a biological sample of said subject compared with the level in control sample.
40. The method of any one of embodiments 36-39, wherein a lower basal level of miR-34a, miR-376a, or miR-132, or any combination thereof in a biological sample from a subject to be treated compared with a biological sample from a responder patient, indicates a non-responder to MSC-NTF cell treatment.
41. A method for modulating a neurotrophic or an inflammatory factor or a pro-apoptotic factor or a factor that influence inflammatory factors in a subject, the method comprising administering to said subject a therapeutically effective amount of a cell population of mesenchymal stem cells (MSC) cells that have been induced to secrete at least one neurotrophic factor (NTF), wherein said cell population comprises MSC-NTF cells, thereby modulating said neurotrophic or an inflammatory factor or a pro-apoptotic factor or a factor that influence inflammatory factors in said subject.

## Claims

1. A method for detecting CHIT1 in a subject having amyotrophic lateral sclerosis (ALS), the method comprising detecting the level of chitinase 1 (CHIT1) in a cerebrospinal fluid (CSF) sample previously obtained from the subject, wherein the level of CHIT1 is in the range of 1,600-107,600 pg/ml, and wherein the detecting determines progression of ALS.

2. The method of claim 1, wherein the method further comprises detecting MCP-1 in the subject.

3. A method of diagnosis of amyotrophic lateral sclerosis (ALS) in a subject, the method comprising detecting the level of chitinase 1 (CHIT1) in a cerebrospinal fluid (CSF) sample previously obtained from the subject, wherein a level of CHIT1 in the range of 1,600-107,600 pg/ml indicates that the subject has ALS disease, and wherein the diagnosis determines progression of ALS.

4. The method of claim 3, wherein the method further comprises detecting MCP-1 in the subject, and wherein the level of MCP-1 indicates that the subject has ALS disease.

5. The method of any one of claims 1 to 4, wherein the level of CHIT1 is baseline level of CHIT1.

6. The method of any one of claims 1 to 5, wherein the level of CHIT1 is correlated with ALS-FRS-R score.

7. The method of claim 6, wherein the ALS-FRS-R score is baseline ALS-FRS-R score.

8. The method of any one of claims 1 to 5, wherein the level of CHIT1 is correlated with ALS-FRS-R slope.

9. The method of claim 8, wherein the ALS-FRS-R slope is baseline ALS-FRS-R slope.

10. The method of any one of claims 1 to 5, wherein the level of CHIT1 is correlated with Slow Vital Capacity (SVC) slope.

11. The method of claim 10, wherein the SVC slope is baseline SVC slope.
